# EUROPÄISCHE PATENTSCHRIFT

(11) **EP 0 284 563 B1**
(45) Veröffentlichungstag und Bekanntmachung des Hinweises auf die Patenterteilung: **15.06.1994**
(21) Anmeldenummer: 88810175.5
(22) Anmeldetag: 18.03.1988
(51) Int. Cl.: C07D 493/22, A01N 43/90, A61K 31/35

(54) **Milbemycin-Derivate zur Bekämpfung von Parasiten an Nutztieren**
Milbemycin derivatives for combating parasites on livestock
Dérivés de la milbémycine utiles pour combattre des parasites sur des animaux de ferme

(30) Priorität: 24.03.1987 CH 1117/87; 15.12.1987 CH 4878/87
(43) Veröffentlichungstag der Anmeldung: 28.09.1988
(73) Patentinhaber: CIBA-GEIGY AG, 4002 Basel (CH)
(72) Erfinder: Maienfisch, Peter, Dr., CH-4147 Aesch (CH)

(56) Entgegenhaltungen:
- EP-A- 0 184 173

## Beschreibung

Die vorliegende Erfindung betrifft neue 13-Spiro-2'-[tetrahydrofuran]-milbemycin-Derivate der nachstehenden Formel I, deren Herstellung, Mittel, die mindestens eine dieser Substanzen als Wirkstoff enthalten, sowie deren Verwendung zur Bekämpfung von Ekto- und Endoparasiten am Nutztier.

Die neuen Verbindung haben die allgemeine Formel I 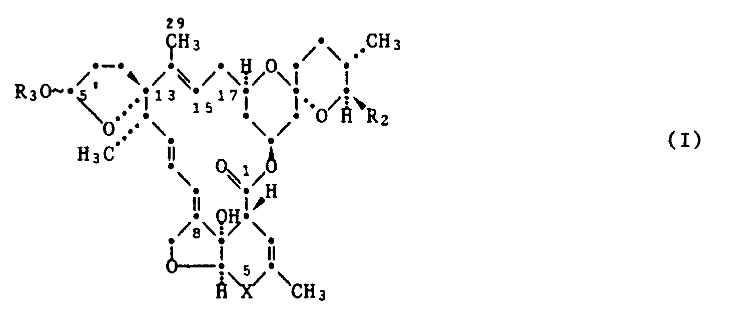 in welcher
X eine der Gruppen -CH(OR₁)-, -C( = O)- oder -C( = N-OH)- repräsentiert;
R₁ Wasserstoff oder eine OH-Schutzgruppe bedeutet;
R₂ für Methyl, Ethyl, Isopropyl oder sek.Butyl oder die Gruppe -C(CH3)=CH-A, worin A Methyl, Ethyl oder Isopropyl bedeutet, steht; und
R₃ Wasserstoff, C₁-C₁₀-Alkyl, durch mindestens einen Substituenten der Gruppe Halogen, C₁-C₆-Alkoxy, C₂-C₆-Alkoxyalkoxy, C₃-C₉-Alkoxyalkoxyalkoxy, C₁-C₆-Alkylthio, C₃-C₇-Cycloalkyl, durch C₁-C₃-Alkyl substituiertes C₃-C₇-Cyclaolkyl, Hydroxy, Benzyloxy, C₁-C₆-Acyl und C₁-C₆-Acyloxy substituiertes C₁-C₁₀-Alkyl, wobei jeder der vorgängig genannten, eine Alkoxygruppe darstellenden oder enthaltenden Reste an einer endständigen Alkoxygruppe terminal durch Hydroxy, Halogen, C₁-C₆-Acyl oder C₁-C₆-Acyloxy substituiert sein kann, C₃-C₇-Cycloalkyl, durch mindestens einen Substituenten der Gruppe Halogen und C₁-C₃-Alkyl substituiertes C₃-C₇-Cycloalkyl, C₃-C₇-Cycloalkenyl, C₂-C₁₀-Alkenyl, C₂-C₁₀-Alkinyl, einen durch Halogen, C₁-C₆-Alkoxy oder Ci-C₆-Acyloxy substituierten Rest aus der Gruppe C₂-C₁₀-Alkenyl und C₂-C₁₀-Alkinyl, 1-Adamantylmethyl, Menthyl, Carveyl, Phenyl, Benzyl, Naphthyl, einen durch mindestens einen Substituenten der Gruppe Halogen, C₁-C₃-Alkyl, C₁-C₃-Haloalkyl, C₁-C₃-Alkoxy, C₁-C₃-Haloalkoxy, C₁-C₃-Alkylthio, Nitro und Cyano substituierten Rest aus der Gruppe Phenyl, Benzyl und Naphthyl, durch eine Phenoxygruppe substituiertes Benzyl, oder einen unsubstituierten oder durch mindestens einen Substituenten der Gruppe Halogen, C1-C3-Alkyl, C₁-C₃-Haloalkyl, C₁-C₃-Alkoxy, C₁-C₃-Haloal- koxy, C₁-C₃-Alkylthio, Nitro und Cyano substituierten vier- bis sechsgliedrigen Heterocyclus mit ein bis drei Heteroatomen aus der Gruppe Sauerstoff, Schwefel und Stickstoff repräsentiert, wobei besagter Heterocyclus auch über eine C₁-C₆-Alkylenbrücke an das Sauerstoffatom in 5'-Position des Tetrahydrofuranrings gebunden sein kann.

Als Menthylgruppen kommen die vom o-, m- und p-Menthan abgeleiteten Menthylgruppen in Betracht, welche über eines der unsubstituierten Ringkohlenstoffatome mit dem am C₅'-Atom befindlichen Sauerstoffatom verknüpft sein können. Als bevorzugte Menthylgruppe ist 2-Methyl-6-isopropyl-cyclohexyl hervorzuheben. Carveyl steht vorzugsweise für 2-Methyl-5-(1-methylvinyl)-2-cyclohexen-2-yl.

Die Verbindungen der Formel I können als Epimerengemisch bezüglich Kohlenstoffatom Cs im Tetrahydrofuranring vorliegen. Die reinen Epimeren erhält man mittels üblicher physikalischer Trennmethoden. Die beiden Epimeren werden im folgenden mit den Buchstaben A und B gekennzeichnet.

Unter OH-Schutzgruppen für den Substituenten R₁ sollen hier und im folgenden die in der organischen Chemie üblichen Schutzfunktionen verstanden werden. Dabei handelt es sich insbesondere um Acyl- und Silylgruppen. Geeignete Acylgruppen sind beispielsweise die Reste R₄-C(0)-, wobei R₄ für C₁-C₁₀-Alkyl, C₁-C₁₀-Haloalkyl oder einen unsubstituierten oder durch mindestens einen Substituenten der Gruppe Halogen, C₁-C₃-Alkyl, C₁-C₃-Haloalkyl, C₁-C₃-Alkoxi, C₁-C₃-Haloalkoxi, Cyano und Nitro substituierten Rest aus der Gruppe Phenyl und Benzyl steht und vorzugsweise C₁-C₆-Alkyl, C₁-C₆-Haloalkyl oder unsubstituiertes oder durch Halogen, C₁-C₃-Alkyl, CF₃ oder Nitro substituiertes Phenyl bedeutet. Als geeignete Silylgruppe für R₁ kommt der Rest -Si(R₅)(R₆)(R₇) in Frage, wobei Rs, R₆ und R₇ vorzugsweise unabhängig voneinander für C₁-C₆-Alkyl, Benzyl oder Phenyl stehen und zusammen mit dem Siliciumatom beispielsweise eine der Gruppen Trimethylsilyl, Thexyldimethylsilyl (Thexyl = 1,1,2-Trimethyl-l-propyl: (CH₃)₂CH-C-(CH₃)₂-), Diphenyl-tert.butylsilyl, bis(lsopropyl)methylsilyl, Triphenylsilyl und insbesondere tert.Butyl-dime- thylsilyl bilden. Die 5-OH-Gruppe kann auch verethert als Benzylether oder Methoxiethoximethylether vorliegen oder gemäss der Europäischen Offenlegungsschrift Nr. 185,623 an einen Kohlenhydratrest, im folgenden einfachheitshalber als Zuckerrest bezeichnet, gebunden sein.

Als Strukturelemente, welche "durch mindestens einen Substituenten" einer näher bezeichneten Gruppe von Substituenten substituiert sind, kommen solche in Betracht, die sich von nach üblichen chemischen Methoden herstellbare Verbindungen ableiten lassen. Vorzugsweise sind besagte Strukturelemente durch 1 bis 3 Substituenten substituiert, wobei im allgemeinen nicht mehr als eine Nitro- oder Cyanogruppe vorliegt.

Verbindungen der Formel I, worin X für -CH(OR1)- steht und R₁ eine Schutzgruppe darstellt, lassen sich durch einfache, z.B. hydrolytische Abspaltung der Schutzfunktion in die hochaktiven freien 5-Hydroxiderivate (X = -CH(OR₁)-, R₁ = H) überführen und haben somit auch Zwischenprodukte-Charakter.

Als Substituenten der Phenyl-Gruppen sind 1 bis 3 Halogenatome, C1-C2-Alkyl, C₁-C₂-Alkoxi, Cₗ-C₂-Alkylthio, C₁-C₂-Haloalkyl oder Nitro und Cyano bevorzugt. Unter allen Resten an Phenylgruppen, die eine Alkylgruppe enthalten, sind insbesondere solche mit 1 C-Atom bevorzugt. Diese Substituenten können unabhängig voneinander in untereinander gemischter Anordnung auftreten. Als eine durch Alkyl substituierte Benzylgruppe ist auch eine a-Methylbenzylgruppe anzusehen.

Unter dem Begriff Alkyl selbst oder als Bestandteil eines anderen Substituenten sind je nach Zahl der angegebenen Kohlenstoffatome beispielsweise folgende Reste zu verstehen: Methyl, Ethyl, Propyl, Butyl, Pentyl, Hexyl, Heptyl, Octyl, Nonyl und Decyl, sowie die Isomeren, wie z.B. Isopropyl, Isobutyl, tert.-Butyl und Isopentyl. Haloalkyl steht für einen einfach bis perhalogenierten Alkylsubstituenten, wie z.B. CHCI₂, CHF₂, CH₂CI, CCl₃, CF₃, CH₂F, CH₂CH₂CI und CHBr₂, vorzugsweise für CF₃. Unter Halogen soll hier und im folgenden Fluor, Chlor, Brom oder Jod, vorzugsweise Fluor, Chlor oder Brom, verstanden werden. Alkenyl steht für einen durch mindestens eine C = C-Doppelbindung charakterisierten aliphatischen Kohlenwasserstoffrest, wie z.B. für Vinyl, Propenyl-(1), Allyl, Butenyl-(1), Butenyl-(2) und Butenyl-(3).

C₂-C₆-Alkoxyalkoxy steht für einen Alkoxyrest, dessen Kohlenstoffkette aus bis zu 6 C-Atomen besteht und durch ein Sauerstoffatom unterbrochen ist, z.B. für OCH₂OCH₃, OCH₂CH₂OCH₃, OCH₂0C₂H₅, OCH₂CH₂CH₂0C₃H₇ oder OC(CH₃)₂0C₂H₅. C₃-C₉-Alkoxyalkoxyalkoxy besteht aus einem Alkoxyrest, dessen Kohlenstoffkette aus 3 bis 9 C-Atomen besteht und an zwei Stellen durch ein Sauerstoffatom unterbrochen ist, z.B. OCH₂OCH₂OCH₃, OC₂H₄OC₂H₄OC₂H₅ oder OCH₂CH₂OCH₂CH₂OCH₂CH₂CH₃. Alkinyl steht z.B. für Ethinyl, Propinyl-(1), Propargyl oder Butinyl-(1). Cycloalkyl steht z.B. für Cyclopropyl, Cyclobutyl, Cyclopentyl, Cyclohexyl, Cycloheptyl oder Cyclooctyl. Von den durch Benzyloxy substituierten Alkylgruppen sind diejenigen bevorzugt, die 1 bis 3 Kohlenstoffatome im Alkylteil enthalten und durch Benzyloxy monosubstituiert sind, insbesondere 2-Benzyloxyethyl. Acyl als R₃ oder Teil von R₃ steht vorzugsweise für den von einer geradkettigen oder verzweigten Alkansäure abgeleiteten Alkanoylrest, z.B. CH₃ CO, C₂ H₅ CO, i-C₃ H₇ CO, n-C₃ H₇ CO, n-C₄ H₉-CO oder tert.-ButyICO, in welchen die Alkylreste auch halogeniert sein können wie beispielsweise vorstehend für Halogenalkyl angegeben. Cycloalkenyl steht für einen der obigen Cycloalkylreste, der aber mindestens eine Doppelbindung enthält und keinen aromatischen Charakter aufweist.

Unter den viergliedrigen, heterocyclischen Ringen sind insbesondere jene bevorzugt, die ein Heteroatom aus der Reihe Sauerstoff, Schwefel und Stickstoff enthalten und gesättigt sind. Typische Beispiele sind: 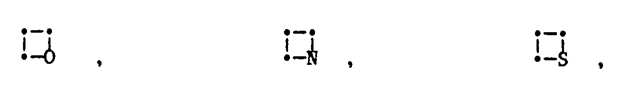 Typische fünfgliedrige heterocyclische Ringe sind: Furan, Thiophen, Pyrrol, Isoxazol, Isothiazol, Furazan, Imidazol, 1,2,4-Triazol, 1,2,3-Triazol, Pyrazol, Pyrrolin, Oxazol, Thiazol, Thiadiazole, Pyrazolin, Thiazolin, Pyrazolidin, Pyrrolidin, Oxazolidin, Thiazolidin, Oxadiazol, Imidazolin, Imidazolidin, Pyrazolidin, Tetrahydrofuran; und typische sechsgliedgrige heterocyclische Ringe sind Pyridin, Pyridazin, Pyrimidin, Pyrazin, Thiazin, Thiadiazine, Pyrane, Piperidin, Piperazin, Morpholin, Perhydrothiazin, Dioxan sowie ihre teilhydrierten bzw. teilgesättigten Homologen. Der Heterocyclus ist im allgemeinen über ein C-Atom, vorzugsweise dasjenige in Nachbarstellung zu einem Heteroatom, an den Rest des Moleküls gebunden.

Verbindungen der Formel I, worin X für -CH(OR₁)- oder -C(=N-OH)-steht, wobei R₁ für Wasserstoff oder eine OH-Schutzgruppe steht, sind bevorzugt, insbesondere diejenigen Verbindungen der Formel I, in denen X -CH(OR₁)- und R₁ Wasserstoff bedeutet. Acyl- und Silylgruppen als R₁ sind im allgemeinen als Schutzgruppen aufzufassen.

Bevorzugt sind Verbindungen der Formel I, in denen R₂ für Methyl, Ethyl, Isopropyl oder sek. Butyl, insbesondere für Ethyl oder Methyl, vorzugsweise für Ethyl steht.

Verbindungen, worin R₂ sek.Butyl darstellt, sollen hier und im folgenden gleichfalls zu den Milbemycin-Derivaten gerechnet werden, obwohl sie nach der üblichen Systematik von Avermectin-Derivaten abgeleitet sind. Avermectin-Aglykone (mit einer OH-Gruppe in 13a-Position) lassen sich jedoch gemäss US-PS 4,173,571 in Milbemycin-Homologe überführen.

In natürlich vorkommenden Milbemycinen (R₁ = H; R₂ = CH₃, C₂H₅ oder iso-C₃H₇) ist die 13-Position stets lediglich mit Wasserstoff besetzt. Bei Avermectinen dagegen steht in der 13-Position ein a-L-Oleandrosyl-a-L-oleandrose-Rest, der über Sauerstoff in a-Konfiguration mit dem Makrolid-Molekül verknüpft ist. Avermectine unterscheiden sich strukturell ausserdem durch eine 23-OH-Gruppe oder Δ^{22,23-} Doppelbindung und in der Regel durch einen Substituenten R₂ = sek.C₄H₉ von den Milbemycinen. Durch Hydrolyse des Zucker-Restes der Avermectine gelangt man leicht zu den entsprechenden Avermectin-Aglykonen, die eine allylische 13a-Hydroxi-Gruppe besitzen. Die Avermectin-Aglykone sind wie vorstehend angegeben, in die Milbemycin-Homologen umwandelbar. Bei den Milbemycin-Derivaten der vorliegenden Anmeldung liegt die Δ^{22,23}-poppelbindung stets in hydrierter Form vor.

Folgende Untergruppen von Verbindungen der Formel I sind auf Grund ihrer ausgeprägten parasitiziden und insektiziden Wirkung besonders bevorzugt:
Gruppe la: Verbindungen der Formel I, worin
   X -CH(OR₁)- oder -C( = N-OH)-, vorzugsweise -CH(OR₁)-, repräsentiert;
   R₁ Wasserstoff oder eine OH-Schutzgruppe bedeutet;
   R₂ für Methyl, Ethyl, Isopropyl oder sek.Butyl steht; und
   R₃ Wasserstoff, C₁-C₁₀-Alkyl, durch mindestens einen Substituenten der Gruppe Halogen, C₁-C₆-Alkoxy, C₂-C₆-Alkoxyalkoxy, C₃-C₉-Alkoxyalkoxyalkoxy, C₁-C₆-Alkylthio, C₃-C₇-Cycloalkyl, Hydroxy und Ci-C₆-Acyl substituiertes C₁-C₁₀-Alkyl, wobei jeder der vorgängig genannten, eine Alkoxygruppe darstellenden oder enthaltenden Reste an einer endständigen Alkoxygruppe terminal durch Hydroxy, Halogen, C₁-C₆-Acyl oder C₁-C₆-Acyloxy substituiert sein kann, eine durch Benzyloxy substituierte Aethylgruppe, C₃-C₇-Cycloalkyl, durch mindestens einen Substituenten der Gruppe Halogen und C₁-C₃-Alkyl substituiertes C₃-C₇-Cycloalkyl, C₃-C₇-Cycloalkenyl, C₂-C₁₀-Alkenyl, C₂-C₁₀-Alkinyl, einen durch Halogen, C₁-C₆-Alkoxy oder C₁-C₆-Acyloxy substituierten Rest aus der Gruppe C₂-C₁₀-Alkenyl und C₂-C₁₀-Alkinyl, 1-Adamantylmethyl, Mentyl, Carveyl, Phenyl, Benzyl, Naphthyl, einen durch mindestens einen Substituenten der Gruppe Halogen, C₁-C₃-Alkyl, C₁-C₃-Haloalkyl, C₁-C₃-Alkoxy, C₁-C₃-Haloalkoxy, C₁-C₃-Alkylthio, Nitro und Cyano substituierten Rest aus der Gruppe Phenyl, Benzyl und Naphthyl, durch eine Phenoxygruppe substituiertes Benzyl, oder einen unsubstituierten oder durch mindestens einen Substituenten der Gruppe Halogen, C₁-C₃-Alkyl, C₁-C₃-Haloalkyl, C₁-C₃-Alkoxy, C₁-C₃-Haloalkoxy, C₁-C₃-Alkylthio, Nitro und Cyano substituierten vier- bis sechsgliedrigen Heterocyclus mit ein bis drei Heteroatomen aus der Gruppe Sauerstoff, Schwefel und Stickstoff repräsentiert, wobei besagter Heterocyclus auch über eine C₁-C₆-Alkylenbrücke an das Sauerstoffatom in 5'-Position des Tetrahydrofuranrings gebunden sein kann.
Gruppe Ib: Verbindungen der Formel I, worin
   X -CH(OR₁)- repräsentiert;
   R₁ Wasserstoff oder eine OH-Schutzgruppe bedeutet;
   R₂ für Methyl, Ethyl, Isopropyl oder sek.Butyl steht; und
   R₃ Wasserstoff, C₁-C₁₀-Alkyl, durch mindestens einen Substituenten der Gruppe Halogen, C₁-C₆-Alkoxy, C₂-C₆-Alkoxyalkoxy, C₃-C₉-Alkoxyalkoxyalkoxy, C₁-C₆-Alkylthio, C₃-C₇-Cycloalkyl, Hydroxy und Ci-C₆-Acyl substituiertes C₁-C₁₀-Alkyl, wobei jeder der vorgängig genannten, eine Alkoxygruppe darstellenden oder enthaltenden Reste an einer endständigen Alkoxygruppe terminal durch Hydroxy, Halogen, C₁-C₆-Acyl oder C₁-C₆-Acyloxy substituiert sein kann, C₃-C₇-Cycloalkyl, durch mindestens einen Substituenten der Gruppe Halogen und C₁-C₃-Alkyl substituiertes C₃-C₇-Cycloalkyl, C₃-C₇-Cycloalkenyl, C₂-C₁₀-Alkenyl, C₂-C₁₀-Alkinyl, einen durch Halogen, C₁-C₆-Alkoxy oder C₁-C₆-Acyloxy substituierten Rest aus der Gruppe C₂-C₁₀-Alkenyl und C₂-C₁ₒ-Alkinyl, 1-Adamantylmethyl, Menthyl, Carveyl, Phenyl , Benzyl, Naphthyl, einen durch mindestens einen Substituenten der Gruppe Halogen, C₁-C₃-Alkyl, C₁-C₃-Haloalkyl, C₁-C₃-Alkoxy, C₁-C₃-Haloalkoxy, C₁-C₃-Alkylthio, Nitro und Cyano substituierten Rest aus der Gruppe Phenyl, Benzyl und Naphthyl, oder einen unsubstituierten oder durch mindestens einen Substituenten der Gruppe Halogen, C₁-C₃-Alkyl, C₁-C₃-Haloalkyl, C₁-C₃-Alkoxy, C₁-C₃-Haloalkoxy, C₁-C₃-Alkylthio, Nitro und Cyano substiutierten vier- bis sechsgliedrigen Heterocyclus mit ein bis drei Heteroatomen aus der Gruppe Sauerstoff, Schwefel und Stickstoff repräsentiert, wobei besagter Heterocyclus auch über eine C₁-C₆-Alkylenbrücke an das Sauerstoffatom in 5'-Position des Tetrahydrofuranrings gebunden sein kann.
Gruppe Ic: Verbindungen der Formel I, worin X für -CH(OR₁)- und R₁ für Wasserstoff, R₄-C(0)- oder -Si-(R₅)(R₆)(R₇) steht; wobei R₄ C₁-C₁₀-Alkyl, C₁-C₁₀-Haloalkyl oder einen unsubstituierten oder durch mindestens einen Substituenten der Gruppe Halogen, C₁-C₃-Alkyl, C₁-C₃-Haloalkyl, C₁-C₃-Alkoxy, Ci-C₃-Haloalkoxy, Cyano und Nitro substituierten Rest aus der Gruppe Phenyl und Benzyl bedeutet und Rₛ, R₆ und R₇ unabhängig voneinander für C₁-C₆-Alkyl, Benzyl oder Phenyl stehen; R₂ für Methyl, Ethyl, Isopropyl oder sek.-Butyl steht; und R₃ Wasserstoff, C₁-C₅-Alkyl, durch mindestens einen Substituenten der Gruppe Halogen, C₁-C₃-Alkoxy, C₂-C₆-Alkoxyalkoxy, C₃-C₉-Alkoxyalkoxyalkoxy, C₁-C₃-Alkylthio, C₃-C₇-Cycloalkyl, Hydroxy und C₁-C₆-Acyl substituiertes C₁-C₅-Alkyl, wobei jeder der vorgängig genannten, eine Alkoxygruppe darstellenden oder enthaltenden Reste an einer endständigen Alkoxygruppe terminal durch Hydroxy, Halogen, C₁-C₆-Acyl oder C₁-C₆-Acyloxy substituiert sein kann; C₃-C₇-Cycloalkyl, durch mindestens einen Substituenten der Gruppe Fluor, Chlor, Brom und Methyl substituiertes C₃-C₇-Cycloalkyl, C₂-C₆-Alkenyl, C₂-C₆-Alkinyl, einen durch Fluor, Chlor, Brom, C₁-C₃-Alkoxy oder C₁-C₆-Acyloxy substituierten Rest aus der Gruppe C₂-C₆-Alkenyl und C₂-C₆-Alkinyl, Phenyl, Benzyl, a-Naphthyl, β-Naphthyl, einen durch mindestens einen Substituenten der Gruppe Fluor, Chlor, Brom, Methyl, Methoxy, CF₃, CF₃0, CH₃S, Nitro und Cyano substituierten Rest aus der Gruppe Phenyl, Benzyl, a-Naphthyl und β-Naphthyl, oder einen unsubstituierten oder durch mindestens einen Substituenten der Gruppe Fluor, Chlor, Brom, Methyl, Ethyl, CF₃, CH₃0, CF₃0 CH₃S, Nitro und Cyano substituierten vier- bis sechsgliedrigen Heterocyclus mit ein bis drei Heteroatomen aus der Gruppe Sauerstoff, Schwefel und Stickstoff repräsentiert, wobei besagter Heterocyclus auch über eine Ci-C₆-Alkylenbrücke an das Sauerstoffatom in 5'-Position des Tetrahydrofuranringes gebunden sein kann.
Gruppe Id: Verbindungen der Formel I, worin X für -CH(OR₁)- und R₁ für Wasserstoff, R₄-C(0)- oder -Si-(R₅)(R₆)(R₇) steht; wobei R₄ C₁-C₁₀-Alkyl, C₁-C₁₀-Haloalkyl oder einen unsubstituierten oder durch mindestens einen Substituenten der Gruppe Halogen, C₁-C₃-Alkyl, C₁-C₃-Haloalkyl, C₁-C₃-Alkoxy, Ci-C₃-Haloalkoxy, Cyano und Nitro substituierten Rest aus der Gruppe Phenyl und Benzyl bedeutet und Rₛ, R₆ und R₇ unabhängig voneinander für C₁-C₄-Alkyl, Benzyl oder Phenyl stehen; R₂ für Methyl, Ethyl, Isopropyl oder sek.-Butyl steht; und R₃ C₁-C₅-Alkyl oder durch mindestens einen Substituenten der Gruppe Halogen, C₁-C₃-Alkoxy, C₂-C₆-Alkoxyalkoxy, C₃-C₉-Alkoxyalkoxyalkoxy, C₁-C₃-Alkylthio, C₃-C₇-Cycloalkyl, Hydroxy und C₁-C₆-Acyl substituiertes C₁-C₅-Alkyl, wobei jeder der vorgängig genannten, eine Alkoxygruppe darstellenden oder enthaltenden Reste an einer endständigen Alkoxygruppe terminal durch Hydroxy, Halogen, C₁-C₆-Acyl oder C₁-C₆-Acyloxy substituiert sein kann, repräsentiert.
Gruppe le: Verbindungen der Formel I, worin X für -CH(OR₁)- und R₁ für Wasserstoff, R₄-C(0)- oder -Si-(R₅)(R₆)(R₇) steht; wobei R₄ C₁-C₁₀-Alkyl, C₁-C₁₀-Haloalkyl oder einen unsubstituierten oder durch mindestens einen Substituenten der Gruppe Halogen, C₁-C₃-Alkyl, C₁-C₃-Haloalkyl, C₁-C₃-Alkoxy, Ci-C₃-Haloalkoxy, Cyano und Nitro substituierten Rest aus der Gruppe Phenyl und Benzyl bedeutet und Rₛ, R₆ und R₇ unabhängig voneinander für C₁-C₄-Alkyl, Benzyl oder Phenyl stehen; R₂ für Methyl, Ethyl, Isopropyl oder sek.-Butyl steht; und R₃ C₃-C₇-Cycloalkyl, durch mindestens einen Substituenten der Gruppe Fluor, Chlor, Brom und Methyl substituiertes C₃-C₇-Cycloalkyl, C₂-C₆-Alkenyl, C₂-C₆-Alkinyl, einen durch Fluor, Chlor, Brom, C₁-C₃-Alkoxy oder C₁-C₆-Acyloxy substituierten Rest aus der Gruppe C₂-C₆-Alkenyl und C₂-C₆-Alkinyl, Phenyl, Benzyl, a-Naphthyl, β-Naphthyl, einen durch mindestens einen Substituenten der Gruppe Fluor, Chlor, Brom, Methyl, Methoxy, CF₃, CF₃0, CH₃S, Nitro und Cyano substituierten Rest aus der Gruppe Phenyl, Benzyl, a-Naphthyl und β-Naphthyl, oder einen unsubstituierten oder durch mindestens einen Substituenten der Gruppe Fluor, Chlor, Brom, Methyl, Ethyl, CF₃, CH₃0, CF₃0, CH₃S, Nitro und Cyano substituiertem vier- bis sechsgliedrigen Heterocyclus mit ein bis drei Heteroatomen aus der Gruppe Sauerstoff, Schwefel und Stickstoff repräsentiert, wobei besagter Heterocyclus auch über eine C₁-C₆-Alkylenbrücke an das Sauerstoffatom in 5'-Position des Tetrahydrofuranringes gebunden sein kann.
Gruppe If: Verbindungen der Formel I, worin X für -CH(OR₁)- und R₁ für Wasserstoff, R₄-C(0)- oder -Si-(R₅)(R₆)(R₇) steht; wobei R₄ C₁-C₁₀-Alkyl, C₁-C₁₀-Haloalkyl oder einen unsubstituierten oder durch mindestens einen Substituenten der Gruppe Halogen, C₁-C₃-Alkyl, C₁-C₃-Haloalkyl, C₁-C₃-Alkoxy, Ci-C₃-Haloalkoxy, Cyano und Nitro substituierten Rest aus der Gruppe Phenyl und Benzyl bedeutet und Rₛ, R₆ und R₇ unabhängig voneinander für C₁-C₄-Alkyl, Benzyl oder Phenyl stehen; R₂ für Methyl, Ethyl, Isopropyl oder sek.-Butyl steht; und R₃ Phenyl, Benzyl, a-Naphthyl, β-Naphthyl, einen durch mindestens einen Substituenten der Gruppe Fluor, Chlor, Brom, Methyl, Methoxy, CF₃, CF₃0, CH₃S, Nitro und Cyano substituierten Rest aus der Gruppe Phenyl, Benzyl, a-Naphthyl und β-Naphthyl repräsentiert.
Gruppe Ig: Verbindungen der Formel I, worin X für -CH(OR₁)- und R₁ für Wasserstoff, R₄-C(0)- oder -Si-(R₅)(R₆)(R₇) steht; wobei R₄ C₁-C₁₀-Alkyl, C₁-C₁₀-Haloalkyl oder einen unsubstituierten oder durch mindestens einen Substituenten der Gruppe Halogen, C₁-C₃-Alkyl, C₁-C₃-Haloalkyl, C₁-C₃-Alkoxy, Ci-C₃-Haloalkoxy, Cyano und Nitro substituierten Rest aus der Gruppe Phenyl und Benzyl bedeutet und Rₛ, R₆ und R₇ unabhängig voneinander für C₁-C₄-Alkyl, Benzyl oder Phenyl stehen; R₂ für Methyl, Ethyl, Isopropyl oder sek.-Butyl steht; und R₃ C₂-C₆-Alkenyl, C₂-C₆-Alkinyl, oder einen durch Fluor, Chlor, Brom, C₁-C₃-Alkoxy oder C₁-C₆-Acyloxy substituierten Rest aus der Gruppe C₂-C₆-Alkenyl und C₂-C₆-Alkinyl repräsentiert.
Gruppe Ih: Verbindungen der Formel I, worin X für -CH(OR₁)- und R₁ für Wasserstoff, R₄-C(0)- oder -Si-(R₅)(R₆)(R₇) steht; wobei R₄ C₁-C₁₀-Alkyl, C₁-C₁₀-Haloalkyl oder einen unsubstituierten oder durch mindestens einen Substituenten der Gruppe Halogen, C₁-C₃-Alkyl, C₁-C₃-Haloalkyl, C₁-C₃-Alkoxy, Ci-C₃-Haloalkoxy, Cyano und Nitro substituierten Rest aus der Gruppe Phenyl und Benzyl bedeutet und Rₛ, R₆ und R₇ unabhängig voneinander für C₁-C₄-Alkyl, Benzyl oder Phenyl stehen; R₂ für Methyl, Ethyl, Isopropyl oder sek.-Butyl steht; und R₃ einen unsubstituierten oder durch mindestens einen Substituenten der Gruppe Fluor, Chlor, Brom, Methyl, Ethyl, CF₃, CH₃0, CF₃0, CH₃S, Nitro und Cyano substituierten vier- bis sechsgliedrigen Heterocyclus mit ein bis drei Heteroatomen aus der Gruppe Sauerstoff, Schwefel und Stickstoff repräsentiert, wobei besagter Heterocyclus auch über eine C₁-C₆-Alkylenbrücke an das Sauerstoffatom in 5'-Position des Tetrahydrofuranringes gebunden sein kann.
Gruppe li: Verbindungen der Formel I, worin X für -CH(OR₁)- und R₁ für Wasserstoff, R₄-C(0)- oder -Si-(R₅)(R₆)(R₇) steht; wobei R₄ C₁-C₁₀-Alkyl, C₁-C₁₀-Haloalkyl oder einen unsubstituierten oder durch mindestens einen Substituenten der Gruppe Halogen, C₁-C₃-Alkyl, C₁-C₃-Haloalkyl, C₁-C₃-Alkoxy, Ci-C₃-Haloalkoxy, Cyano und Nitro substituierten Rest aus der Gruppe Phenyl und Benzyl bedeutet und Rₛ, R₆ und R₇ unabhängig voneinander für C₁-C₄-Alkyl, Benzyl oder Phenyl stehen; R₂ für Methyl, Ethyl, Isopropyl oder sek.-Butyl steht; und R₃ für einen ungesättigten oder vorzugsweise gesättigten viergliedrigen Heterocyclus mit einem Heteroatom aus der Gruppe Sauerstoff, Stickstoff und Schwefel steht oder Furan, Thiophen, Pyrrol, Isoxazol, Isothiazol, Furazan, Imidazol, 1,2,4-Triazol, 1,2,3-Triazol, Pyrazol, Pyrrolin, Oxazol, Thiazol, Thiadiazole, Pyrazolin, Thiazolin, Pyrazolidin, Pyrrolidin, Oxazolidin, Thiazolidin, Oxadiazol, Imidazolin, Imidazolidin, Pyrazolidin, Tetrahydrofuran, Pyridin, Pyridazin, Pyrimidin, Pyrazin, Thiazin, Thiadiazine, Pyrane, Piperidin, Piperazin, Morpholin, Perhydrothiazin oder Dioxan repräsentiert, wobei besagter Heterocyclus auch über eine C₁-C₄-Alkylenbrücke an das Sauerstoffatom in 5'-Position des Tetrahydrofuranrings gebunden sein kann.
Gruppe Ik: Verbindungen der Formel I, worin X für -CH(OR₁)- und R₁ für Wasserstoff oder Si(R₅)(R₆)(R₇) steht, wobei Rₛ, R₆ und R₇ unabhängig voneinander für C₁-C₆-Alkyl stehen; R₂ für Methyl, Ethyl, Isopropyl oder sek.-Butyl steht; und R₃ C₁-C₁₀-Alkyl, durch mindestens einen Substituenten der Gruppe C₁-C₄-Alkoxy, C₁-C₃-Alkylthio, Cᵢ-C₆-Alkanoyloxy, Benzyloxy und C₃-C₇-Cycloalkyl substituiertes Cᵢ-C₁₀-Alkyl, C₃-C₇-Cycloalkyl, Phenyl, Benzyl oder einen durch mindestens einen Substituenten der Gruppe Halogen, C₁-C₃-Alkyl, C₁-C₃-Haloalkyl, C₁-C₃-Alkoxy, C₁-C₃-Haloalkoxy und C₁-C₃-Alkylthio substituierten Rest aus der Gruppe Phenyl und Benzyl repräsentiert.
Gruppe 11: Verbindungen der Formel I, worin X für -CH(OR₁)- und R₁ für Wasserstoff oder Si(R₅)(R₆)(R₇) steht, wobei Rₛ, R₆ und R₇ unabhängig voneinander für C₁-C₆-Alkyl stehen; R₂ für Methyl, Ethyl, Isopropyl oder sek.-Butyl steht; und R₃ Wasserstoff, C₁-C₁₀-Alkyl, durch mindestens einen Substituenten der Gruppe Halogen, C₁-C₆-Alkoxy, C₂-C₆-Alkoxyalkoxy, C₃-C₉-Alkoxyalkoxyalkoxy, C₁-C₆-Alkylthio, C₃-C₇-Cycloalkyl, Hydroxy und C₁-C₆-Alkanoyloxy substituiertes C₁-C₁₀-Alkyl, wobei jeder der vorgängig genannten, eine Alkoxygruppe darstellenden oder enthaltenden Reste an einer endständigen Alkoxygruppe terminal durch Hydroxy, Halogen, C₁-C₆-Acyl oder Cᵢ-C₆-Acyloxy substituiert sein kann, C₃-C₇-Cycloalkyl, durch mindestens einen Substituenten der Gruppe Halogen und C₁-C₃-Alkyl substituiertes C₃-C₇-Cycloalkyl, C₃-C₇-Cycloalkenyl, C₂-C₁₀-Alkenyl, C₂-C₁₀-Alkinyl, einen durch Halogen, C₁-C₆-Alkoxy oder C₁-C₆-Acyloxy substituierten Rest aus der Gruppe C₂-C₁₀-Alkenyl und C₂-C₁₀-Alkinyl, 1-Adamantylmethyl, Menthyl, Carveyl, Phenyl , Benzyl, Naphthyl oder einen durch mindestens einen Substituenten der Gruppe Halogen, C₁-C₃-Alkyl, C₁-C₃-Haloalkyl, C₁-C₃-Alkoxy, C₁-C₃-Haloalkoxy, Ci-C₃-Alkylthio, Nitro und Cyano substituierten Rest aus der Gruppe Phenyl, Benzyl und Naphthyl repräsentiert.
Gruppe Im: Verbindungen der Formel I, worin X für -CH(OR₁)- und R₁ für Wasserstoff, Trimethylsilyl, tert.-Butyldimethylsilyl oder Thexyldimethylsilyl steht; R₂ Methyl, Ethyl, Isopropyl oder sek.-Butyl bedeutet; und R₃ Wasserstoff, C₁-C₁₀-Alkyl, durch mindestens einen Substituenten der Gruppe Halogen, Ci-C₆-Alkoxy, C₂-C₆-Alkoxyalkoxy, C₃-C₉-Alkoxyalkoxyalkoxy, C₁-C₆-Alkylthio, C₃-C₇-Cycloalkyl, Hydroxy und C₁-C₆-Alkanoyloxy substituiertes C₁-C₁₀-Alkyl, wobei jeder der vorgängig genannten, eine Alkoxygruppe darstellenden oder enthaltenden Reste an einer endständigen Alkoxygruppe terminal durch Hydroxy, Halogen, Cᵢ-C₆-Acyl oder Cᵢ-C₆-Acyloxy substituiert sein kann, C₃-C₇-Cycloalkyl, durch mindestens einen Substituenten der Gruppe Halogen und C₁-C₃-Alkyl substituiertes C₃-C₇-Cycloalkyl, C₂-C₁₀-Alkenyl, C₂-C₁₀-Alkinyl, 1-Adamantylmethyl, Menthyl, Carveyl, Phenyl , Benzyl, Naphthyl oder einen durch mindestens einen Substituenten der Gruppe Halogen, C₁-C₃-Alkyl, C₁-C₃-Haloalkyl, Cₗ-C₃-Alkoxy, C₁-C₃-Haloalkoxy, C₁-C₃-Alkylthio, Nitro und Cyano substituierten Rest aus der Gruppe Phenyl oder Benzyl repräsentiert.
Gruppe In: Verbidungen der Formel I, worin X für -CH(OR₁)-, worin R₁ Wasserstoff oder tert.-Butyldimethylsilyl bedeutet, oder -C(=N-OH)-steht; R₂ für Methyl oder bevorzugt Ethyl steht; und R₃
   - Wasserstoff,
   - C₁-C₈-Alkyl,
   - C₁-C₅-Alkyl, welches substituiert ist durch 1 bis 3 Halogenatome, vorzugsweise Chlor- oder Bromatome, oder monosubstituiert ist durch
      - C₁-C₃-Alkoxy,
      - C₂-C₆-Alkoxy, welches durch ein Sauerstoffatom unterbrochen und unsubstituiert oder an der endständigen Alkoxygruppe terminal durch Hydroxy oder halogeniertes, vorzugsweise chloriertes C₁-C₃-Alkanoyloxy monosubstituiert ist,
      - C₁-C₃-Alkylthio,
      - C₃-C₇-Cycloalkyl oder
      - Hydroxy,
   - C₂-C₄-Alkyl, welches durch unsubstituiertes oder halogeniertes, vorzugsweise chloriertes C₁-C₃-Alkanoyloxy oder durch Benzyloxy monosubstituiert ist,
   - C₃-C₇-Cycloalkyl, welches unsubstituiert oder durch C₁-C₃-Alkyl mono- oder disubstituiert ist,
   - 1-Adamantylmethyl,
   - Phenyl,
   - Benzyl, welches unsubstituiert durch Phenoxy monosubstituiert oder durch 1 bis 3 C₁-C₃-Alkoxy substituiert ist,
   - a-Methylbenzyl oder einen
   - Heterocyclus der Gruppe Oxetanyl und Furyl, welcher über C₁-C₃-Alkyl gebunden und unsubstituiert oder durch Methyl substituiert ist,

   repräsentiert.
Gruppe lo: Verbindungen der Formel I, worin X für - CH(OR₁) und R₁ für Wasserstoff oder tert.-Butyldimethylsilyl steht; R₂ für Ethyl steht; und R₃
   - Wasserstoff
   - C₁-C₈-Alkyl,
   - C₁-C₅-Alkyl, welches substituiert ist durch 1 bis 3 Halogenatome, vorzugsweise Chlor- oder Bromatome, oder monosubstituiert ist durch
      - C₁-C₃-Alkoxy,
      - C₂-C₆-Alkoxy, welches durch ein Sauerstoffatom unterbrochen und unsubstituiert oder an der endständigen Alkoxygruppe terminal durch Hydroxy oder halogeniertes, vorzugsweise chloriertes C₁-C₃-Alkanoyloxy monosubstituiert ist,
      - C₁-C₃-Alkylthio,
      - C₃-C₇-Cycloalkyl oder
      - Hydroxy,
   - Ethyl monosubstituiert durch Acetoxy, Chloracetoxy oder Benzyloxy,
   - C₃-C₇-Cycloalkyl, welches unsubstituiert oder durch C₁-C₃-Alkyl mono- oder disubstituiert ist,
   - 1-Adamantylmethyl,
   - Phenyl
   - Benzyl, welches unsubstituiert, durch Phenoxy monosubstituiert oder durch 1-3 C₁-C₃-Alkoxy substituiert ist,
   - a-Methylbnezyl oder einen
   - Heterocyclus der Gruppe Oxetanyl und Furyl, welcher über C₁-C₃-Alkyl gebunden und unsubstituiert oder durch Methyl substituiert ist,

   repräsentiert.
Gruppe Ip: Verbindungen der Formel I, worin X für -CH(OR₁)- und R₁ für Wasserstoff oder tert.-Butyldimethylsilyl steht; R₂ für Ethyl steht; und R₃ Wasserstoff, C₁-C₈-Alkyl, 2,2,2-Tribromethyl, 2,2-Bis-(chlormethyl)-propyl, 3-Chlor-2,2-dimethylpropyl, 2-Ethoxyethyl, 2-(2-Methoxyethoxy)-ethyl, 2-[2-(2-Hy- droxyethoxy)-ethoxy]-ethyl, 2-{2-[2-Chloracetoxy)-ethoxy]-ethoxy}-ethyl, 2-Methylthioethyl, Cyclobutylmethyl, Cyclohexylmethyl, 2-Hydroxyethyl, Benzyloxyethyl, 2-Acetoxyethyl, 2-(Chloracetoxy)-ethyl, Cyclopentyl, Cyclohexyl, Cycloheptyl, 2-Methyl-6-isopropyl-cyclohexyl, 1-Adamantylmethyl, Phenyl, Benzyl, 3-Phenoxybenzyl, 3,4-Dimethoxybenzyl, a-Methylbenzyl, (3-Methyloxetan-3-yl)-methyl oder Furfuryl repräsentiert.
Gruppe Iq: Verbindungen der Formel I, worin X für -CH(OR₁)- und R₁ für Wasserstoff oder tert.-Butyldimethylsilyl steht; R₂ für Ethyl steht; und R₃
   - C₁-C₅-Alkyl,
   - C₂-C₄-Alkyl, welches substituiert ist durch 1 bis 3 Halogenatome, insbesondere Bromatome, oder monosubstituiert ist durch
      - C₁-C₃-Alkoxy,
      - C₂-C₆-Alkoxy, welches durch ein Sauerstoffatom unterbrochen und unsubstituiert oder an der endständigen Alkoxygruppe terminal durch Hydroxy oder halogeniertes, insbesondere chloriertes C₁-C₃-Alkanoyl monosubstituiert ist,
      - C₁-C₃-Alkylthio oder
      - Hydroxy,
   - 2-Acetoxyethyl,
   - Cyclohexyl, welches unsubstituiert oder durch C₁-C₃-Alkyl mono- oder disubstituiert ist,
   - 1-Adamantylmethyl,
   - Benzyl oder einen
   - Heterocyclus der Gruppe Oxetanyl und Furyl, welcher über C₁-C₃-Alkyl gebunden und unsubstituiert oder durch Methyl substituiert ist,

   repräsentiert.
Gruppe Ir: Verbindungen der Formel I, worin X für -CH(OR₁)- und R₁ für Wasserstoff oder tert.-Butyldimethylsilyl steht; R₂ für Ethyl steht und R₃ C₁-C₅-Alkyl, 2,2,2-Tribromethyl, 2-Ethoxyethyl, 2-(2-Methoxyethoxy)-ethyl, 2-[2-(2-Hydroxyethoxy)-ethoxy]-ethyl, 2-{2-[2-(Chloracetoxy)-ethoxy]-ethoxy}-ethyl, 2-Methylthioethyl, 2-Hydroxyethyl, 2-Acetoxyethyl, Cyclohexyl, 2-Methyl-6-isopropylcylcohexyl,1-Adamantylmethyl, Benzyl, (3-Methyloxetan-3-yl)-methyl oder Furfuryl repräsentiert.
Gruppe Is: Verbindungen der Formel I, worin X für -CH(OR₁)- und R₁ für Wasserstoff steht; R₂ für Ethyl steht; und R₃ C₄-C₅-Alkyl, C₄-C₆-Cycloalkyl, über Methyl gebundenes C₄-C₆-Cycloalkyl, Phenyl, Benzyl oder a-Methylbenzyl repräsentiert. Als Vertreter dieser Gruppe sind insbesondere Milbemycin A₄-13- spiro-2'-[5'-(2"-methylbutoxy)-tetrahydrofuran] und Milbemycin A₄-13-spiro-2'-[5'-(1" -methyl-propoxy)-tetrahydrofuran von Interesse.
Gruppe It: Verbindungen der Formel I, worin X für -C(=N-OH)-steht, R₂ Methyl oder bevorzugt Ethyl bedeutet; und R₃ C₁-C₅-Alkyl, C₁-C₃-Alkyl, welches durch C₁-C₃-Alkoxy substituiert ist, oder C₃-C₇-Cycloalkyl repräsentiert.

Innerhalb der Gruppen la bis Im sind jene Vertreter der Formel I bevorzugt, worin R₂ für Methyl oder Ethyl, insbesondere für Ethyl steht.

Bevorzugte Einzelsubstanzen der Formel I sind beispielsweise:
Milbemycin A₄-13-spiro-2'-[5'-(2"-ethoxy-ethoxy)-tetrahydrofuran],
Milbemycin A₄-13-spiro-2'-[5'-(2",2"-dimethyl-propoxy)-tetrahydrofuran],
Milbemycin A₄-13-spiro-2'-[5'-cyclohexyloxy-tetrahydrofuran],
Milbemycin A₄-13-spiro-2'-[5'-benzyloxy-tetrahydrofuran],
Milbemycin A₄-13-spiro-2'-[5'-{2"-(2'''-ethoxy-ethoxy)-ethoxy}-tetrahydrofuran],
Milbemycin A₄-13-spiro-2'-[5'-{2"-(2'''-(hydroxymethoxy)-ethoxy)-ethoxy}-tetrahydrofuran],
Milbemycin A₄-13-spiro-2'-[5'-{2"-(2'''-(2""-(chloracetoxy)-ethoxy)-ethoxy)-ethoxy}-tetrahydrofuran],
Milbemycin A₄-13-spiro-2'-[5'-methoxy-tetrahydrofuran] und
Milbemycin A₄-13-spiro-2'-[5'-(2"-hydroxy-ethoxy)-tetrahydrofuran].

Weitere erwähnenswerte Einzelsubstanzen sind ferner auch
Milbemycin A₄-13-spiro-2'-[5'-(2"-methyl-butoxy)-tetrahydrofuran] und
Milbemycin A₄-13-spiro-2'-[5'-(1"-methyl-propoxy)-tetrahydrofuran].

Desgleichen sind auch die analogen in 5-Stellung durch eine 5-O-tert.-Butyldimethylsilylgruppe geschützten Vertreter der Formel I bevorzugt.

Die vorliegende Erfindung betrifft auch Verfahren, die es erlauben, in der 13-Position von Milbemycin-, 13-Deoxi-22,23-dihydro-Avermectinaglykon- oder 23-Deoxy-Antibiotika-S541-Derivaten einen zusätzlichen, spiro-verknüpften Tetrahydrofuranring gezielt einzuführen, um so zu hochwirksamen neuen Parasitiziden der Formel I zu gelangen. Die Erfindung betrifft auch verfahrensgemäss erhältliche Zwischenprodukte.

Im Umfang der Formel I liegen die Verbindungen der Formeln la, Ib und Ic 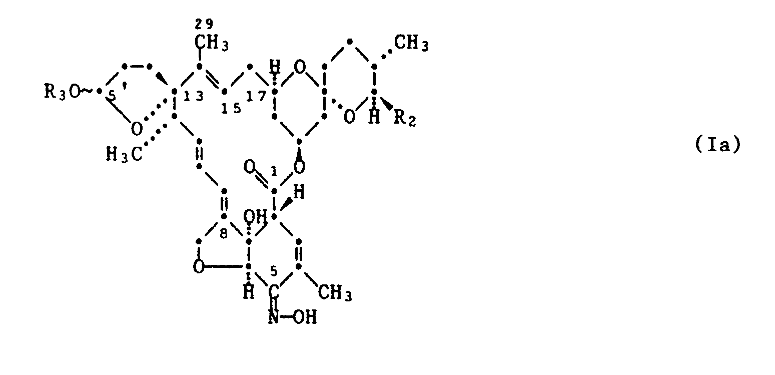 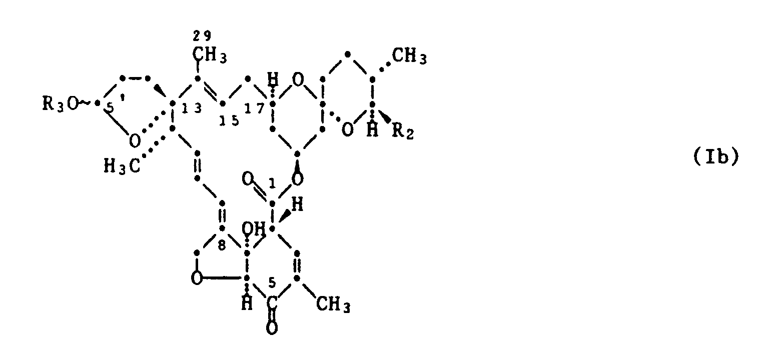 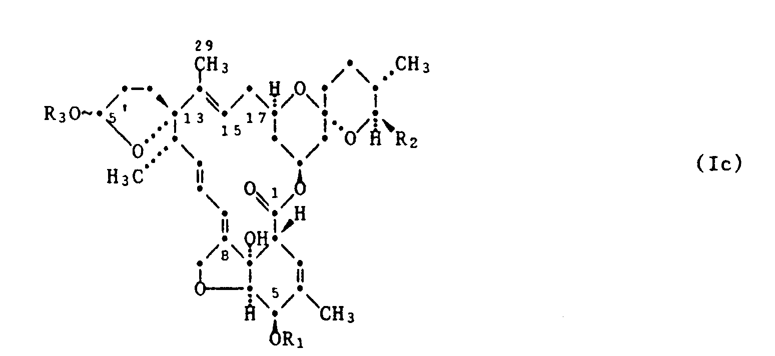 in denen Ri, R₂ und R₃ die für Formel I angegebenen Bedeutungen haben und die sich nach den nachstehend beschriebenen Methoden herstellen lassen.

Die Herstellung der Oxime [X = -C(=N-OH)-] im Umfang der Formel I, also von Verbindungen der Formel la, erfolgt durch Reaktion eines Derivats der Formel Ib mit Hydroxylamin oder einem seiner Salze, vorzugsweise einem seiner Mineralsäuresalze, insbesondere seinem Hydrochlorid. Die Reaktion wird zweckmässigerweise in einem geeigneten Lösungsmittel, z.B. einem Niederalkanol, wie Methanol, Ethanol, Propanol; einer etherartigen Verbindung, wie Tetrahydrofuran oder Dioxan; einer aliphatischen Carbonsäure, wie Essigsäure oder Propionsäure; Wasser oder in Gemischen dieser Lösungsmittel untereinander oder mit anderen üblichen reaktionsinerten Lösungsmitteln durchführt. Die Reaktionstemperaturen können in weiten Bereichen variieren. Man arbeitet zweckmässigerweise etwa im Bereich von +0° bis +100°C. Wird Hydroxylamin in Form eines seiner Salze, z.B. als Hydrochlorid eingesetzt, so ist es vorteilhaft, wenn man zum Abfangen der Säure (z.B. HCI) eine der für solche Zwecke üblichen Basen zusetzt und gegebenenfalls in Gegenwart eines Wasserbinders, z.B. eines Molekularsiebes, arbeitet. Als geeignete Basen kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide, Hydride und Hydroxide, Carbonate und Hydrogencarbonate von Alkali- und Erdalkalimetallen (CaO, BaO, NaOH, KOH, NaH, Ca(OH)₂, KHCO₃, NaHCO₃, Ca(HC0₃)₂, K₂C0₃, Na₂C0₃), sowie Alkaliacetate wie CH₃COONa oder CH₃COOK. Darüberhinaus eignen sich auch Alkalialkoholate wie C₂ H₅ ONa, n-C₃ H₇ ONa usw. Bevorzugt wird Triethylamin. Am vorteilhaftesten lässt sich die Oximierung mit Hydroxylaminhydrochlorid in Pyridin durchführen.

Die Derivate der Formel Ib lassen sich durch milde Oxidation z.B. mit Braunstein (Mn0₂), Cr0₃/Pyridin oder durch Oppenauer-Oxydation aus den entsprechenden freien 5-Hydroxi-derivaten der Formel Ic herstellen. Die Reaktion lässt sich in einem Lösungsmittel, wie beispielsweise einem Vertreter der ätherartigen Verbindungen oder der halogenierten Kohlenwasserstoffe oder Mischungen dieser Verbindungen untereinander, durchführen, besonders vorteilhaft in Dichlormethan.

Zur Herstellung von Verbindungen der Formel Ic wird erfindungsgemäss folgendes Verfahren durchgeführt, welches darin besteht, dass man eine Verbindung der Formel II in Gegenwart eines Säurekatalysators in einem inerten Lösungsmittel mit einer Verbindung der Formel III umsetzt: 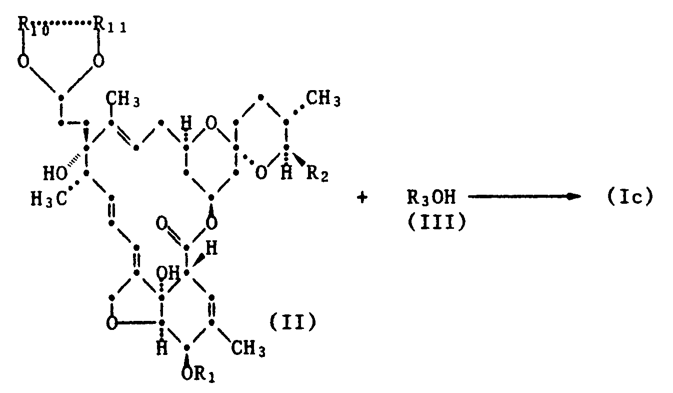 wobei die Substituenten Ri, R₂ und R₃ die unter Formel 1 angegebenen Bedeutungen haben und R₁₀ und R₁₁ unabhängig voneinander für C₁-C₆-Alkyl stehen oder eine C₂-C₁₀-Alkylenbrücke bilden, und gewünschtenfalls die so erhaltene Verbindung der Formel Ic 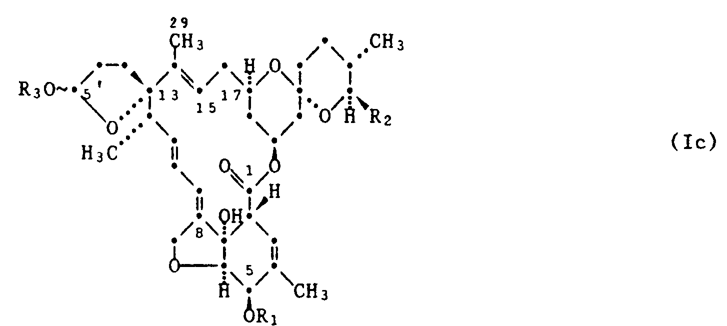 worin Ri, R₂ und R₃ die für Formel I angegebenen Bedeutungen haben, durch milde Oxydation in die entsprechende Verbindung der Formel Ib 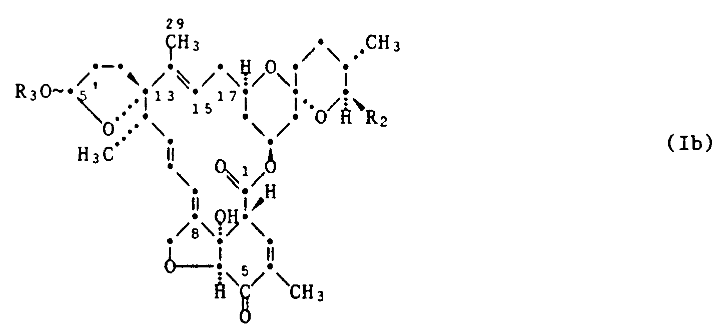 worin R₂ und R₃ die für Formel Ic angegebenen Bedeutungen haben, überführt und gewünschtenfalls die Verbindung der Formel Ib durch Umsetzung mit Hydroxylamin oder einem seiner Salze in die entsprechende Verbindung der Formel la 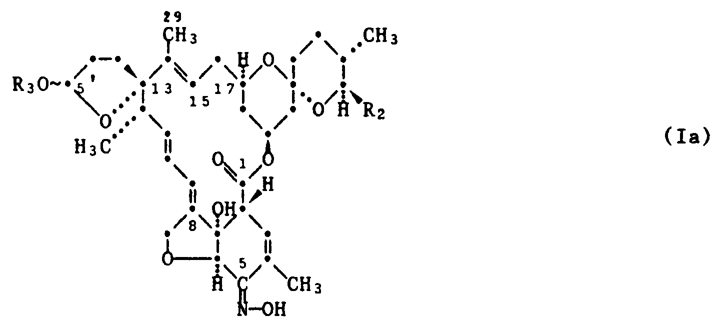 worin R₂ und R₃ die für Formel Ib angegebenen Bedeutungen haben, überführt.

Die Verbindungen der Formel II sind neu und haben Zwischenproduktecharakter. Auf Grund ihrer Struktur sind sie prädestiniert zur Herstellung der Wirkstoffe der Formel I. Die Verbindungen der Formel II sind daher ein Bestandteil vorliegender Erfindung. Für die Herstellung der erfindungsgemässen Verfahrensprodukte werden vorzugsweise solche Ausgangsstoffe der Formel II verwendet, welche zu den vorgängig als besonders bevorzugt geschilderten Verbindungen, insbesondere Einzelverbindungen der Formel I führen.

Die Reaktion zur Herstellung von Verbindungen der Formel Ic wird im allgemeinen bei Temperaturen von -30 bis +70°C, vorzugsweise von -10 _{°} bis +50°C durchgeführt. Man arbeitet dabei zweckmässigerweise in Gegenwart eines reaktionsinerten Lösungsmittels oder Lösungsmittelgemisches. Es eignen sich hierfür z.B. aliphatische und aromatische Kohlenwasserstoffe wie Benzol, Toluol, Xylole, Petrolether, Hexan; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; und Gemische solcher Lösungsmittel untereinander.

Die Verbindung der Formel III wird meist im Ueberschuss eingesetzt. Die Verbindungen der Formel III sind bekannt oder können analog bekannten Verfahren hergestellt werden.

Als Säurekatalysatoren eignen sich beispielsweise Carbonsäuren wie Oxalsäure und insbesondere Sulfonsäuren wie Methansulfonsäure, p-Toluolsulfonsäure, Campher-10-sulfonsäure bzw. deren Salze mit tert. Aminen wie z.B. Pyridinium-p-toluolsulfonat.

Verbindungen der Formel Ic werden dabei als Epimerengemisch bezüglich C5' erhalten. Die Epimeren lassen sich daraus durch eine physikalische Trennoperation in reiner Form herstellen. Als physikalische Trennoperationen eignen sich z.B. Säulenchromatographie, Flashchromatographie, Dickschichtchromatographie, HPLC und fraktionierte Kristallisation.

Verbindungen der Formel Ic können aber auch durch geeignete Reaktionen aus anderen Verbindungen der Formel Ic hergestellt werden.

Die Herstellung von Verbindungen der Formel II stellt ebenfalls einen Aspekt der vorliegenden Erfindung dar und erfolgt, indem man eine Verbindung der Formel IV in einem inerten Lösungsmittel mit einem Grignard-Reagenz der Formel V umsetzt: 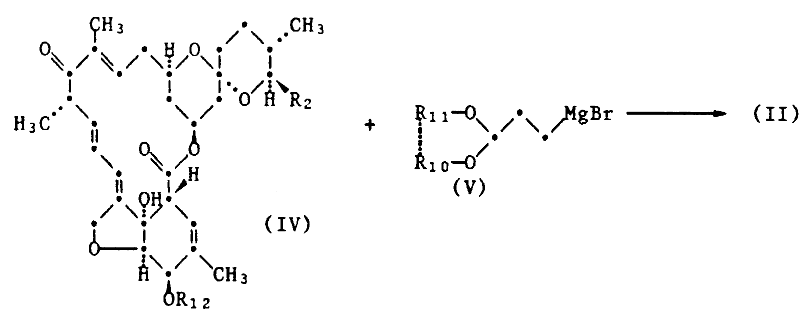 wobei R₂ wie unter Formel definiert ist, R₁₀ und R₁₁ unabhängig voneinander für C₁-C₆-Alkyl stehen oder zusammen eine C₂-C₁₀-Alkylenbrücke bilden und R₁₂ für Wasserstoff oder eine beispielsweise unter Formel I angegebene Silylgruppe steht.

Die Reaktion wird im allgemeinen bei Temperaturen von -80 bis + 70 _{°} C, vorzugsweise von -50 bis +50°C durchgeführt. Man arbeitet dabei zweckmässigerweise in Gegenwart eines reaktionsinerten Lösungsmittels oder Lösungsmittelgemisches. Es eignen sich hierfür z.B. aliphatische und aromatische Kohlenwasserstoffe wie Benzol, Toluol, Xylole, Petrolether, Hexan; Ether und etherartige Verbindungen wie Dialkylether (Diethylether, Diisopropylether, tert.-Butylmethylether usw.), Anisol, Dioxan, Tetrahydrofuran; und Gemische solcher Lösungsmittel untereinander.

Die Grignard-Reagenzien der Formel V können in Lösung durch Umsetzung der entsprechenden Bromide in einem der oben genannten Lösungsmittel mit Magnesium erhalten und direkt weiterverwendet werden, ohne dass sie vorher isoliert und gereinigt werden müssen.

Die Verbindungen der Formel IV sind bekannt oder lassen sich analog bekannten Methoden herstellen. Beispielsweise lassen sich Verbindungen der Formel IV nach dem in EP 180 539 und EP 184 989 beschriebenen Verfahren oder analog dazu herstellen, indem man eine Verbindung der Formel VI 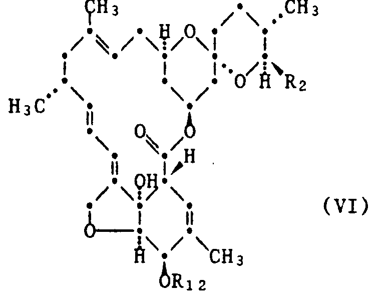 wobei R₂ wie für Formel 1 und R₁₂ wie für Formel IV definiert sind, in einer ersten Stufe mit Persäuren in die 14,15-Epoxide der Formel VII überführt: 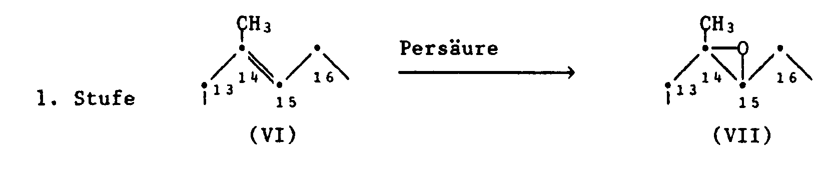 und die 14,15-Epoxide der Formel VII anschliessend mit Hilfe eines speziellen Komplexreagenz zu 15-Hydroxi-Verbindungen der Formel VIII umsetzt: 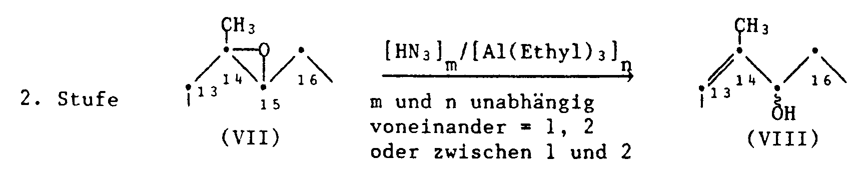 In einer weiteren Stufe werden dann die 15-Hydroxi-Verbindungen der Formel VIII mit Chromat-, Halochromat- oder Dichromat-Ionen, insbesondere mit Pyridiniumdichromat umgesetzt, wobei als Ausgangsverbindung der Formel VIII vorzugsweise eine solche eingesetzt wird, bei welcher die 5-OH-Gruppe geschützt ist und beispielsweise als 5-Silyloxygruppe vorliegen kann: 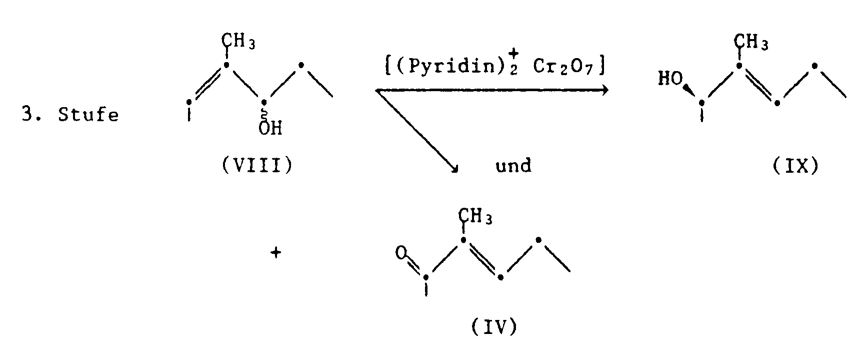 wobei ausser den erwünschten 13-Hydroxi-milbemycinen der Formel IX auch 13-Oxo-Verbindungen entstehen, die sich nach bekannten Methoden voneinander trennen lassen.

Die meisten der beschriebenen Reaktionen werden vorteilhafterweise unter Schutzgas, wie z.B. Stickstoff oder Argon durchgeführt.

Die Verbindungen der Formel VI sind bekannt oder lassen sich aus den bekannten Verbindungen analog bekannten Verfahren herstellen.

Die 13-Oxo-Derivate der Formel IV lassen sich ferner gezielt aus den 13ß-Hydroxyderivaten der Formel IX durch Oxydation mit Dimethylsulfoxid (DMSO)/Oxalylchlorid herstellen. Die Reaktion wird vorzugsweise bei Temperaturen von -60 _{°} C bis Raumtemperatur durchgeführt. Als Lösungsmittel kommen DMSO selbst sowie aromatische Kohlenwasserstoffe wie Benzol, Toluol, Xylole und chlorierte Kohlenwasserstoffe, wie beispielsweise Dichlormethan in Frage. Man arbeitet vorzugsweise unter Zugabe einer Base, wie beispielsweise Triethylamin.

Zu den bekannten Verbindungen zählen die Milbemycine der Formel M:

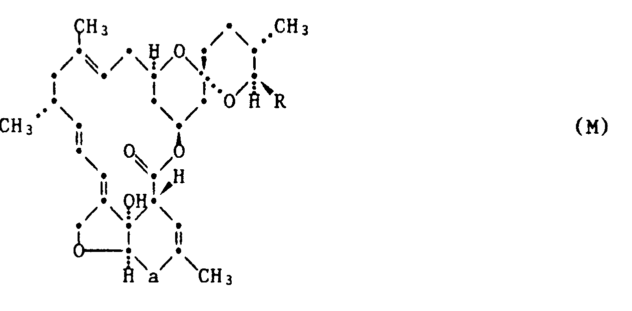

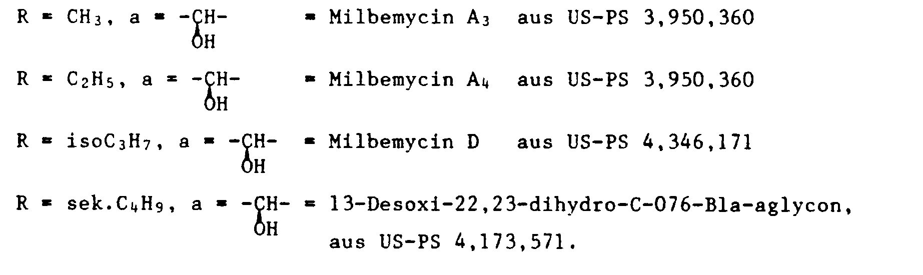

Verbindungen, worin R sek.Butyl darstellt, sollen hier und im folgenden gleichfalls zu den Milbemycin-Derivaten gerechnet werden, obwohl sie nach der üblichen Systematik von Avermectin-Derivaten abgeleitet sind. Avermectin-Aglykone (mit einer OH-Gruppe in 13-Position) lassen sich jedoch gemäss US-PS 4,173,571 in Milbemycin-Homologe überführen.

Die Konstitution von natürlichen Antibiotika S541 ist aus der DE-OS-35 32 794 bekannt und sieht wie folgt aus:

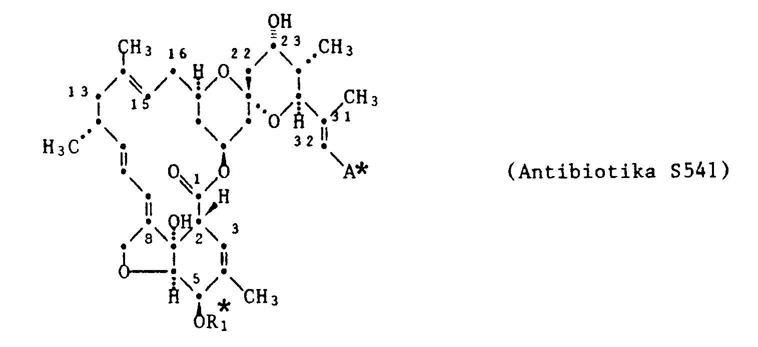

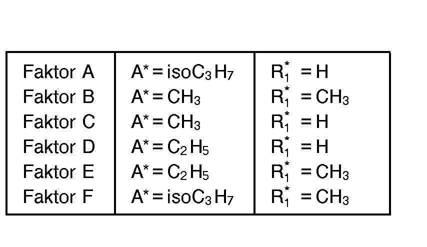

Je nach Faktor werden im folgenden zur Vereinfachung der Benennung die Abkömmlinge von Antibiotikum S541 als Derivate von S541 A, S541 B, S541 C, S541 D, S541 E oder S541 F klassifiziert.

Verbindungen der Formel VI, worin R₂ für die Gruppe 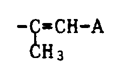 steht und A die für Formel VI angegebene Bedeutung hat, welche als Ausgangsprodukte in dem erfindungsgemässen Verfahren verwendet werden können, lassen sich auf an sich bekannte Weise aus den natürlichen Antibiotika S541 herstellen.

Die in 23-Position befindliche Hydroxygruppe in den Antibiotika S541 lässt sich analog der in US-PS 4,328,335 beschriebenen Methode entfernen und die Antibiotika S541 lassen sich so in die entsprechenden 23-Deoxi-Derivate überführen. Dabei müssen diejenigen Verbindungen mit einer freien 5-OH-Gruppe (R; = H) zunächst selektiv geschützt werden durch Reaktion mit einem der nachstehend angegebenen Silylierungsreagenzien Y-Si(R₅)(R₆)(R₇) bzw. mit tert.-Butyldimethylsilyloxiacetylchlorid. Die Umsetzung dieser geschützten Verbindungen, in denen R; durch Si(R₅)R₆)(R₇) bzw. C(=O)CH₂OSi(CH₃)₂t-C₄H₉ ersetzt und das 23-C-Atom durch OH substituiert ist, mit p-Methylphenyl-chlorthionoformiat ergibt Derivate der Antibiotika S541, welche in Position 23 durch p-CH₃-C₆H₄-O-C( = S)-O-substituiert sind. Diese 23-O-(4-Methylphenoxi)-thiocarbonylderivate von Antibiotika S541 werden dann mit Tributylzinnhydrid in Toluol in Gegenwart von Azobisisobutyronitril bei 80-120 °C zu den entsprechenden 23-Deoxi-Derivaten (Position 23 unsubstituiert) reduziert.

Durch Silylierung oder Acylierung der 5-OH-Gruppe werden alle jene Derivate der Formeln I, II und IV hergestellt, bei denen R₁ eine andere Bedeutung als Wasserstoff (R₁ = OH-Schutzgruppe) hat. Zur Silylierung verwendet man zweckmässigerweise ein Silan der Formel Y-Si(R₅)(R₆)(R₇), wobei Rs, R₆ und R₇ vorzugsweise unabhängig voneinander für C₁-C₆-Alkyl, Benzyl oder Phenyl stehen und zusammen mit dem Siliciumatom beispielsweise eine der Gruppen Trimethylsilyl, tris(tert.Butyl)silyl, Thexyldimethylsilyl, Diphenyl-tert.butylsilyl, bis(lsopropyl)methylsilyl, Triphenylsilyl und insbesondere tert.Butyl-dimethylsilyl bilden. Y bedeutet eine Silylabgangsgruppe, wozu beispielsweise Brom, Chlor, Cyan, Azido, Acetamid, Trifluoracetoxy, Trifluormethansulfonyloxy zählen. Diese Aufzählung stellt keine Limitierung dar, der Fachmann kennt weitere typische Silylabgangsgruppen. Die 5-OH-Gruppe kann auch als Benzylether oder Methoxiethoximethylether vorliegen.

Die Einführung der Acylgruppe erfolgt üblicherweise mit den entsprechenden Acylhalogeniden oder Acylanhydriden und wird vorzugsweise zur Einführung der eingangs definierten R₄-C(0)-Gruppe benützt. Unter den Acylhalogeniden sind die Chloride und Bromide bevorzugt. 5-O-Silylierungen und 5-O-Acylierungen werden in wasserfreiem Milieu, vorzugsweise in inerten Lösungsmitteln und besonders bevorzugt in aprotischen Lösungsmitteln durchgeführt. Die Reaktion läuft vorteilhaft im Temperaturbereich von 0 bis + 80 _{°} C, bevorzugt bei + 10 bis + 40 _{°} C, ab. Vorzugsweise wird eine organische Base zugegeben. Es kommen als solche beispielsweise tertiäre Amine wie Triethylamin, Triethylendiamin, Triazol und bevorzugt Pyridin, Imidazol oder 1,8-Diazabicyclo[5.4.0]-undec-7-en (DBU) in Frage.

Die Entfernung dieser Silylreste R₁ in der 5-Position geschieht durch selektive milde Hydrolyse (→ R₁ = H) mit z.B. Arylsulfonsäure in alkoholischer Lösung, HF in Acetonitril, HFₓ• Pyridin in Tetrahydrofuran oder nach einer anderen dem Fachmann geläufigen Methode.

Das beschriebene Verfahren zur Herstellung der Verbindungen der Formel 1 ist in allen Teilschritten ein Bestandteil vorliegender Erfindung.

Die Verbindungen der Formel I eignen sich ausgezeichnet zur Bekämpfung von Schädlingen an Tieren und Pflanzen, darunter insbesondere von tierparasitären Ekto-Parasiten. Zu letzteren zählen unter der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haematopinidae); unter der Ordnung Diptera insbesondere Schädlinge der Familien Muscidae, Calliphoridae, Oestridae, Tabanidae, Hippoboscidae, Gastrophilidae.

Die Verbindungen der Formel I sind auch einsetzbar gegen Hygiene-Schädlinge, insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen der Formel I besitzen auch nachhaltige Wirksamkeit gegen pflanzenparasitäre Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Coccidae, Diaspididae und Eriophydidae (z.B. die Rostmilbe auf Citrusfrüchten): Der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizid gegen Schädlinge im Erdboden geeignet.

Die Verbindungen der Formel sind daher gegen alle Entwicklungsstadien saugender und fressender Insekten an Kulturen von Nutzpflanzen wie Getreide, Baumwolle, Reis, Mais, Soja, Kartoffeln, Gemüse, Früchten, Tabak, Hopfen, Citrus, Avocados und anderen wirksam.

Die Verbindungen der Formel sind auch wirksam gegen Pflanzen-Nematoden der Gattungen Meloidogyne, Heterodera, Pratylenchus, Ditylenchus, Radopholus, Rhizoglyphus und andere.

Ferner sind die Verbindungen gegen Helminthen in allen Entwicklungsstadien wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris und Parascaris. Der besondere Vorteil der Verbindungen der Formel 1 ist ihre Wirksamkeit gegen solche Parasiten, die gegen Wirkstoffe auf Benzimidazol-Basis resistent sind.

Gewisse Spezies der Gattungen Nematodirus, Cooperia und Oesophagostomum greifen den Intestinaltrakt des Wirtstiers an, während andere der Gattungen Haemonchus und Ostertagia im Magen und solche der Gattung Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel sind gegen diese Parasiten hoch wirksam.

Weiter sind die Verbindungen der Formel 1 zur Bekämpfung von humanpathogenen Parasiten geeignet, unter denen als typische, im Verdauungstrakt vorkommende Vertreter solche der Gattungen Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Gattungen Wuchereria, Brugia, Onchocerca und Loa aus der Familie der Filariidae, die im Blut, im Gewebe und verschiedenen Organen vorkommen, ferner gegen Dracunculus und Parasiten der Gattungen Strongyloides und Trichinella, die speziell den Gastro-Intestinaltrakt infizieren.

Die Verbindungen der Formel werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formel werden bei Warmblütern in Aufwandmengen von 0,01 bis 10 mg/kg Körpergewicht dosiert. Ueber geschlossenen Kultur-Anbauflächen werden sie zweckmässigerweise in Mengen von 10 g bis 1000 g pro Hektar angewendet. Sie kommen ferner in Pferchen, Gehegen, Stallungen oder sonstigen Räumen zur Anwendung.

Die Formulierungen, d.h. die den Wirkstoff der Formel 1 enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen C₈ bis C₁₂, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon,Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali- oder Erdalkalisalze oder gegebenenfalls substituierte Ammoniumsalze von höheren Fettsäuren (C₁ₒ-C₂₂), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgender Publikation beschrieben: "1986 International Mc Cutcheon's Emulsifiers and Detergents"

The Manufacturing Confectioner Publishing Co.,

Glen Rock, New Jersey, USA.

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als Wirkstoff mindestens eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

### Herstellungsbeispiele

### Herstellung von Zwischenprodukten

### A1. Herstellung von 5-O-tert.-Butyldimethylsilyl-13β-[2-(1,3-dioxolan-2-yl)äthyl]-13α-hydroxy-milbemycin A₄

Zu einer Suspension von 650 mg Magnesiumspänen in 20 ml Tetrahydrofuran (THF) wurde innert 2 1/2 Std. bei 40°C eine Lösung von 2,40 ml 2-(2-Bromäthyl)-1,3-dioxolan in 10 ml THF gegeben. Um die Bildung des Grignard-Reagenz zu starten, wurden zu Beginn der Umsetzung wenige Kristalle Jod zugegeben. Das Reaktionsgemisch wurde anschliessend noch weitere 30 Min. bei 40 °C unter Argon-Atmosphäre gerührt und dann dekantiert. Man erhielt so eine 0,2 M Lösung von 2-(1,3-Dioxolan-2-yl)-äthylmagnesiumbromid in THF.

Eine Lösung von 1,006 g 5-O-tert.-Butyldimethylsilyl-13-oxo-milbemycin A₄ in 8 ml THF wurde auf -15°C abgekühlt und dann gab man innerhalb von 30 Min. 17,0 ml der 0,2 M Lösung von 2-(1,3-Dioxolan-2-yl)äthylmagnesiumbromid in THF zu. Nach 30 Min. Rühren bei -15°C wurden vorsichtig 5 ml gesättigte NH₄CI-Lösung zugegeben, dann das Reaktionsgemisch auf 100 ml gesättigte NaHCO₃-Lösung gegossen und dreimal mit 150 ml Aether extrahiert. Die organischen Phasen wurden mit 100 ml gesättigter NaCI-Lösung gewaschen, mit MgS0₄ getrocknet und eingedampft. Chromatographie des Rohprodukts an Kieselgel mit Hexan/ Dimethoxyäthan 6:1 ergab neben 87 mg (8 %) des C(13)-Epimeren 969 mg (84 %) Produkt.
Massenspektrum (MS): m/e: 772 (M⁺, C₄₃H₆₈O₁₀Si)
¹H-NMR (300 MHz, CDCl₃):
   3,05 ppm (dt, J_{d} = 2,5, Jₜ = 9) (C2sH)
   4,42 ppm (bs, wt = 11) (Cs H)
   4,85 ppm (t, J = 3) (OCH(CH₂)O)

### A2. Herstellung von 13ß-[2-(1,3-Dioxolan-2-yl)äthyl]-13a-hydroxy-milbemycin A₄

Eine Lösung von 40 mg 5-O-tert.-Butyldimethylsilyl-13β-[2-(1,3-dioxolan-2-yl)äthyl]-13a-hydroxy-milbemycin A₄ in 1 ml einer HFₓ• Pyridin/THF-Lösung (hergestellt aus 6,5 ml HFₓ• Pyridin, 15,7 ml Pyridin und 50 ml THF) wurde 18 Std. bei Raumtemperatur gerührt. Das Reaktionsgemisch wurde dann auf 50 ml gesättigte NaHCO₃-Lösung gegossen und mit 100 ml Diäthyläther extrahiert. Die organische Phase wurde mit 50 ml gesättigter NaCI-Lösung gewaschen, mit MgS0₄ getrocknet und eingedampft. Nach Chromatographie des Rohprodukts an Kieselgel mit Hexan/Essigsäureäthylester 1:1 erhielt man 27 mg (79 %) Produkt.
MS: (m/e): 658 (M⁺, C₃₇H₅₄O₁₀)
¹H-NMR (300 MHz, CDCl₃):
   3,07 ppm (dt, J_{d} = 2,5, Jₜ = 9) (C₂₅ H)
   4,28 ppm (t, J = 7) (Cs H)
   4,85 ppm (t, J = 4) (OCH(CH₂)O)

### A3. Herstellung von 5-O-tert.-Butyldimethylsilyl-13β-[2-(1,3-dioxolan-2-yl)äthyl]-13α-hydroxy-milbemycin A₃

Analog Beispiel A1 erhält man aus 420 mg 5-O-tert.-Butyldimethylsilyl-13-oxo-milbemycin A₃ und 7,0 ml einer 0,2 M Lösung von 2-(1,3-Dioxolan-2-yl)äthylmagnesiumbromid die Titelverbindung. Massenspektrum (MS): (m/e): 758 (M⁺, C₄₂H₆₆O₁₀Si)

### A4. Herstellung von 13ß-[2-(1,3-Dioxolan-2-yl)äthyl]-13a-hydroxy-milbemycin A₃

Die Titelverbindung lässt sich analog Beispiel A2 aus 5-O-tert.-Butyldimethylsilyl-13β-[2-(1,3-dioxolan-2-yl)äthyl]-13a-hydroxy-milbemycin A₃ herstellen.
Massenspektrum (MS): (m/e): 644 (M⁺, C₃₆H₅₂O₁₀)
Herstellung von Verbindungen der Formel 1

### H1: Herstellung von Milbemycin A₄-13-spiro-2'-[5'-methoxy-tetrahydrofuran]

Eine Lösung von 50 mg 5-O-tert.-Butyldimethylsilyl-13β-[2-(1,3-dioxolan-2-yl)äthyl]-13α-hydroxy-milbemycin A₄ in 1 ml einer 1 % Lösung von p-Toluolsulfonsäure in Methanol wurde 90 Min. bei Raumtemperatur gerührt. Das Reaktionsgemisch wurde dann auf 50 ml gesättigte NaHCO₃-Lösung gegossen und mit 100 ml Diäthyläther extrahiert. Die organische Phase wurde mit 50 ml gesättigter NaCI-Lösung gewaschen, mit MgS0₄ getrocknet und eingedampft. Nach Chromatographie des Rohprodukts an Kieselgel mit Hexan/Essigsäureäthylester 2:1 konnten 35 mg (86 %) Produkt als Epimerengemisch an C5' (Isomer A:Isomer B ca. 3:1), isoliert werden.
MS: (m/e): 628 (M⁺, C₃₆H₅₂O₉)
¹H-NMR (300 MHz, CDCl₃):
   3,09 ppm (dt. J_{d} = 2,5, Jₜ = 9) (C₂₅H)
   3,40* und 3,45 ppm (2s) (CH₃0)
   4,03 und 4,12* ppm (2s) (OH)
   4,29 ppm (6s, w 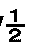 = 15) (Cs H)
   4,99* ppm (d, J = 4) und 5,10 ppm (dd, J = 4, J' = 2) (OCH(CH₂)O).

### H2: Herstellung von 5-0-tert.-Butyldimethylsilyl-milbemycin A4-13-spiro-2'-[5'-(2"-äthoxy-äthoxy)-tetrahydrofuran]

Zu einer Lösung von 80 mg 5-O-tert.-Butyldimethylsilyl-13ß-[2-(1,3-dioxolan-2-yl)äthyl]-13a-hydroxy- milbemycin A₄ und 200 µl Aethylenglykol-monoäthyl-äther in 2 ml Methylenchlorid wurden 24 mg (±)-Campher-10-sulfonsäure gegeben. Nach 2 Std. Rühren bei Raumtemperatur wurde das Reaktionsgemisch auf 50 ml gesättigte NaHCO₃-Lösung gegossen und mit 100 ml Diäthyläther extrahiert. Die organische Phase wurde mit 50 ml gesättigter NaCI-Lösung gewaschen, mit MgS0₄ getrocknet und eingedampft. Die Chromatographie des Rohprodukts an Kieselgel mit Hexan/Essigsäureäthylester 6:1 ergab 62 mg (50 %) Isomer A-Produkt und 32 mg (26 %) Isomer B-Produkt.

### Isomer A:

MS: (m/e): 800 (M⁺, C₄₅H₇₂O₁₀Si)
¹H-NMR (300 MHz, CDCl₃):
   3,08 ppm (dt, J_{d} = 2,5, Jₜ = 9) (C₂₅ H)
   4,09 ppm (s) (OH)
   4,42 ppm (bs, w 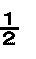 = 10) (Cs H)
   5,13 ppm (bs, w 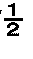 = 6) (OCH(CH₂)O)

### Isomer B:

MS: (m/e): 800 (M⁺, C₄₅H₇₂O₁₀Si)
¹H-NMR (300 MHz, CDCl₃):
   3,07 ppm (dt, J_{d} = 2,5, Jₜ = 9) (C₂₅H)
   4,03 ppm (s) (OH)
   4,42 ppm (bs, wt = 10) (Cs H)
   5,23 ppm (bd, J = 3) (OCH(CH₂)0)
   5,51 ppm (dd, J = 11, J' = 6) (C₁₅ H)

### H3: Herstellung von Milbemycin A₄-13-spiro-2'-[5'-(2"-äthoxy-äthoxy)-tetrahydrofuran]

### a) Isomer A

Eine Lösung von 58 mg 5-O-tert.-Butyldimethylsilyl-13-spiro-2'-[5'-(2"-äthoxy-äthoxy)-tetrahydrofuran] (Isomer A) in 1 ml HFₓ• Pyridin/THF-Lösung (vgl. oben) wurde 16 Std. bei Raumtemperatur gerührt. Das Reaktionsgemisch wurde dann auf 50 ml gesättigte NaHCO₃-Lösung gegossen und mit 100 ml Diäthyläther extrahiert. Die organische Phase wurde mit 50 ml gesättigter NaCI-Lösung gewaschen, mit MgSO₃ getrocknet und eingedampft. Chromatographie des Roprodukts an Kieselgel mit Hexan/Essigsäureäthylester 2:1 lieferte 47 mg (94 %) Produkt.
MS: (m/e): 686 (M⁺, C₃₉H₅₈O₁₀)
1 H-NMR (300 MHz, CDCl₃):
   3,08 ppm (dt, J_{d} = 2,5, Jₜ = 9) (C₂₅ H)
   4,10 ppm (s) (OH)
   4,28 ppm (t, J = 7) (Cs H)
   5,13 ppm (bd, J = 4) (OCH(CH₂)O)

### b) Isomer B

Aus 29,5 mg 5-O-tert.-Butyldimethylsilyl-13-spiro-2'-[5'-(2"-äthoxy-äthoxy)-tetrahydrofuran] (Isomer B) erhielt man analog zu Vorschrift H3.a. 22 mg (88 %) Produkt.
MS: (m/e): 686 (M⁺, C₃₉H₅₈O₁₀)
¹H-NMR (300 MHz, CDCl₃):
   3,06 ppm (dt, J_{d} = 2,5, Jₜ = 9) (C₂₅H)
   4,03 ppm (s) (OH)
   4,28 ppm (t, J = 7) (Cs H)
   5,22 ppm (bd, J = 4) (OCH(CH₂)0)
   5,50 ppm (dd, J = 11, J' = 5) (C₁₅H)

### H4: Herstellung von 5-O-tert.-Butyldimethylsilyl-milbemycin A4-13-spiro-2'-[5'-(2",2"-dimethyl-propoxy)-tetrahydrofuran]

Zu einer Lösung von 120 mg 5-O-tert.-Butyldimethylsilyl-13β-[2-(1,3-dioxolan-2-yl)äthyl]-13α-hydroxy- milbemycin A₄ und 274 mg Neopentylalkohol in 2 ml Methylenchlorid wurden 36 mg (±)-Campher-10-sulfonsäure gegeben. Nach 2 Std. Rühren bei Raumtemperatur wurde das Reaktionsgemisch, wie unter Herstellungsbeispiel H2 beschrieben, aufgearbeitet. Nach Chromatographie des Rohprodukts an Kieselgel mit Hexan/Diäthyläther 5:1 konnten 64 mg (52 %) Isomer A-Produkt und 35 mg (28 %) Isomer-B-Produkt isoliert werden.

### Isomer A:

MS: (m/e): 798 (M⁺, C₄₆H₇₄O₉Si)
¹H-NMR (300 MHz, CDCl₃):
   0,88 ppm (s) (C(CH₃)₃)
   2,96 ppm (d, J = 9) (OCHHC(CH₃)₃)
   3,08 ppm (dt, J_{d} = 2,5, Jₜ = 9) (C₂₅H)
   3,50 ppm (d, J = 9) (OCHHC(CH₃)₃)
   4,09 ppm (s) (OH)
   4,42 ppm (bs, w 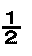 = 10) (C₅ H)
   4,07 ppm (bd, J = 3) (OCH(CH₂)O)

### Isomer B:

MS: (m/e): 798 (M⁺, C₄₆H₇₄O₉Si)
¹H-NMR (300 MHz, CDCl₃):
   0,88 ppm (s) (C(CH₃)₃)
   3,02 ppm (dt, J_{d} = 2,5, Jₜ = 9) (C₂₅H)
   3,06 ppm (d, J = 9) (OCHHC(CH₃)₃)
   3,51 ppm (d, J = 9) (OCHHC(CH₃)₃)
   4,11 ppm (s) (OH)
   4,42 ppm (bs, w 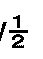 = 10) (C₅ H)
   5,14 ppm (dd, J = 5, J' = 2,5) (OCH(CH₂)O)
   5,56 ppm (t, J = 7,5) (C₁₅H)

### H5: Herstellung von Milbemycin A₄-13-spiro-2'-[5'-(2",2"-dimethylpropoxy)-tetrahydrofuran]

### a) Isomer A

Aus 61 mg 5-O-tert.-Butyldimethylsilyl-milbemycin A₄-13-spiro-2'-[5'-(2",2"-dimethyl-propoxy)-tetrahydrofuran] (Isomer A) erhielt man analog Herstellungsbeispiel H3 47 mg (90 %) Produkt.
MS: (m/e): 684 (M⁺, C₄₀H₆₀O₉)
¹H-NMR (300 MHz, CDCl₃):
   0,87 ppm (s) (C(CH₃)₃)
   2,95 ppm (d, J = 9) (OCHHC(CH₃)₃)
   3,07 ppm (dt, J_{d} = 2,5 Jₜ = 9) (C₂₅H)
   3,40 ppm (d, J = 9) (OCHHC(CH₃)₃)
   4,11 ppm (s) (OH)
   4,28 ppm (t, J = 7) (Cs H)
   5,06 ppm (bs, w 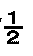 = 6) (OCH(CH₂)O)

### b) Isomer B

Aus 29 mg 5-O-tert.-Butyldimethylsilyl-milbemycin A₄-13-spiro-2'-[5'-(2",2"-dimethyl-propoxy)-tetrahydrofuran] (Isomer B) konnten analog Herstellungsbeispiel H3 22 mg (85 %) Produkt gewonnen werden.
MS: (m/e): 684 (M⁺, C₄₀H₆₀O₉)
¹H-NMR (300 MHz, CDCl₃):
   0,88 ppm (s) (C(CH₃)₃)
   3,01 ppm (dt, J_{d} = 2,5, Jₜ = 9) (C₂₅H)
   3,05 ppm (d, J = 9) (OCHHC(CH₃)₃)
   3,50 ppm (d, J = 9) (OCHHC(CH₃)₃)
   4,09 ppm (s) (OH)
   4,28 ppm (t, J = 7) (Cs H)
   5,15 ppm (dd, J = 5,m J' = 2,5) (OCH(CH₂)O)
   5,55 ppm (t, J = 8) (C_{1 5} H)

### H6: Herstellung von 5-O-tert.-Butyldimethylsilyl-milbemycin A4-13-spiro-2'-[5'-(2"-(2'-(2""-hydroxyäthoxy)-äthoxy)-äthoxy)-tetrahydrofuran]

Zu einer Lösung von 120 mg 5-O-tert.Butyldimethylsilyl-13β-[2-(1,3-dioxolan-2-yl)äthyl]-13α-hydroxy- milbemycin A₄ und 828 µl Triäthylenglykol in 2 ml Methylenchlorid wurden 36 mg (±)-Campher-10-sulfonsäure gegeben. Nach 2 Std. Rühren bei Raumtemperatur wurde das Reaktionsgemisch, wie unter Herstellungsbeispiel H2 beschrieben, aufgearbeitet. Chromatographie des Rohprodukts an Kieselgel mit Hexan/Essigsäureäthylester 1:1 ergab 96 mg (72 %) Produkt, als Epimerengemisch an C5' (Isomer A:Isomer B ca. 3:1).
MS: (m/e): 860 (M⁺, C₄₇H₇₆0₁₂Si)
¹H-NMR (300 MHz, CDCl₃):
   3,08 ppm (dt, J_{d} = 2,5, Jₜ = 9) (C₂₅ H)
   4,02 ppm und 4,10* ppm (2s) (OH)
   4,42 ppm (bs, w 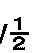 = 10) (Cs H) 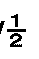
   5,12* ppm (bs, w= 6) und 5,16 ppm (dd, J = 5, J' = 2) (OCH(CH₂)O)

### H7: Herstellung von Milbemycin A₄-13-spiro-2'-[5'-(2"-(2'(2""-hydroxyäthoxy)-äthoxy)-äthoxy)-tetrahydrofuran]

Aus 50 mg 5-O-tert.-Butyldimethylsilyl-milbemycin A₄-13-spiro-2'-[5'-(2"-(2 (2""-hydroxyäthoxy)-ät- hoxy)-äthoxy)-tetrahydrofuran] erhielt man analog Herstellungsbeispiel H3 39 mg (90 %) Produkt, als Epimerengemisch an C5' (Isomer A: Isomer B ca. 2,5:1).
MS: (m/e): 746 (M⁺, C₄₁ H₆₂ O₁₂)
¹H-NMR (300 MHz, CDCl₃):
   3,07 ppm (bt, J = 9) (C₂₅ H)
   4,00 ppm und 4,10* ppm (2s) (OH)
   4,27 ppm (t, J = 7) (Cs H)
   5,11* ppm (bt, J = 2) und 5,22 ppm (dd, J = 5, J' = 2) (OCH(CH₂)O)

### H8: Herstellung von 5-O-tert.-Butyldimethylsilyl-milbemycin A4-13-spiro-2'-[5'-(2"-(2'-(2""-(chloracetoxy)-ät- hoxy)-äthoxy)-äthoxy)-tetrahydrofuran]

Zu einer Lösung von 42 mg 5-O-tert.-Butyldimethylsilyl-milbemycin A₄-13-spiro-2'-[5'-(2"-(2'(2""-hy- droxyäthoxy)-äthoxy)-äthoxy)-tetrahydrofuran] [Epimerengemish an C5' (Isomer A:Isomer B ca. 3:1)] und 39 µl Pyridin in 2 ml Methylenchlorid wurden bei 0°C 1 µl Chloracetylchlorid gegeben. Nach 6 Std. Rühren bei 0°C wurde das Reaktionsgemisch auf 50 ml 1 N HCI-Lösung gegeben und mit 100 ml Diäthyläther extrahiert. Die organische Phase wurde mit je 50 ml gesättigter NaHCO₃- und gesättigter NaCI-Lösung gewaschen, mit MgS0₄ getrocknet und eingeengt. Die Chromatographie des Rohprodukts an Kieselgel mit Hexan/Essigsäureäthylester 3:1 ergab 42 mg (92 %) Produkt, als Epimerengemisch an C5' (Isomer A:Isomer B ca. 3:1).
MS: (m/e): 936 (M⁺, C₄₉H₇₇ClO₁₃Si)
¹H-NMR (300 MHz, CDCl₃):
   3,07 ppm (dt, J_{d} = 2,5, Jₜ = 9) (C₂₅H)
   4,00 ppm und 4,08* ppm (2s) (OH)
   4,08 ppm (s) (CH₂CI)
   4,41 ppm (bs, w 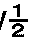 = 10) (C₅H)
   5,10* ppm (bt, J = 3) und 5,22 ppm (dd, J = 5, J' = 2,5) (OCH(CH₂)O)

H9: Herstellung von Milbemycin A₄-13-spiro-2'-[5'-(2"-(2'-(2""-(chloracetoxy)-äthoxy)-äthoxy)-äthoxy)-tetrahydrofuran]

Aus 39 mg 5-O-tert.-Butyldimethylsilyl-milbemycin A₄-13-spiro-2'-[5'-(2"-(2'-(2""-(chloracetoxy)-äthoxy)-äthoxy)-äthoxy)-tetrahydrofuran] [Epimerengemisch an C5' (Isomer A:Isomer B ca. 3:1)] erhielt man analog Herstellungsbeispiel H3 26 mg (75%) Produkt, als Epimerengemisch an C5' (Isomer A:Isomer B ca. 3:1).
MS: (m/e): 822 (M⁺, C₄₃H₆₃ClO₁₃)
¹H-NMR (300 MHz, CDCl₃):
   3,07 ppm (dt, J_{d} = 2,5, Jₜ = 9) (C₂₅H)
   4,00 ppm und 4,10* ppm (2s) (OH)
   4,08 ppm (s) (CH₂CI)
   4,27 ppm (t, J = 7) (Cs H)
   5,11* ppm (bt, J = 2,5) und 5,22 ppm (m) (OCH(CH₂)O)

Nach den vorstehend beschriebenen Verfahren lassen sich beispielsweise auch folgende Verbindungen der Formel I herstellen:

### H10: 5-O-tert.-Butyldimethylsilyl-milbemycin A₄-13-spiro-2'-[5'-benzyloxy-tetrahydrofuran]

### a) Isomer A:

MS: (m/e): 818 (M⁺, C₄₈H₇₀O₉Si)
¹H-NMR (300 MHz, CDCl₃):
   3,07 ppm (dt, J_{d} = 2,5, Jₜ = 9) (C₂₅H)
   4,09 ppm (s) (OH)
   4,42 pm (bs, w 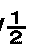 = 11) (Cs H)
   4,48 ppm (d, J = 11,5) (OCHHC₆ H₅ )
   4,82 ppm (d, J = 11,5) (OCHHC₆H₅)
   5,20 ppm (bt, J = 2,5) (OCH(CH₂)O)
   7.33 ppm (m) (C₆ H₅)

### b) Isomer B

MS: (m/e): 818 (M⁺, C₄₈H₇₀O₉Si)
¹H-NMR (300 MHz, CDCl₃):
   3,00 ppm (dt, J_{d} = 2,5, Jₜ = 9) (C₂₅H)
   4,12 ppm (s) (OH)
   4,41 ppm (bs, w 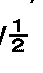 = 10) (C₅ H)
   4,57 ppm (d, J = 11,5) (OCHHC₆ H₅)
   4,86 ppm (d, J = 11,5) (OCHHC₆ H₅)
   5,54 ppm (dd, J = 10,J' = 5) (C₁₅ H)
   7,32 ppm (m) (C₆ H₅)

### H11: Milbemycin A₄-13-spiro-2'-[5'-benzyloxy-tetrahydrofuran]

### a) Isomer A:

MS: (m/e): 704 (M⁺, Ca ₂ Hs s Os )
¹H-NMR (300 MHz, CDCl₃):
   3,07 ppm (dt, J_{d} = 2,5, Jₜ = 9) (C₂₅H)
   4,10 ppm (s) (OH)
   4,28 ppm (t, J = 7) (Cs H)
   4,48 ppm (d, J = 11,5) (OCHHC₆ H₅)
   4,82 ppm (d, J = 11,5) (OCHHC₆ H₅)
   5,20 ppm (bt, J = 2,5) (OCH(CH₂)O)
   7,33 ppm (m) (C₆ H₅)

### b) Isomer B

MS: (m/e): 704 (M⁺, C₄₂H₅₆O₉)
¹H-NMR (300 MHz, CDCl₃):
   3,00 ppm (dt, J_{d} = 2,5, Jₜ = 9) (C₂₅H)
   4,10 ppm (s) (OH)
   4,27 ppm (t, J = 7) (Cs H)
   4,57 ppm (d, J = 11,5) (OCHHC₆ H₅)
   4,85 ppm (d, J = 11,5) (OCHHC₆ H₅)
   5,30 ppm (bt, J = 2,5) (OCH(CH₂)O)
   7,31 ppm (m) (C6 Hs)

### H12: 5-O-tert.-Butyldimethylsilyl-milbemycin A₄-13-spiro-2'-[5'-cyclohexyloxy-tetrahydrofuran]

### a) Isomer A:

MS: (m/e): 810 (M⁺, C₄₇H₇₄O₉Si)
¹H-NMR (300 MHz, CDCl₃):
   3,07 ppm (dt, J_{d} = 2,5, Jₜ = 9) (C₂₅ H)
   3,60 ppm (m) (OCH(CH₂)CH₂)
   4,09 ppm (s) (OH)
   4,41 ppm (bs, w 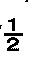 = 10) (C₅ H)

### b) Isomer B

MS: (m/e): 810 (M⁺, C₄₇H₇₄O₉Si)
¹H-NMR (300 MHz, CDCl₃):
   3,01 ppm (dt, J_{d} = 2,5, Jₜ = 9) (C₂₅H)
   3,62 ppm (m) (OCH(CH₂)CH₂)
   4,08 ppm (s) (OH)
   4,40 ppm (bs, w 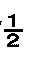 = 10) (C₅ H)
   5,55 ppm (bs, J = 8) (C₁₅H)

### H13: Milbemycin A₄-13-spiro-2'-[5'-cyclohexyloxy-tetrahydrofuran]

### a) Isomer A:

MS: (m/e): 696 (M⁺, C₄₁ H₆₀O₉)
¹H-NMR (300 MHz, CDCl₃):
   3,08 ppm (dt, J_{d} = 2,5, Jₜ = 9) (C₂₅ H)
   3,62 ppm (m) (OCH(CH₂)CH₂)
   4,10 ppm (s) (OH)
   4,28 ppm (t, J = 7) (Cs H)

### b) Isomer B

MS: (m/e): 696 (M⁺, C₄₁ H₆₀O₉)
¹H-NMR (300 MHz, CDCl₃):
   3,02 ppm (dt, J_{d} = 2,5, Jₜ = 9) (C₂₅H)
   3,62 ppm (m) (OCH(CH₂)CH₂)
   4,07 ppm (s) (OH)
   4,27 ppm (t, J = 7) (Cs H)
   5,56 ppm (dd, J = 10, J' = 5) (C₁₅H)

### H14: 5-O-tert.-Butyldimethylsilyl-milbemycin A₄-13-spiro-2'-[5'-2"-(2"'-methoxy-äthoxy)-äthoxy)-tetrahydrofuran]

### Epimerengemisch an C5' (Isomer A:Isomer B ca. 3:1)

MS: (m/e): 830 (M⁺, C₄₆H₇₄O₁₁ Si)
¹H-NMR (300 MHz, CDCl₃):
   3,08 ppm (bt, J = 9) (C₂₅ H)
   3,36 ppm (s) (CH₃0)
   4,02 und 4,09* ppm (2s) (OH)
   4,41 ppm (bs,w 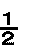 = 11) (C₅H)
   5,12* ppm (bs, w 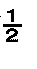 = 6) und 5,23 ppm (bs, w 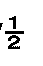 = 6) (OCH(CH₂)O)

### H15: Milbemycin A₄-13-spiro-2'-[5'-(2"-(2"'-methoxy-äthoxy)-äthoxy)-tetrahydrofuran]

### Epimerengemisch an C5' (Isomer A:Isomer B ca. 2:3)

MS: (m/e): 716 (M⁺, C₄₀H₆₀O₁₁)
¹H-NMR (300 MHz, CDCl₃):
   3,08 ppm (dt, J_{d} = 2,5, Jₜ = 9) (C₂₅H)
   3,38 ppm (s) (CH₃0)
   4,01* und 4,10 ppm (2s) (OH)
   4,29 ppm (t, J = 7) (Cs H)
   5,12 ppm (bs, w 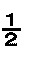 = 7) und 4,23* ppm (bs, w 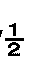 = 6) (OCH(CH₂)O)

### H16: 5-O-tert.-Butyldimethylsilyl-milbemycin A₄-13-spiro-2'-[5'-((3"-methyl-oxetan-3"-yl)-methoxy)-tetrahydrofuran]

### Epimerengemisch an C5' (Isomer A:Isomer B ca. 1:1)

MS: (m/e): 812 (M⁺, C₄₆H₇₂O₁₀Si)
¹H-NMR (300 MHz, CDCl₃):
   3,02 und 3,07 ppm (2dt, J_{d} = 2,5, Jₜ = 9) (C₂₅H)
   3,40 und 3,47 ppm (2d, J = 10) (OCHHC)
   3,87 und 3,88 ppm (2d, J = 10) (OCHHC)
   4,09 und 4,10 ppm (2s) (OH)
   5,12 ppm (bd, J = 3,5) und 5,20 ppm (dd, J = 4,5, J' = 2) (OCH(CH₂)O)

### H17: Milbemycin A₄-13-spiro-2'-[5'-((3"-methyl-oxetan-3"-yl)-methoxy)-tetrahydrofuran]

### Epimerengemisch an C5' (Isomer A:Isomer B ca. 1:1)

MS: (m/e): 698 (M⁺, C₄₀H₅₈O₁₀)
¹H-NMR (300 MHz, CDCl₃):
   3,02 und 3,07 ppm (2dt, J_{d} = 2,5, Jₜ = 9) (C₂₅H)
   3,40 und 3,46 ppm (2d, J = 10) (OCHHC)
   3,86 und 3,88 ppm (2d, J = 10) (OCHHC)
   4,09 und 4,10 ppm (2s) (OH)
   4,28 ppm (t, J = 7) (Cs H)
   5,12 ppm (bd, J = 3,5) und 5,20 ppm (dd, J = 5, J' = 2) (OCH(CH₂)O)

### H18: 5-tert.-Butyldimethylsilyl-milbemycin A₄-13-spiro-2'-[5'-(2"-hydroxy-äthoxy)-tetrahydrofuran]

### Epimerengemisch an C5' (Isomer A:Isomer B ca. 1:1)

MS: (m/e): 772 (M⁺, C₄₃H₆₈O₁₀Si)
¹H-NMR (300 MHz, CDCl₃):
   3,07 ppm (dt, J_{d} = 2,5, Jₜ = 9) (C₂₅ H)
   3,98 und 4,06* ppm (2s) (OH)
   4,41 ppm (bs, w 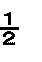 = 11) (C₅H)
   5,13* ppm (d, J = 4,5) und 5,22 ppm (dd, J = 5, J' = 3) (OCH(CH₂)O)

### H19: Milbemycin A₄-13-spiro-2'-[5'-(2"-hydroxy-äthoxy)-tetrahydrofuran]

### a) Isomer A

MS: (m/e): 658 (M⁺, C₃₇H₅₄O₁₀)
¹H-NMR (300 MHz, CDCl₃):
   3,07 ppm (dt, J_{d} = 2,5, Jₜ = 9) (C₂₅ H)
   4,06 ppm (s) (OH)
   4,27 ppm (t, J = 7) (Cs H)
   5,13 ppm (d, J = 4,5) (OCH(CH₂)O)

### b) Isomer B:

MS: (m/e): 658 (M⁺, C₃₇H₅₄O₁₀)
¹H-NMR (300 MHz, CDCl₃):
   3,07 ppm (dt, J_{d} = 2,5, Jₜ = 9) (C₂₅H)
   3,98 ppm (s) (OH)
   4,27 ppm (t, J = 7) (Cs H)
   5,22 ppm (dd, J = 5, J' = 3) (OCH(CH₂)0)
   5,53 ppm (dd, J = 10,5, J' = 4,5) (C₁₅H)

### H20: Herstellung von 5-Oximino-milbemycin A₄-13-spiro-2'-[5'-(2",2"-dimethyl-propoxy)-tetrahydrofuran]

a) Eine Lösung von 50 mg Milbemycin A₄-13-spiro-2'-[5'-(2",2"-dimethyl-propoxy)-tetrahydrofuran] in 3 ml Dichlormethan wird mit 95 mg Mangandioxid während 5 Stunden bei Raumtemperatur gerührt. Das Mangandioxid wird über Kieselgur abfiltriert und man erhält nach dem Einengen der Lösung 47 mg rohe 5-Oxo-milbemycin A4-13-spiro-2'-[5'-(2",2"-dimethyl-propoxy)-tetrahydrofuran].
   MS: (m/e): 682 (M⁺, C₄₀H₅₈O₉)
b) Dieses Rohprodukt wird zusammen mit 47 mg Hydroxylaminhydrochlorid in 1,0 ml Pyridin gelöst. Nach 1 Std. Rühren bei Raumtemperatur wird mit Diäthyläther und 1 N HCI aufgearbeitet. Die Chromatographie des Rohproduktes an Kieselgel mit Essigsäureäthylester/Hexan 1:3 ergibt 35 mg der Titelverbindung.
   MS: (m/e): 697 (M⁺, C₄₀H₅₉NO₉)
   ¹H-NMR (300 MHz, CDCl₃):
   0,88 ppm (s) (C(CH₃)₃)
   2,96 ppm (d, J = 9,5) (OCHHC(CH₃)₃)
   3,08 ppm (dt, J_{d} = 2,5, Jₜ = 9,5) (C₂₅H)
   3,48 ppm (d, J = 9,5) (OCHHC(CH₃)₃)
   4,64 ppm (s) (C₆ H)
   7,62 ppm (s) (N-OH)

### H21: Herstellung von 5-Oximino-milbemycin A₄-13-spiro-2'-[5'-cyclohexyloxy-tetrahydrofuran]

a) Eine Lösung von 30 mg Milbemycin A₄-13-spiro-2'-[5'-cyclohexyloxytetrahydrofuran] in 3 ml Dichlormethan wird mit 56 mg Mangandioxid versetzt. Nach 2 Std. Rühren bei Raumtemperatur wird das Reaktionsgemisch über Kieselgur filtriert. Nach dem Einengen der Lösung erhält man 30 mg rohes 5-Oxo-milbemycin A₄-13-spiro-2'-[5'cyclohexyloxy-tetrahydrofuran].
   MS: (m/e): 694 (M⁺, C₄₁ H₅₈ O₉)
b) 29 mg dieses Rohproduktes und 30 mg Hydroxylaminhydrochlorid in 1,0 ml Pyridin werden 2 Std. bei Raumtemperatur gerührt. Das Reaktionsgemisch wird mit Diäthyläther und 1 N HCI aufgearbeitet und man erhält nach Chromatographie des Rohproduktes an Kieselgel mit Essigsäureäthylester/Hexan 1:3 23 mg der Titelverbindung.
   MS: (m/e): 709 (M⁺, C₄₁H₅₉NO₉)
   ¹H-NMR (300 MHz, CDCl₃):
      3,09 ppm (dt, J_{d} = 2,5, Jₜ = 9,5) (C₂₅H)
      3,62 ppm (m) (OCH(CH₂)CH₂)
      4,55 ppm (s) (C₆ H)
      7,65 ppm (s) (N-OH)

### H22: Herstellung von 5-Oximino-milbemycin A₄-13-spiro-2'-[5'-(2"-methyl-butoxy)-tetrahydrofuran]

a) Eine Lösung von 30 mg Milbemycin A₄-13-spiro-2'[5'-(2"-methylbutoxy)-tetrahydrofuran] in 3 ml Dichlormethan wird mit 57 mg Mangandioxid versetzt. Nach 2 Std. Rühren bei Raumtemperatur wird das Reaktionsgemisch über Kieselgur filtriert und man erhält nach dem Einengen der Lösung 27 mg rohes 5-Oxo-milbemycin A₄-13-spiro-2'-[5'-(2"-methyl-butoxy)-tetrahydrofuran].
   MS: (m/e): 682 (M⁺, C₄₀H₅₈O₉)
b) 26 mg dieses Rohprodukts werden zusammen mit 26 mg Hydroxylaminhydrochlorid in 1,0 ml Pyridin gelöst. Nach 90 min. Rühren bei Raumtemperatur wird mit Diäthyläther und 1 N HCI aufgearbeitet. Die Chromatographie des Rohprodukts an Kieselgel mit Essigsäureäthylester/Hexan 1:3 liefert 21 mg der Titelverbindung.
   MS: (m/e): 697 (M⁺, C₄₀H₅₉NO₉)
   ¹H-NMR (300 MHz, CDCl₃):
      3,10 ppm (dd, J₁ = 6, J₂ = 9,5) (OCHHCH(CH₃)C₂H₅)
      3,66 ppm (dd, J₁ = 6, J₂ = 9,5) (OCHHCH(CH₃)C₂H₅)
      4,64 ppm (s) (C₆ H)
      7,68 ppm (s) (N-OH)

### H23: Herstellung von 5-Oximino-milbemycin A₃-13-spiro-2'-[5'-(2"-äthoxy-äthoxy)-tetrahydrofuran]

a) Eine Lösung von 14,5 mg Milbemycin A₃-13-spiro-2'-[5'-(2"-äthoxy-äthoxy)-tetrahydrofuran] in 3 ml Dichlormethan wird mit 60 mg Mangandioxid versetzt. Nach 90 Minuten Rühren bei Raumtemperatur wird das Reaktionsgemisch über Kieselgur filtriert und man erhält nach dem Einengen der Lösung 14 mg rohes 5-Oxo-milbemycin A₃-13-spiro-2'-[5'-(2"-äthoxy-äthoxy)-tetrahydrofuran].
   MS: (m/e): 670 (M⁺, C₃₈H₅₄O₁₀)
b) Dieses Rohprodukt wird zusammen mit 20 mg Hydroxylaminhydrochlorid in 1,0 ml Pyridin gelöst. Nach 1 Std. Rühren bei Raumtemperatur wird mit Diäthyläther und 1 N HCI aufgearbeitet. Die Chromatographie des Rohprodukts an Kieselgel mit Essigsäureäthylester/Hexan 1:3 ergibt 10 mg der Titelverbindung.
   MS: (m/e): 685 (M⁺, C₃₈H₅₅NO₁₀)

Analog zu den beschriebenen Arbeitsweisen werden auch die nachfolgend genannten Verbindungen hergestellt:

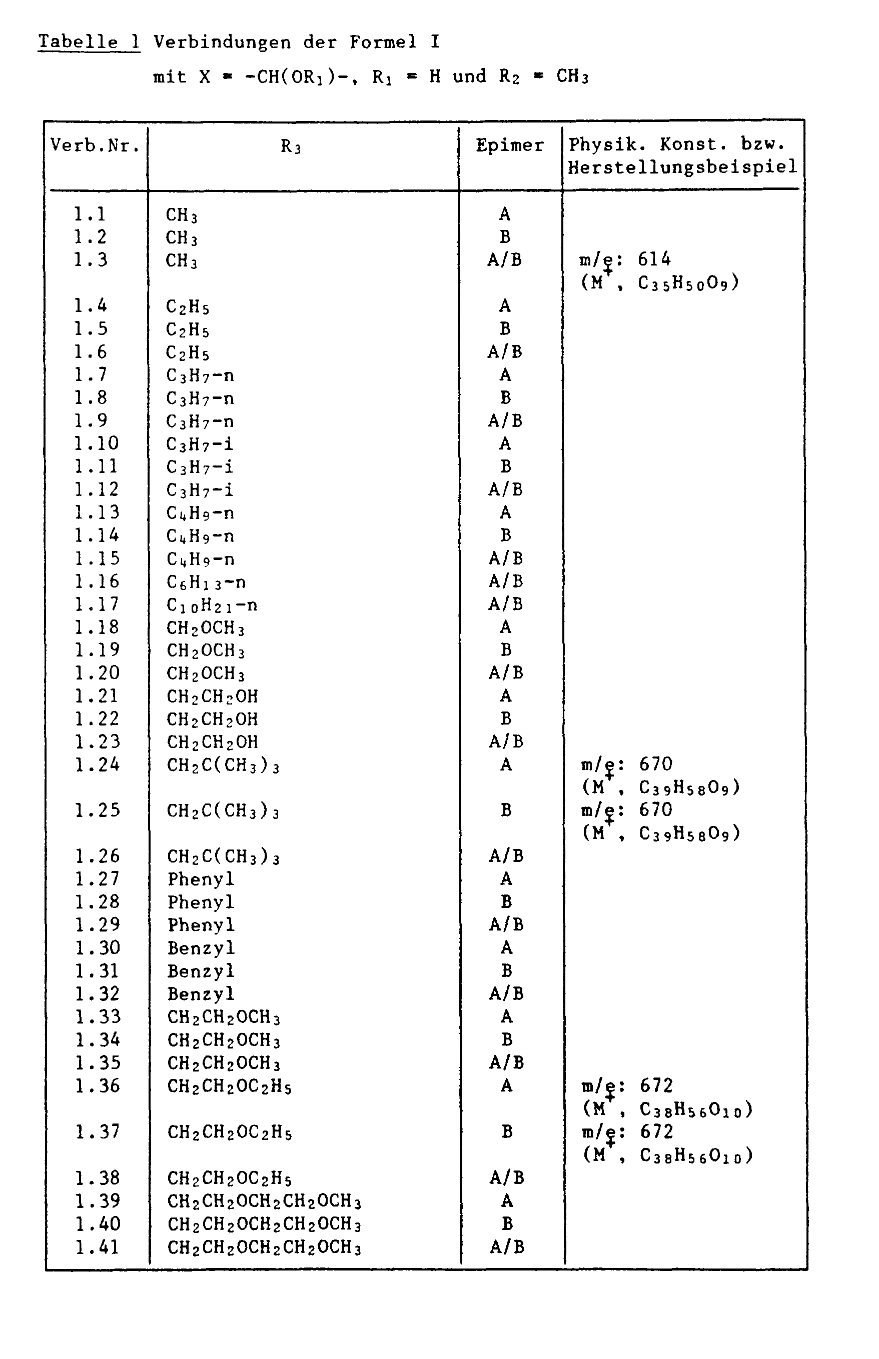

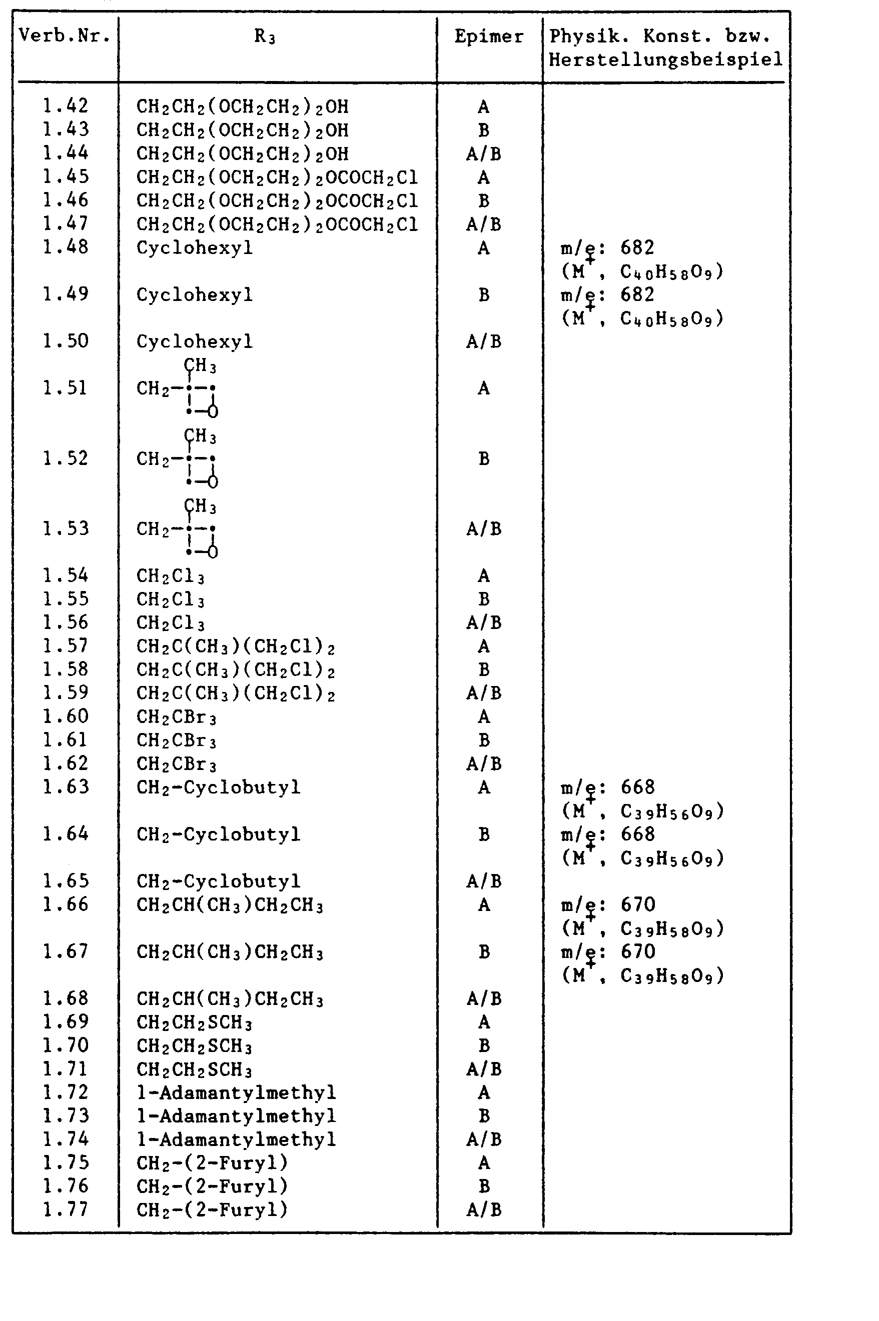

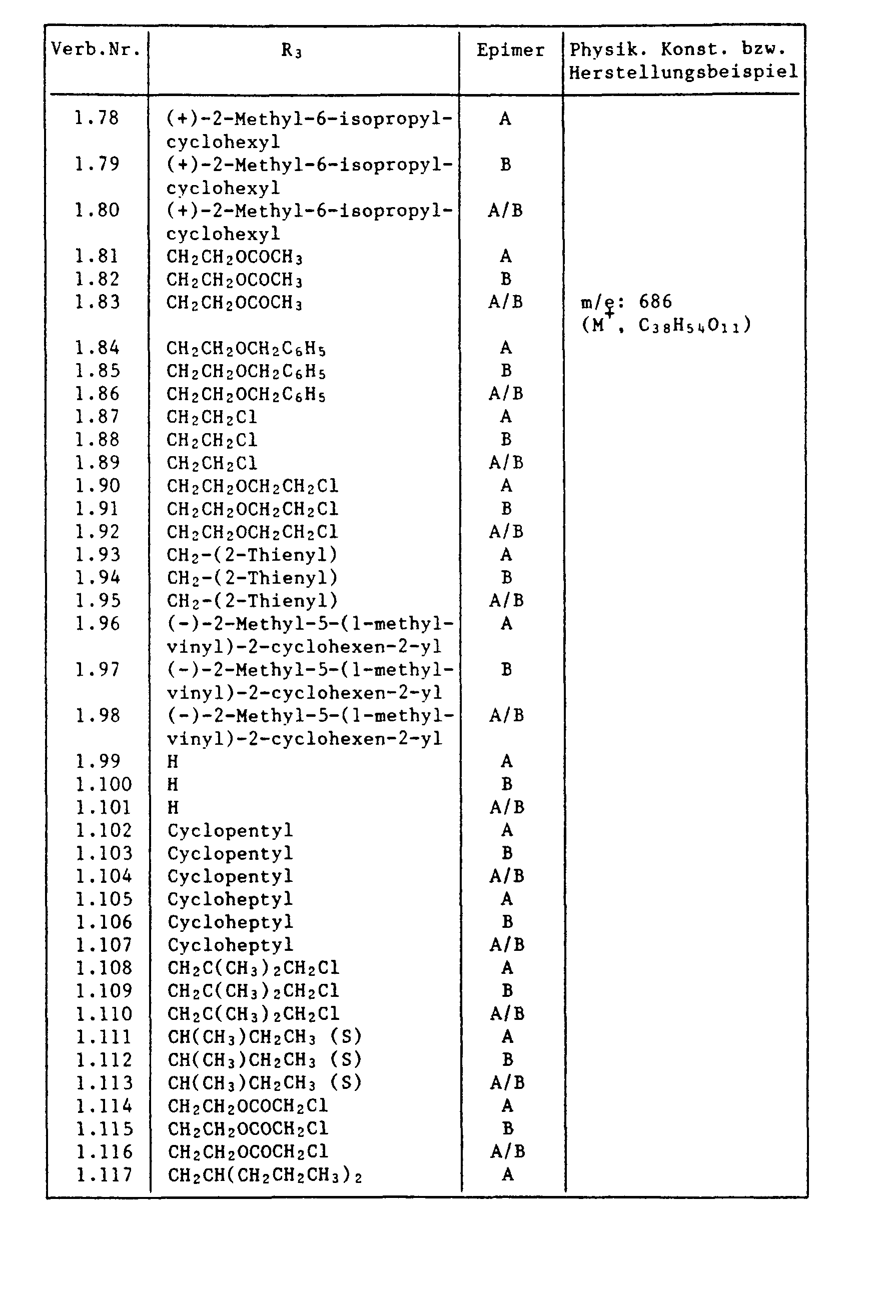

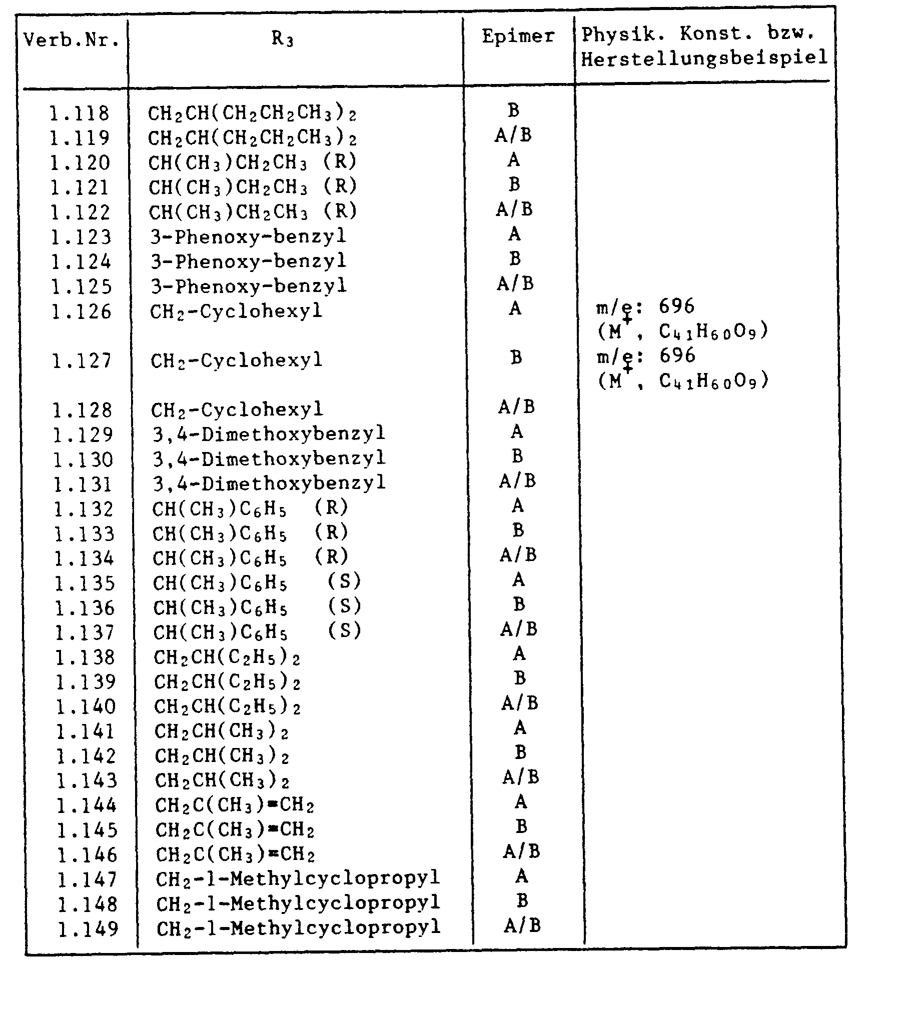

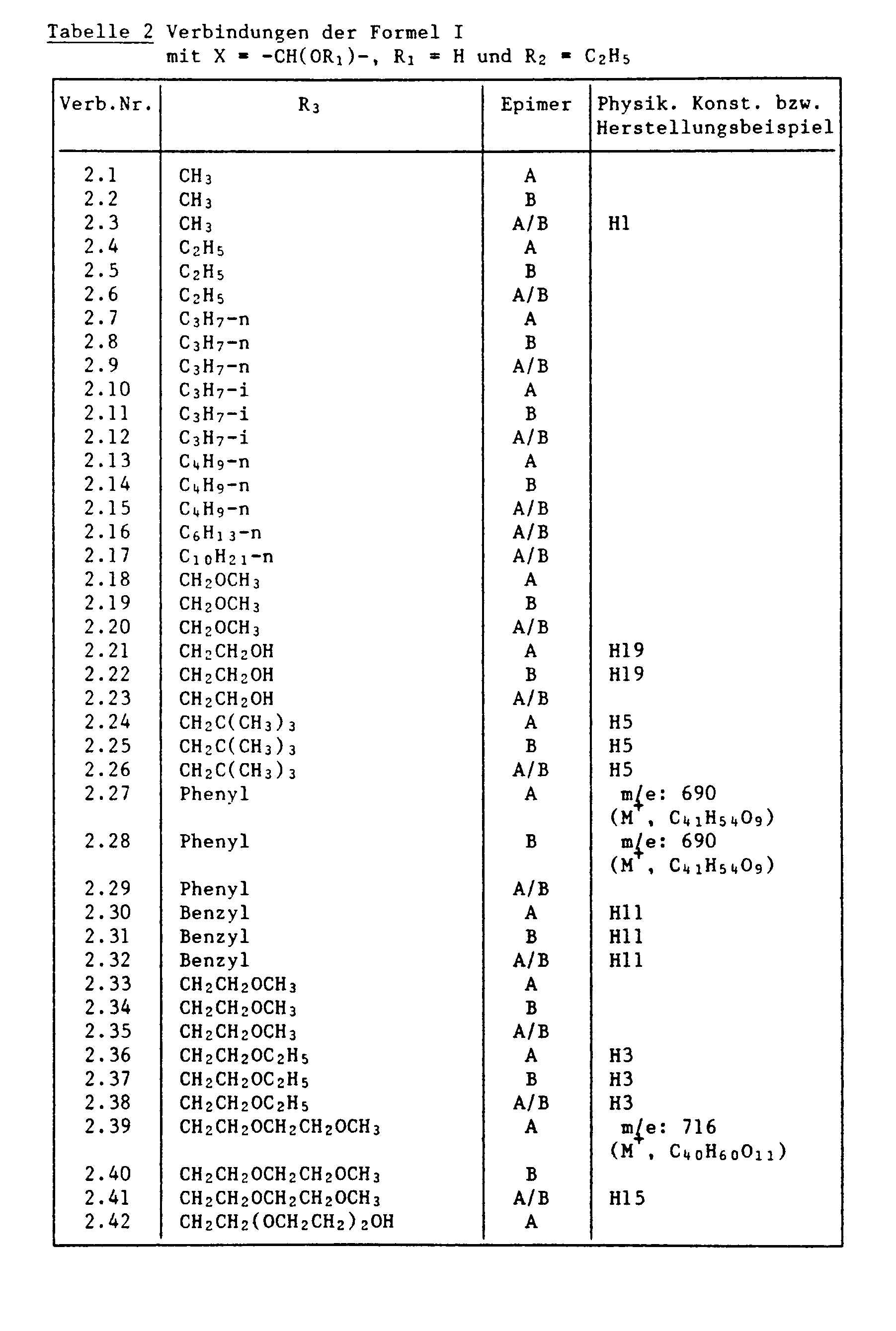

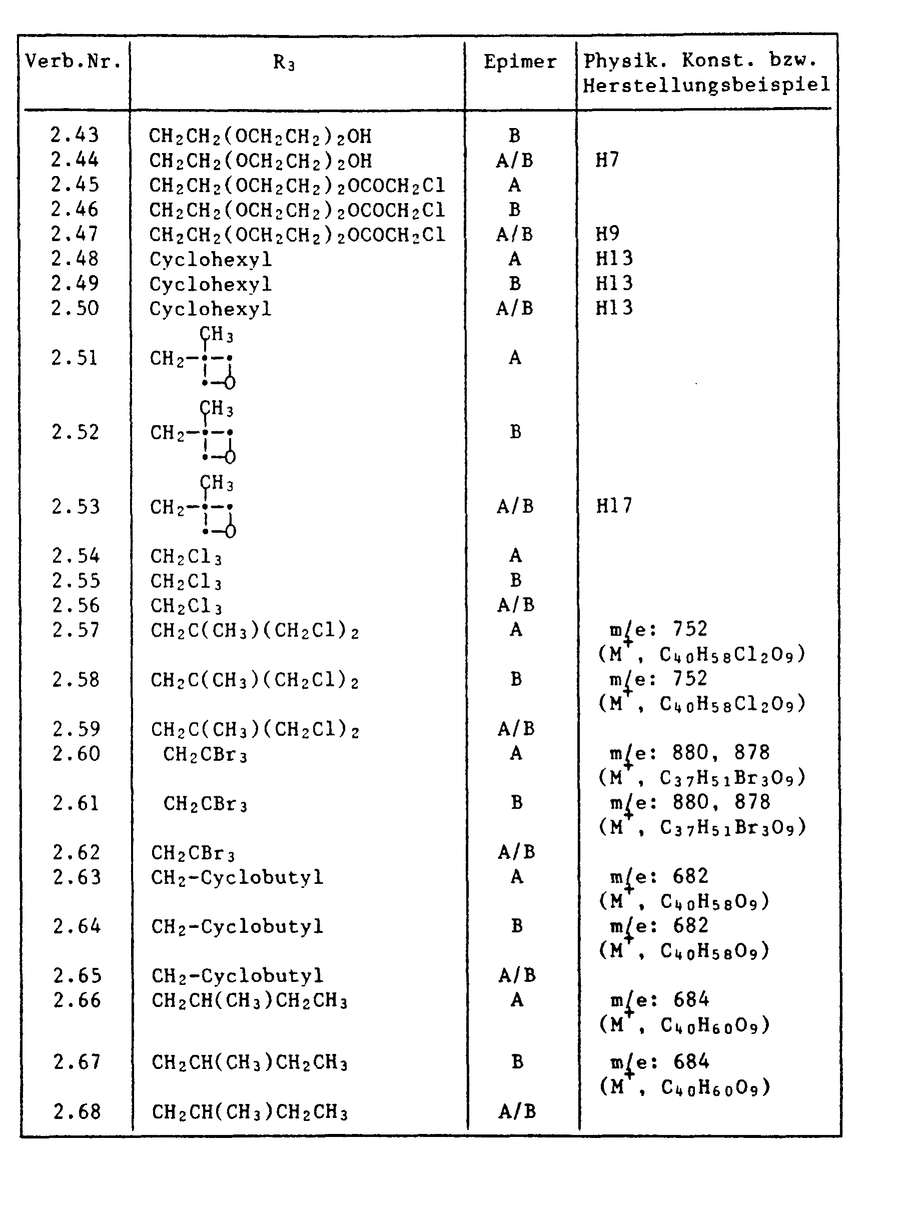

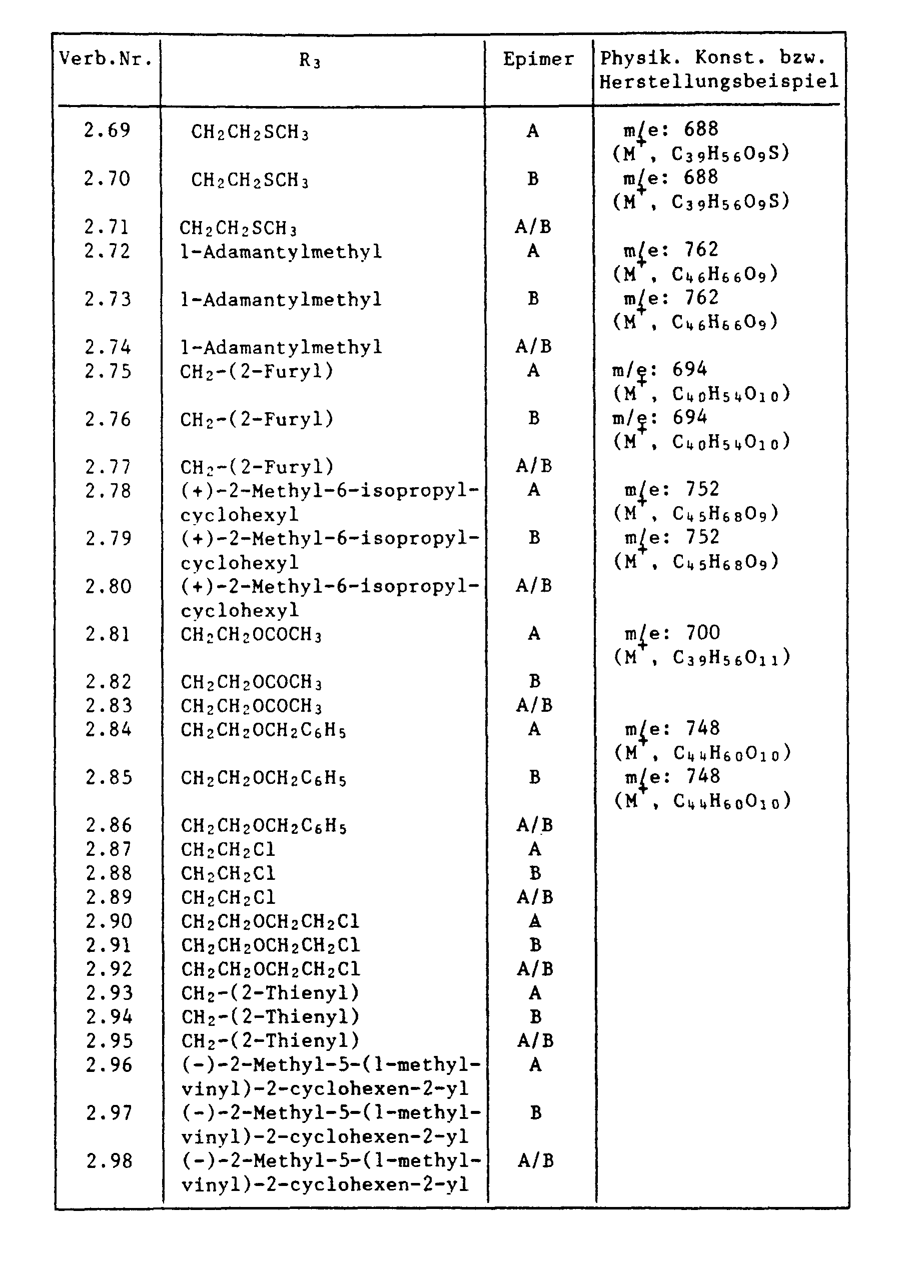

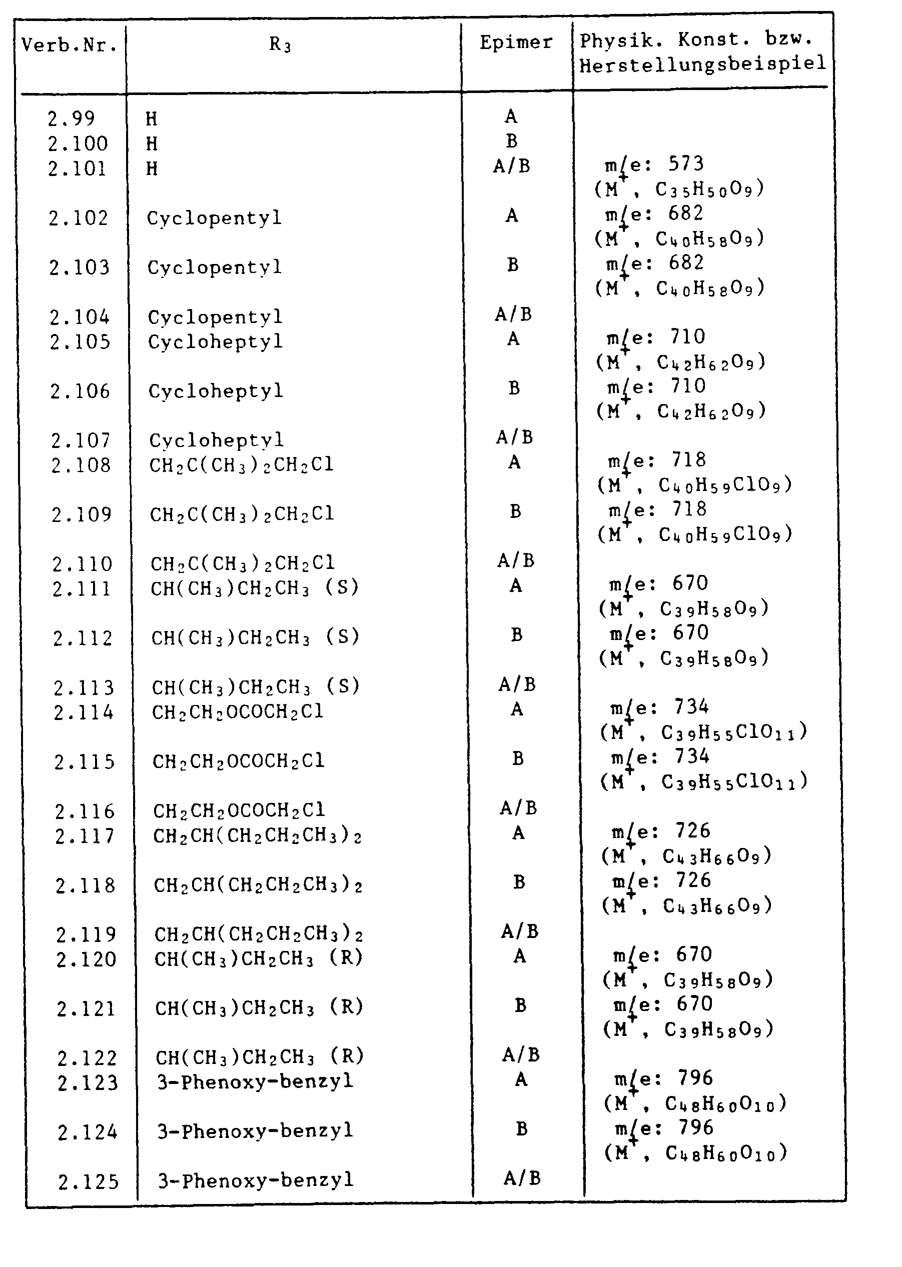

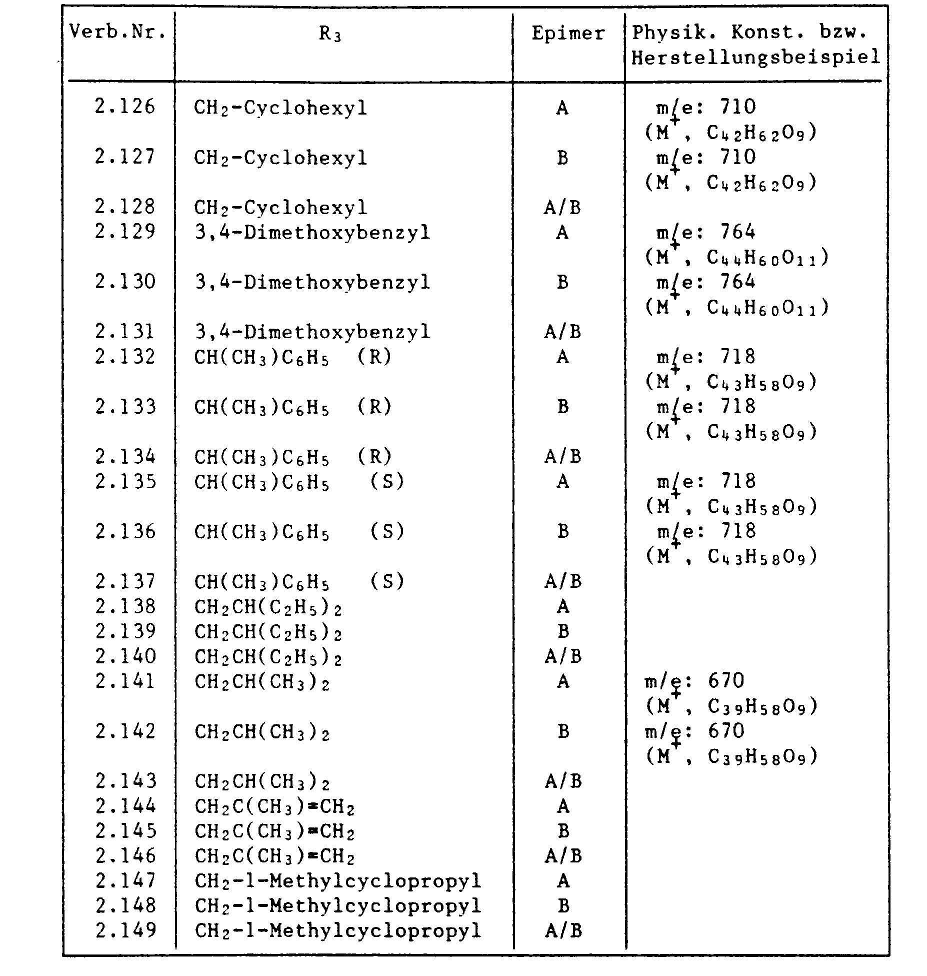

sowie die entsprechenden Verbindungen 3.1 bis 3.149, in denen X, R₁ und R₃ die in Tabelle 2 für die Verbindungen 2.1 bis 2.149 angegebenen Bedeutungen haben und R₂ für Isopropyl steht; sowie ferner die entsprechenden Verbindungen 4.1 bis 4.149, in denen X, R₁ und R₃ die in Tabelle 2 für die Verbindungen 2.1 bis 2.149 angebenen Bedeutungen haben und R₂ für sek.-Butyl steht;

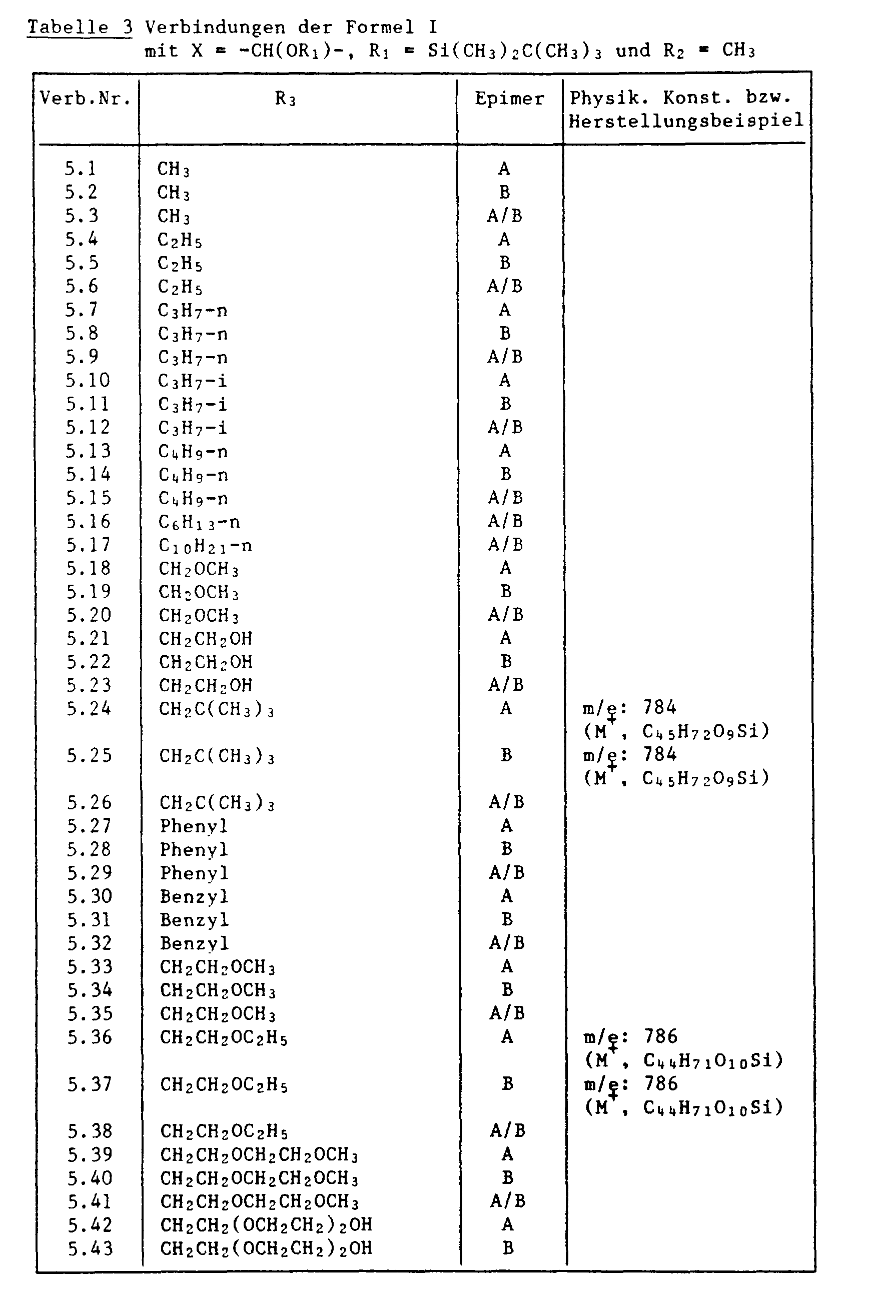

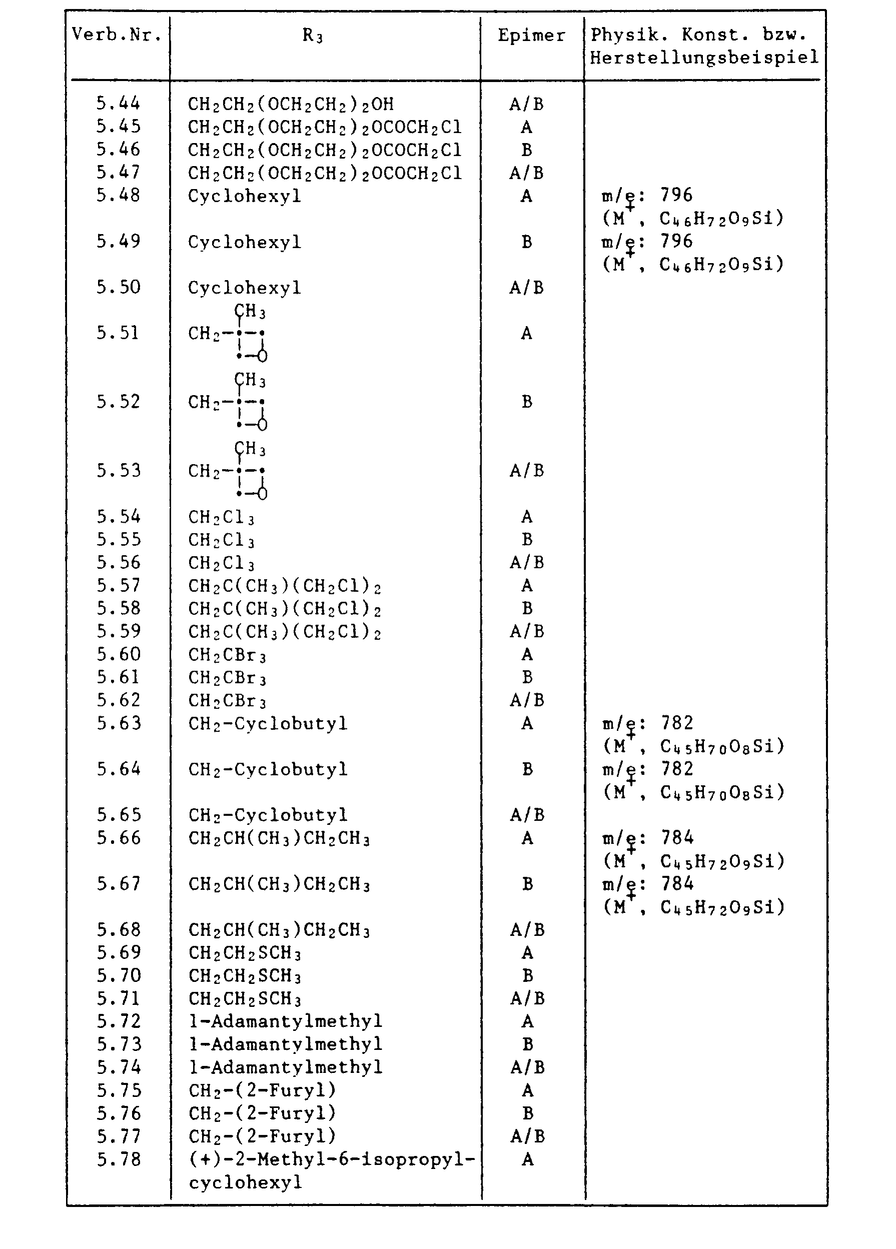

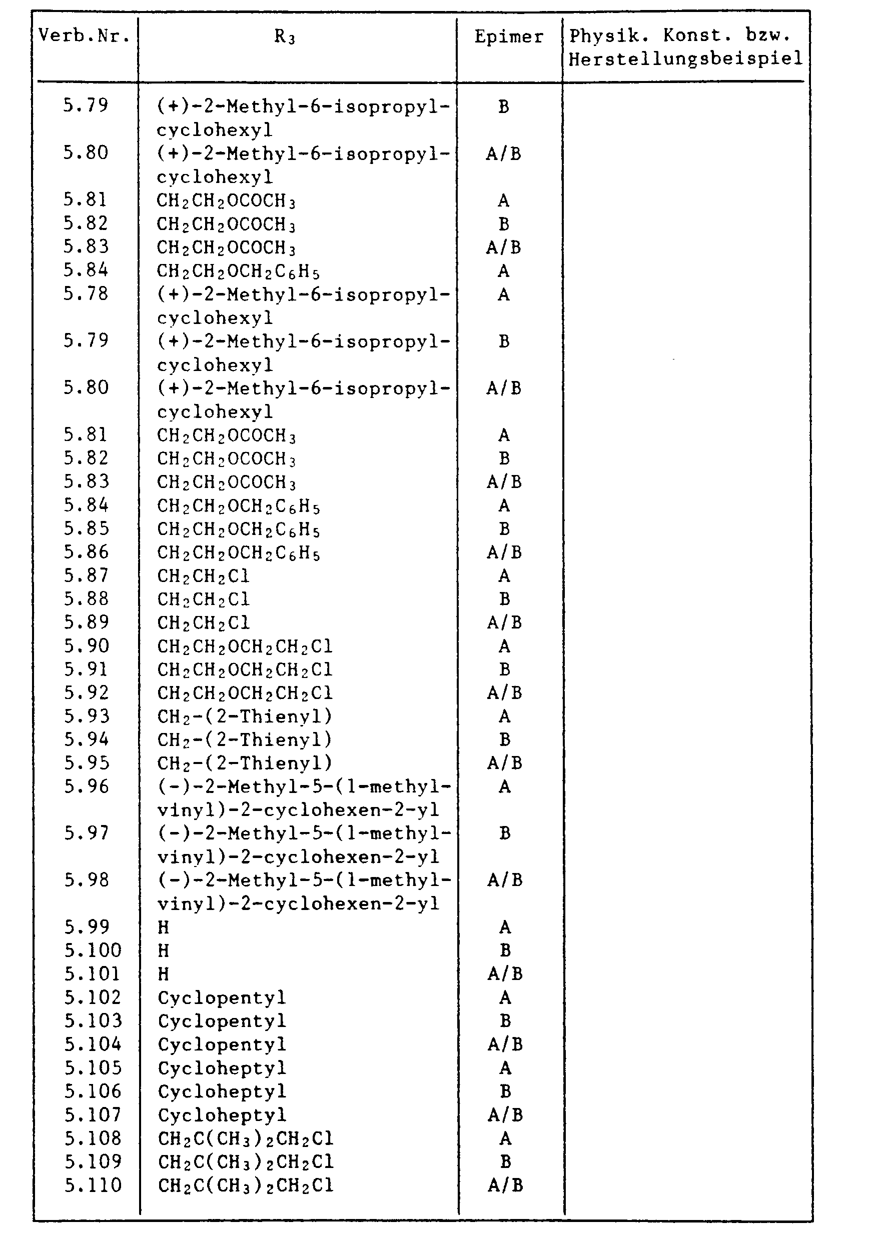

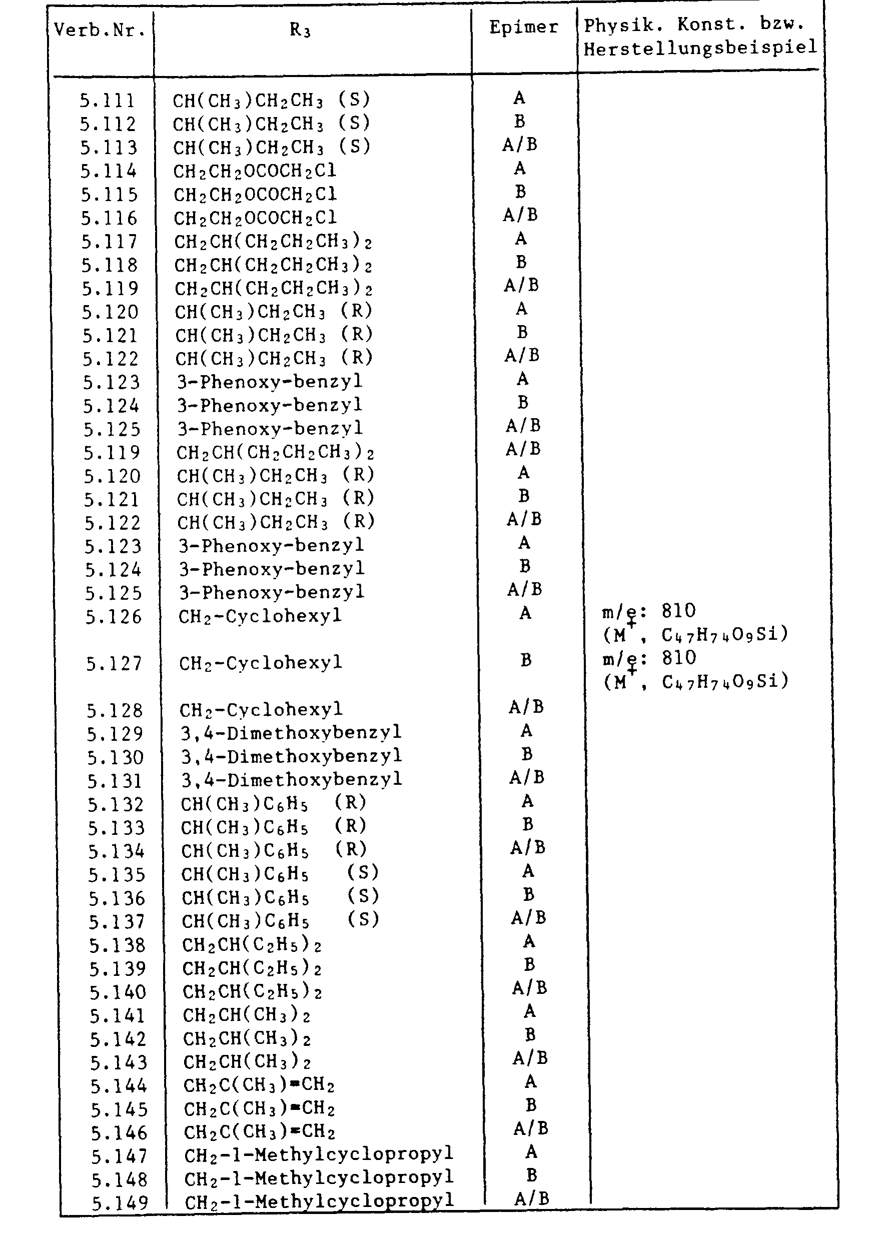

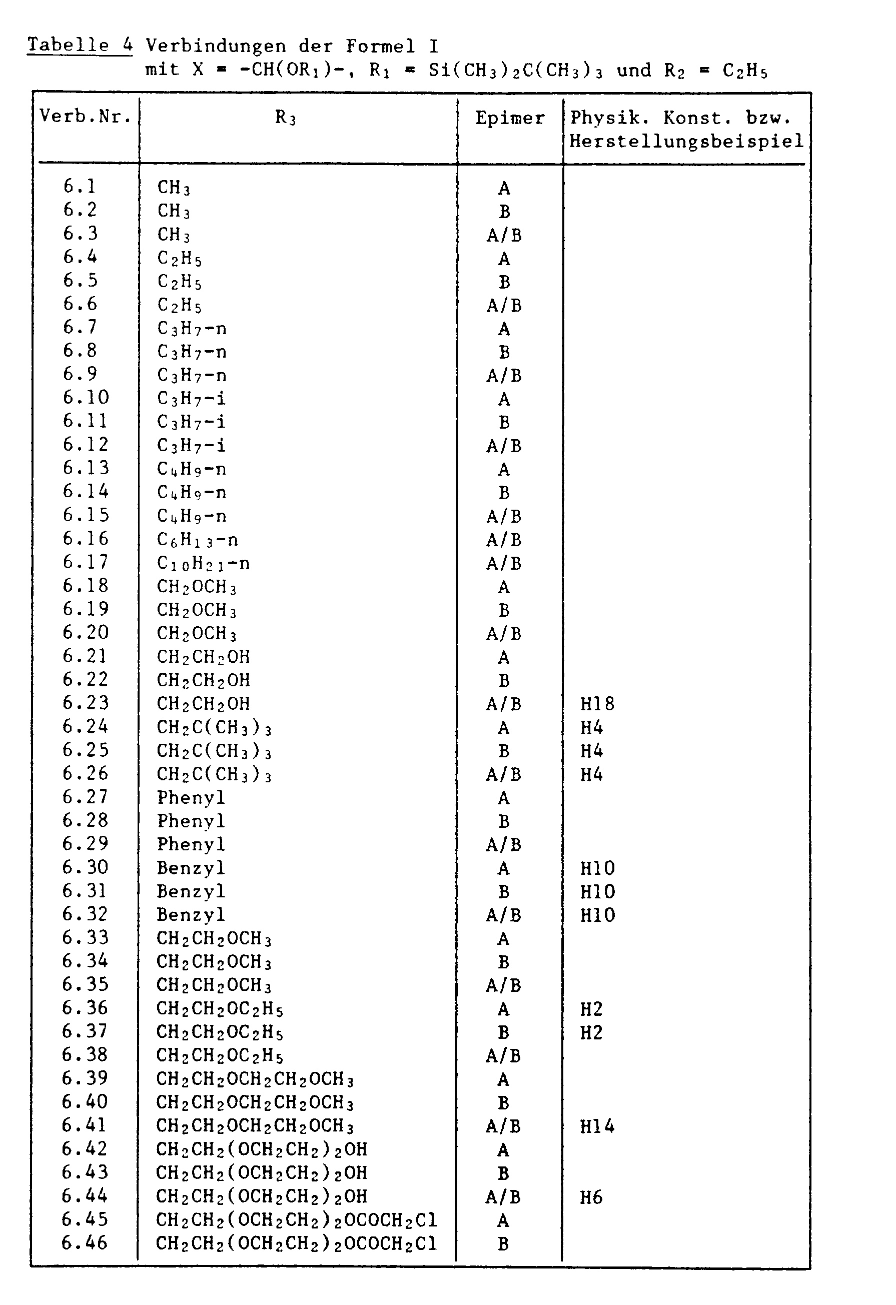

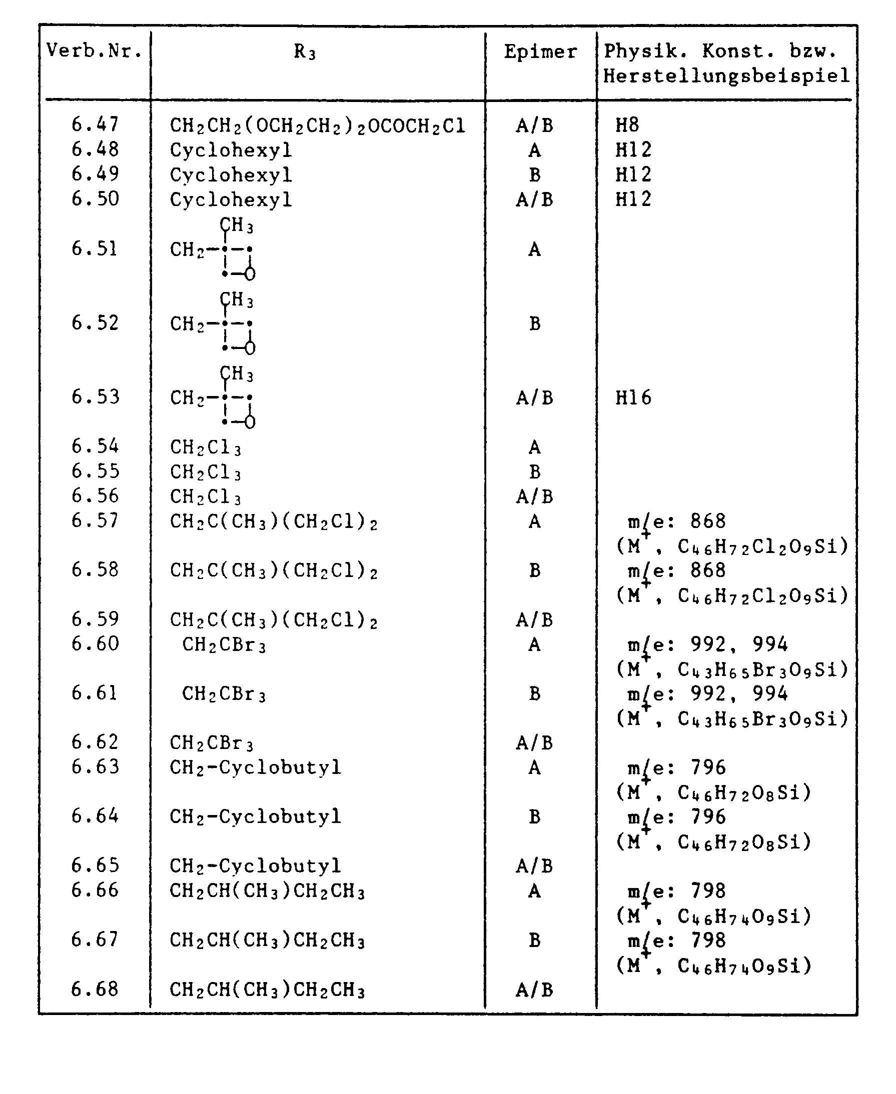

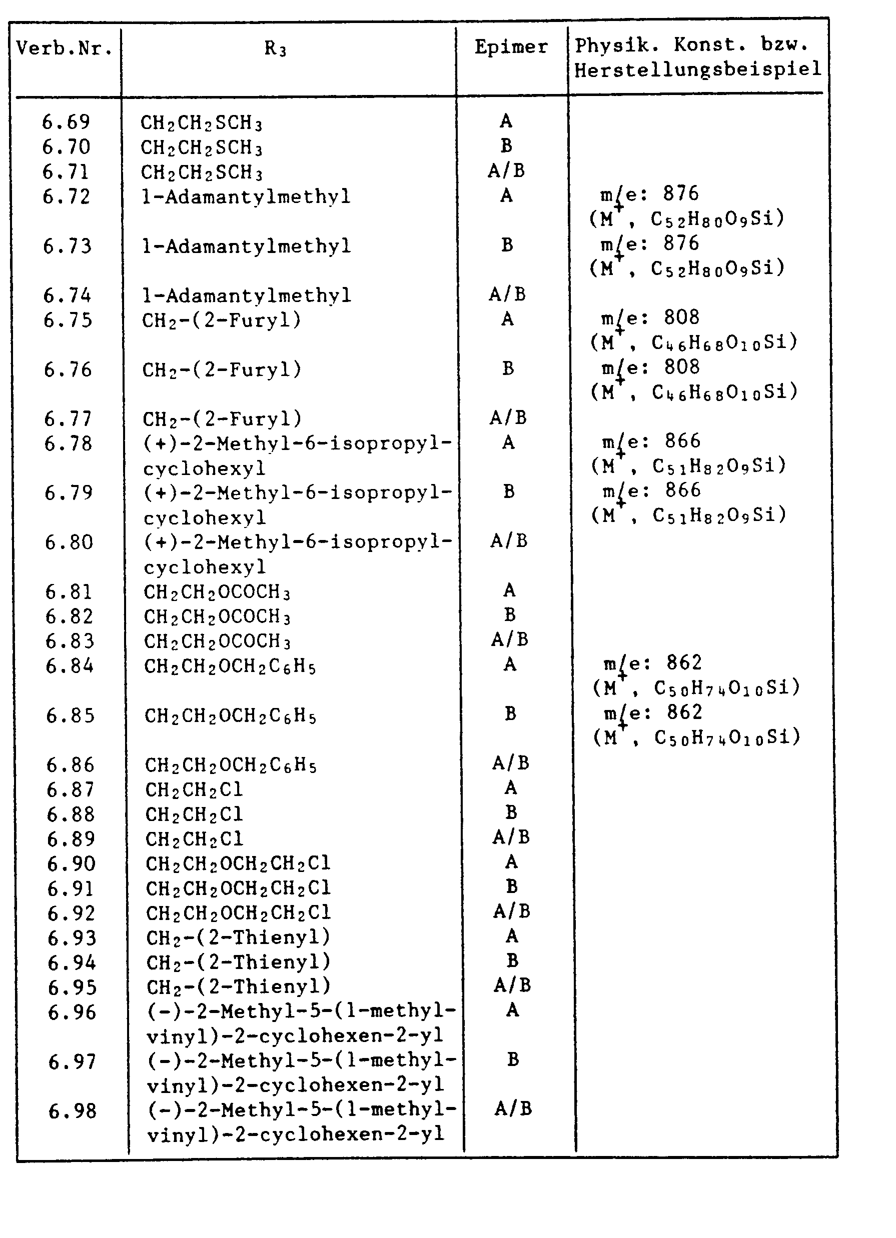

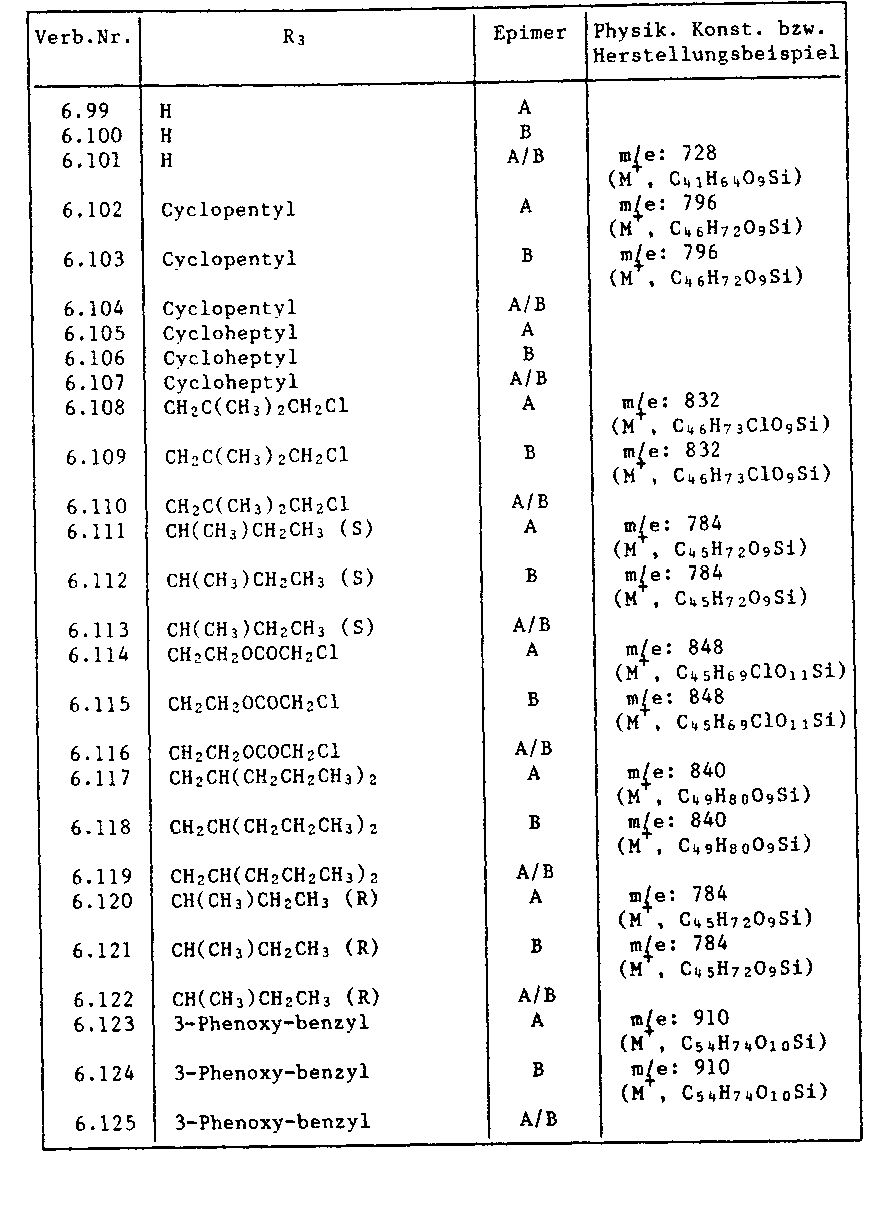

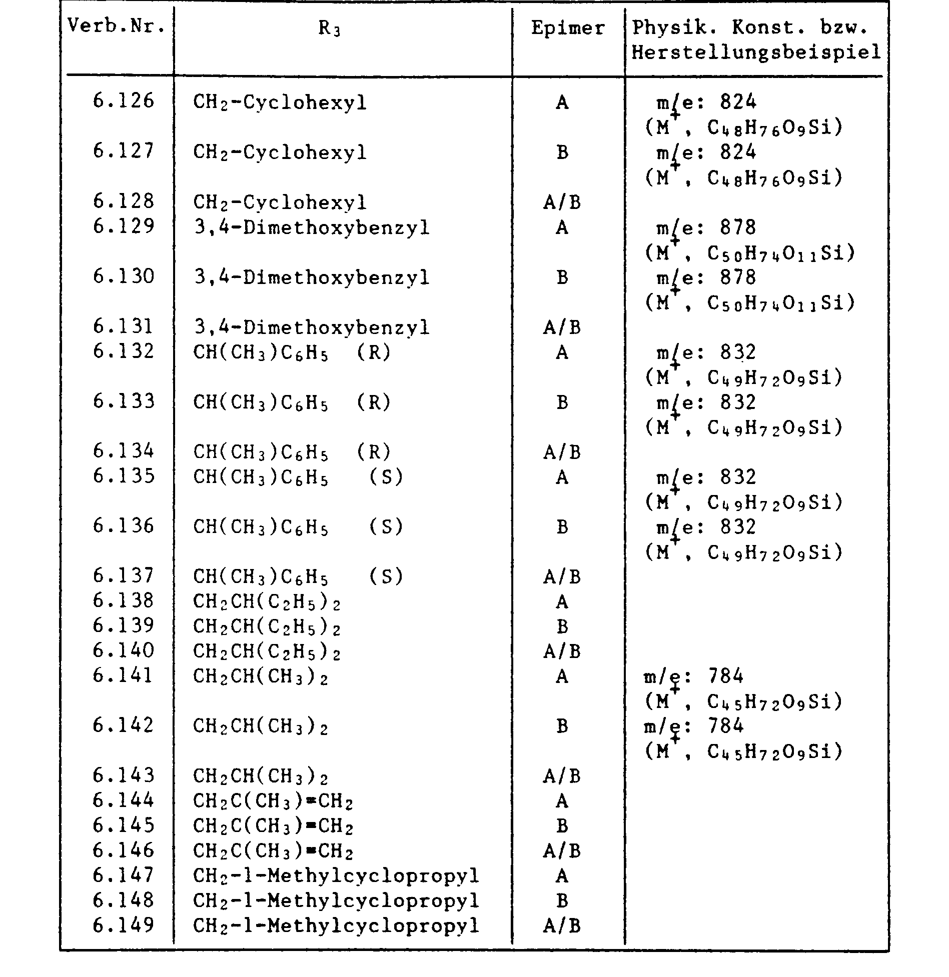

sowie die entsprechenden Verbindungen 7.1 bis 7.149 in denen X, R₁ und R₃ die in Tabelle 6 für die Verbindungen 6.1 bis 6.149 angegebenen Bedeutungen haben und R₂ für Isopropyl steht; sowie ferner die entsprechenden Verbindungen 8.1 bis 8.149 in denen X, R₁ und R₃ die in Tabelle 6 für die Verbindungen 6.1 bis 6.149 angegebenen Bedeutungen haben und R₂ für sek.-Butyl steht.

### Formulierungsbeispiele für Wirkstoffe der Formel 1

### (% = Gewichtsprozent)

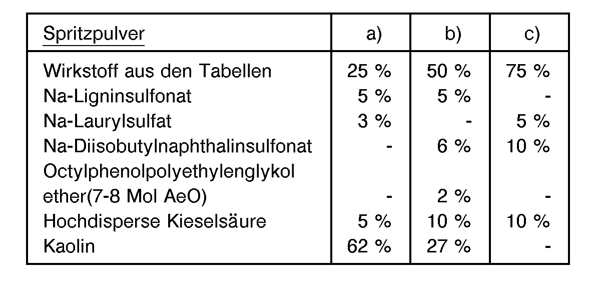

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

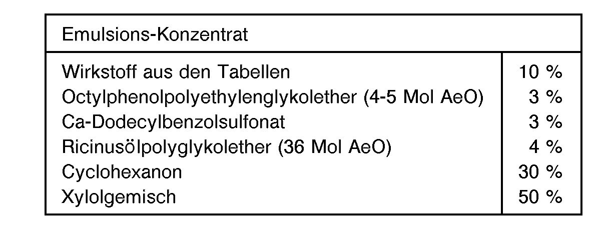

Aus diesem Konzentrat können durch Verdünnen mit Wasser Emulsionen jeder gewünschten Konzentration hergestellt werden.

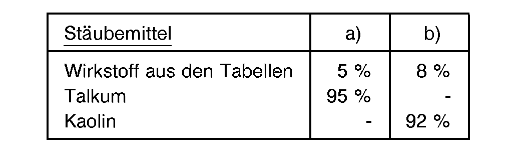

Man erhält anwendungsfertige Stäubemittel, indem der Wirkstoff mit dem Träger vermischt und auf einer geeigneten Mühle vermahlen wird.

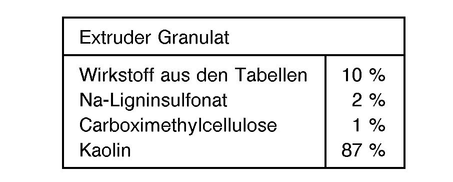

Der Wirkstoff wird mit den Zusatzstoffen vermischt, vermahlen und mit Wasser angefeuchtet. Dieses Gemisch wird extrudiert und anschliessend im Luftstrom getrocknet.

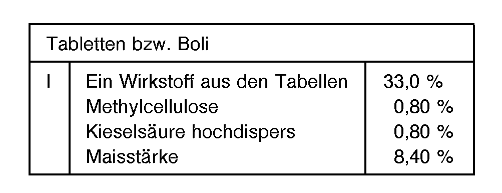

Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb (Maschenweite 12 M) granulieren und dann trocknen.

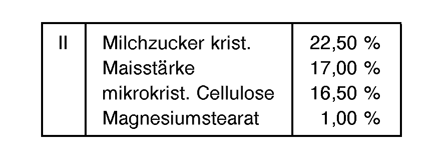

### Alle 4 Hilfsstoffe gut mischen

Phasen 1 und II mischen und zu Tabletten oder Boli verpressen.

### Injektabiles

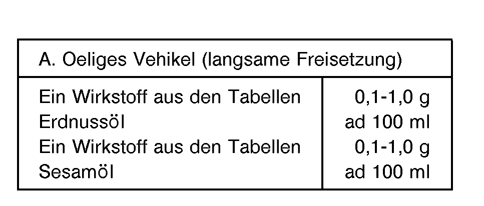

Herstellung: Der Wirkstoff wird in einem Teil des Oels unter Rühren und gegebenenfalls leichtem Erwärmen gelöst, nach Abkühlung auf das Sollvolumen aufgefüllt und durch ein geeignetes Membranfilter mit 0,22 um sterilfiltriert.

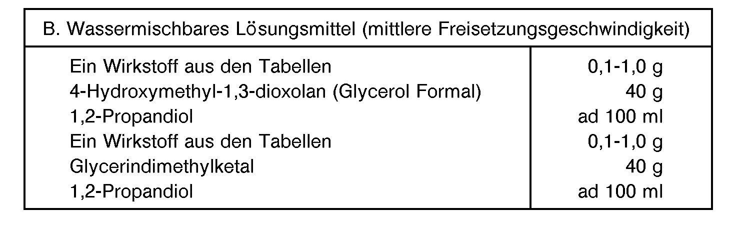

Herstellung: Der Wirkstoff wird in einem Teil des Lösungsmittels unter Rühren gelöst, auf das Sollvolumen aufgefüllt und durch ein geeignetes Membranfilter mit 0.22 um sterilfiltriert.

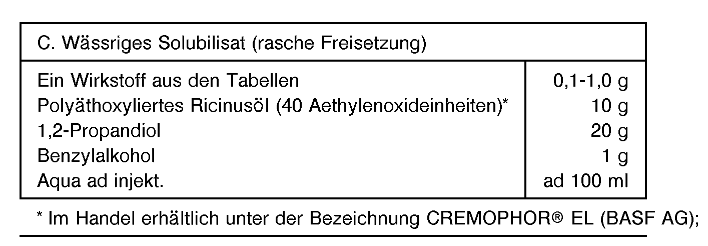

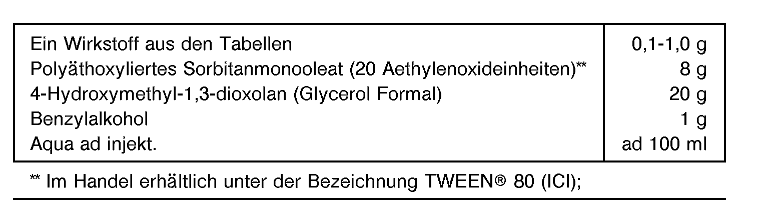

Herstellung: Der Wirkstoff wird in den Lösungsmitteln und dem Tensid gelöst und mit Wasser auf das Sollvolumen aufgefüllt. Sterilfiltration durch geeignetes Membranfilter mit 0,22 um Porendurchmesser.

Die wässrigen Systeme können bevorzugterweise auch für die orale und/oder intraruminale Applikation eingesetzt werden.

Wenn die Verbindungen der Formel I bzw. entsprechende Mittel zur Bekämpfung von endoparasitären Nematoden, Cestoden und Trematoden bei Haus- und Nutztieren, wie Rindern, Schafen, Ziegen, Katzen und Hunden, verwendet werden, können sie den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirkstoffkombinationen vorzugsweise in einer Konzentration von 0,005 bis 0,1 Gew. %. Die Mittel können in Form von Lösungen, Emulsionen, Suspensionen, Pulver, Tabletten, Bolussen oder Kapseln peroral den Tieren verabreicht werden. Soweit die physikalischen und toxikologischen Eigenschaften von Lösungen oder Emulsionen dies zulassen, können die Verbindungen der Formel I bzw. die enthaltende Mittel an Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

### Biologische Beispiele

### B-1. Wirkung gegen Li -Larven von Lucilia sericata

1 ml einer wässrigen Suspension der zu prüfenden Aktivsubstanz werden so mit 3 ml eines speziellen Larvenzuchtmediums bei ca. 50°C vermischt, dass ein Homogenisat von wahlweise 250 ppm oder 125 ppm Wirkstoffgehalt entsteht. In jede Reagenzglas-Probe werden ca. 30 Lucilia-Larven (L₁) eingesetzt. Nach 4 Tagen wird die Mortalitätsrate bestimmt. Verbindungen der Formel 1 wie z.B. Nr. 3.2, 3.6, 3.7, 3.10, 3.34 und 3.38 erzielten mit 125 ppm eine Wirkung von 100 %.

### B-2. Akarizide Wirkung gegen Boophilus microplus (Biarra-Stamm)

Auf einer PVC-Platte wird waagrecht ein Klebstreifen so befestigt, das darauf 10 mit Blut vollgesogene Zecken-Weibchen von Boophilus microplus (Biarra-Stamm) nebeneinander in einer Reihe mit dem Rücken aufgeklebt werden können. Jeder Zecke wird mit einer Injektionsnadel 1 µl einer Flüssigkeit injiziert, die eine 1:1-Mischung von Polyethylenglykol und Aceton darstellt und in der eine bestimmte Wirkstoffmenge von 1,0 ug pro Zecke gelöst ist. Kontrolltiere erhalten eine wirkstofffreie Injektion. Nach der Behandlung werden die Tiere unter Normalbedingungen in einem Insektarium bei ca. 28 °C und 80 % relativer Luftfeuchtigkeit gehalten, bis die Eiablage erfolgt und die Larven aus den Eiern der Kontrolltiere geschlüpft sind.

Verbindungen der Formel I, wie z.B. die der Herstellungsbeispiele, bewirken bei dieser Wirkstoffkonzentration, dass noch nach 30 Tagen 9 von 10 Zeckenweibchen (= 90 %) Eier ablegen, die nicht schlupffähig sind.

### B-3. Versuch an mit Nematoden (Haemonchus contortus und Trichostrongylus colubriformis) infizierten Schafen

Der Wirkstoff wird als Suspension formuliert mit einer Magensonde oder durch Injektion in den Pansen eines Schafes gegeben, das mit Haemonchus contortus und Trichostrongylus colubriformis künstlich infiziert worden ist. Pro Dosis werden 1 bis 3 Tiere verwendet. Jedes Schaf wird nur einmal mit einer einzigen Dosis behandelt, und zwar wahlweise mit 1 mg oder 0,2 mg/kg Körpergewicht. Die Evaluierung erfolgt durch Vergleich der Anzahl der vor und nach Behandlung im Kot der Schafe ausgeschiedenen Wurmeier. Gleichzeitig und gleichartig infizierte aber unbehandelte Schafe dienen als Kontrolle. Verbindungen der Formel I, wie z.B. die der Herstellungsbeispiele, zeigen in diesem Test bei einer Dosis von 0,2 mg/kg eine gute Wirkung, d.h. dass im Vergleich zu unbehandelten, aber infizierten Vergleichsgruppen die behandelten Schafe keinen Nematodenbefall aufweisen (= komplette Reduktion der Wurmeier im Kot).

### B-4. Larvizidwirkung gegen Aedes aegypti

Auf die Oberfläche von 150 ml Wasser, das sich in einem Behälter befindet, wird soviel einer 0,1 %igen acetonischen Lösung des Wirkstoffes pipettiert, dass Konzentrationen von wahlweise 10 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Verbindungen der Formel I, wie z.B. die der Herstellungsbeispiele bewirkten in diesem Test bei einer Konzentration von 1,6 ppm bereits nach einem Tag eine vollständige Abtötung sämtlicher Larven.

### B-5. Milbizide Wirkung gegen Dermanvssus gallinae

2 bis 3 ml einer Testlösung (100, 10, 1 und 0,1 ppm Aktivsubstanz) werden in einen nach oben offenen Glasbehälter gegeben und ca. 200 Milben in unterschiedlichen Entwicklungsstadien in diesen Behälter eingesetzt. Der Glasbehälter wird mit einem Wattebausch verschlossen und 10 Minuten gleichmässig bis zur vollständigen Benetzung der Milben geschüttelt. Danach wird der Behälter umgekehrt hingestellt, bis die überschüssige Testlösung von der Watte aufgesogen ist. Der Behälter wird erneut gekehrt und die behandelten Milben zur Evaluierung der Wirksamkeit der Testsubstanzen drei Tage unter Laborbedingungen beobachtet, wobei die Mortalität als Massstab für Wirksamkeit herangezogen wird.

Verbindungen der Formel I, wie z.B. die der Herstellungsbeispiele, zeigen bei 100 ppm eine Wirkung von 100 %.

## Patentansprüche (Patentansprüche für folgende(n) Vertragsstaat(en) : AT, BE, CH, DE, FR, GB, IT, LI, LU, NL, SE)

1. Eine Verbindung der Formel I, 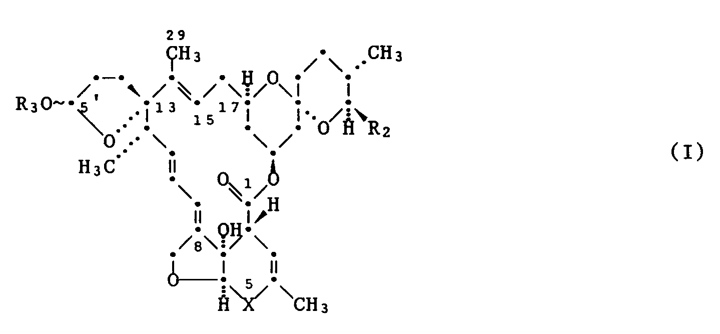 in welcher
X eine der Gruppen -CH(OR₁)-, -C( = O)- oder -C( = N-OH)- repräsentiert;
R₁ Wasserstoff oder eine OH-Schutzgruppe bedeutet;
R₂ für Methyl, Ethyl, Isopropyl oder sek.Butyl oder die Gruppe -C(CH3)=CH-A, worin A Methyl, Ethyl oder Isopropyl bedeutet, steht; und
R₃ Wasserstoff, C₁-C₁₀-Alkyl, durch mindestens einen Substituenten der Gruppe Halogen, Ci-C₆-Alkoxy, C₂-C₆-Alkoxyalkoxy, C₃-C₉-Alkoxyalkoxyalkoxy, C₁-C₆-Alkylthio, C₃-C₇-Cycloalkyl, durch C₁-C₃-Alkyl substituiertes C₃-C₇-Cyclaolkyl, Hydroxy, Benzyloxy, C₁-C₆-Acyl und Cᵢ-C₆-Acyloxy substituiertes Ci-Cio-Alkyl, wobei jeder der vorgängig genannten, eine Alkoxygruppe darstellenden oder enthaltenden Reste an einer endständigen Alkoxygruppe terminal durch Hydroxy, Halogen, Cᵢ-C₆-Acyl oder Cᵢ-C₆-Acyloxy substituiert sein kann, C₃-C₇-Cycloalkyl, durch mindestens einen Substituenten der Gruppe Halogen und C₁-C₃-Alkyl substituiertes C₃-C₇-Cycloalkyl, C₃-C₇-Cycloalkenyl, C₂-C₁₀-Alkenyl, C₂-C₁₀-Alkinyl, einen durch Halogen, C₁-C₆-Alkoxy oder C₁-C₆-Acyloxy substituierten Rest aus der Gruppe C₂-Cio-Alkenyl und C₂-C₁₀-Alkinyl, 1-Adamantylmethyl, Menthyl, Carveyl, Phenyl , Benzyl, Naphthyl, einen durch mindestens einen Substituenten der Gruppe Halogen, C₁-C₃-Alkyl, C₁-C₃-Haloalkyl, C₁-C₃-Alkoxy, C₁-C₃-Haloalkoxy, C₁-C₃-Alkylthio, Nitro und Cyano substituierten Rest aus der Gruppe Phenyl, Benzyl und Naphthyl, durch eine Phenoxygruppe substituiertes Benzyl, oder einen unsubstituierten oder durch mindestens einen Substituenten der Gruppe Halogen, C₁-C₃-Alkyl, C₁-C₃-Haloalkyl, C₁-C₃-Alkoxy, C₁-C₃-Haloalkoxy, Cₗ-C₃-Alkylthio, Nitro und Cyano substituierten vier- bis sechsgliedrigen Heterocyclus mit ein bis drei Heteroatomen aus der Gruppe Sauerstoff, Schwefel und Stickstoff repräsentiert, wobei besagter Heterocyclus auch über eine Cᵢ-C₆-Alkylenbrücke an das Sauerstoffatom in 5'-Position des Tetrahydrofuranrings gebunden sein kann.

2. Eine Verbindung der Formel I nach Anspruch 1, in welcher
X eine der Gruppen -CH(OR₁)- oder -C( = N-OH)- repräsentiert;
R₂ für Methyl, Ethyl, Isopropyl oder sek.Butyl steht; und
R₃ Wasserstoff, C₁-C₁₀-Alkyl, durch mindestens einen Substituenten der Gruppe Halogen, Ci-C₆-Alkoxy, C₂-C₆-Alkoxyalkoxy, C₃-C₉-Alkoxyalkoxyalkoxy, C₁-C₆-Alkylthio, C₃-C₇-Cycloalkyl, Hydroxy und C₁ -C₆-Acyl substituiertes C₁-C₁₀-Alkyl, wobei jeder der vorgängig genannten, eine Alkoxygruppe darstellenden oder enthaltenden Reste an einer endständigen Alkoxygruppe terminal durch Hydroxy, Halogen, C₁-C₆-Acyl oder C₁-C₆-Acyloxy substituiert sein kann, eine durch Benzyloxy substituierte Aethylgruppe, C₃-C₇-Cycloalkyl, durch mindestens einen Substituenten der Gruppe Halogen und C₁-C₃-Alkyl substituiertes C₃-C₇-Cycloalkyl, C₃-C₇-Cycloalkenyl, C₂-C₁₀-Alkenyl, C₂-C₁₀-Alkinyl, einen durch Halogen, C₁-C₆-Alkoxy oder Cᵢ-C₆-Acyloxy substituierten Rest aus der Gruppe C₂-C₁₀-Alkenyl und C₂-C₁₀-Alkinyl, 1-Adamantylmethyl, Menthyl, Carveyl, Phenyl , Benzyl, Naphthyl, einen durch mindestens einen Substituenten der Gruppe Halogen, C₁-C₃-Alkyl, C₁-C₃-Haloalkyl, C₁-C₃-Alkoxy, C₁-C₃-Halo- alkoxy, C₁-C₃-Alkylthio, Nitro und Cyano substituierten Rest aus der Gruppe Phenyl, Benzyl und Naphthyl, durch eine Phenoxygruppe substituiertes Benzyl, oder einen unsubstituierten oder durch mindestens einen Substituenten der Gruppe Halogen, C₁-C₃-Alkyl, C₁-C₃-Haloalkyl, C₁-C₃-Alkoxy, C₁-C₃-Haloalkoxy, C₁-C₃-Alkylthio, Nitro und Cyano substituierten vierbis sechsgliedrigen Heterocyclus mit ein bis drei Heteroatomen aus der Gruppe Sauerstoff, Schwefel und Stickstoff repräsentiert, wobei besagter Heterocyclus auch über eine Ci-C₆-Alkylenbrücke an das Sauerstoffatom in 5'-Position des Tetrahydrofuranrings gebunden sein kann.

3. Eine Verbindung der Formel I nach Anspruch 2, worin X -CH(OR₁ )-repräsentiert.

4. Eine Verbindung der Formel I nach Anspruch 1, worin X -CH(OR₁ )-repräsentiert;
R₂ für Methyl, Ethyl, Isopropyl oder sek.Butyl steht; und
R₃ Wasserstoff, C₁-C₁₀-Alkyl, durch mindestens einen Substituenten der Gruppe Halogen, Ci-C₆-Alkoxy, C₂-C₆-Alkoxyalkoxy, C₃-C₉-Alkoxyalkoxyalkoxy, C₁-C₆-Alkylthio, C₃-C₇-Cycloalkyl, Hydroxy und C₁-C₆-Acyl substituiertes C₁-C₁₀-Alkyl, wobei jeder der vorgängig genannten, eine Alkoxygruppe darstellenden oder enthaltenden Reste an einer endständigen Alkoxygruppe terminal durch Hydroxy, Halogen, C₁-C₆-Acyl oder C₁-C₆-Acyloxy substituiert sein kann, C₃-C₇-Cycloalkyl, durch mindestens einen Substituenten der Gruppe Halogen und Cᵢ-C₃-Alkyl substituiertes C₃-C₇-Cycloalkyl, C₃-C₇-Cycloalkenyl, C₂-C₁₀-Alkenyl, C₂-C₁₀-Alkinyl, einen durch Halogen, C₁-C₆-Alkoxy oder C₁-C₆-Acyloxy substituierten Rest aus der Gruppe C₂-C₁₀-Alkenyl und C₂-C₁₀-Alkinyl, 1-Adamantylmethyl, Menthyl, Carveyl, Phenyl , Benzyl, Naphthyl, einen durch mindestens einen Substituenten der Gruppe Halogen, C₁-C₃-Alkyl, Ci-C₃-Haloalkyl, C₁-C₃-Alkoxy, C₁-C₃-Haloalkoxy, C₁-C₃-Alkylthio, Nitro und Cyano substituierten Rest aus der Gruppe Phenyl, Benzyl und Naphthyl, oder einen unsubstituierten oder durch mindestens einen Substituenten der Gruppe Halogen, C₁-C₃-Alkyl, C₁-C₃-Haloalkyl, C₁-C₃-Alkoxy, C₁-C₃-Haloalkoxy, C₁-C₃-Alkylthio, Nitro und Cyano substituierten vier- bis sechsgliedrigen Heterocyclus mit ein bis drei Heteroatomen aus der Gruppe Sauerstoff, Schwefel und Stickstoff repräsentiert, wobei besagter Heterocyclus auch über eine C₁-C₆-Alkylenbrük- ke an das Sauerstoffatom in 5'-Position des Tetrahydrofuranrings gebunden sein kann.

5. Eine Verbindung der Formel nach Anspruch 4, worin R₁ für Wasserstoff, R₄-C(0)- oder -Si(Rs)(R₆)(R₇) steht; wobei R₄ C₁-C₁₀-Alkyl, C₁-C₁₀-Haloalkyl oder einen unsubstituierten oder durch mindestens einen Substituenten der Gruppe Halogen, C₁-C₃-Alkyl, C₁-C₃-Haloalkyl, C₁-C₃-Alkoxy, C₁-C₃-Haloal- koxy, Cyano und Nitro substituierten Rest aus der Gruppe Phenyl und Benzyl bedeutet und Rₛ, R₆ und R₇ unabhängig voneinander für C₁-C₆-Alkyl, Benzyl oder Phenyl stehen; und R₃ Wasserstoff, C₁-C₅-Alkyl, durch mindestens einen Substituenten der Gruppe Halogen, C₁-C₃-Alkoxy, C₂-C₆-Alkoxyalkoxy, C₃-C₉-Alkoxyalkoxyalkoxy, C₁-C₃-Alkylthio, C₃-C₇-Cycloalkyl, Hydroxy und C₁-C₆-Acyl substituiertes C₁-C₅-Alkyl, wobei jeder der vorgängig genannten, eine Alkoxygruppe darstellenden oder enthaltenden Reste an einer endständigen Alkoxygruppe terminal durch Hydroxy, Halogen, C₁-C₆-Acyl oder C₁-C₆-Acyloxy substituiert sein kann; C₃-C₇-Cycloalkyl, durch mindestens einen Substituenten der Gruppe Fluor, Chlor, Brom und Methyl substituiertes C₃-C₇-Cycloalkyl, C₂-C₆-Alkenyl, C₂-C₆-Alkinyl, einen durch Fluor, Chlor, Brom, C₁-C₃-Alkoxy oder C₁-C₆-Acyloxy substituierten Rest aus der Gruppe C₂-C₆-Alkenyl und C₂-C₆-Alkinyl, Phenyl, Benzyl, a-Naphthyl, ß-Naphthyl, einen durch mindestens einen Substituenten der Gruppe Fluor, Chlor, Brom, Methyl, Methoxy, CF₃, CF₃0, CH₃S, Nitro und Cyano substituierten Rest aus der Gruppe Phenyl, Benzyl, a-Naphthyl und ß-Naphthyl, oder einen unsubstituierten oder durch mindestens einen Substituenten der Gruppe Fluor, Chlor, Brom, Methyl, Ethyl, CF₃, CH₃0, CF₃0, CH₃S, Nitro und Cyano substituierten vier- bis sechsgliedrigen Heterocyclus mit ein bis drei Heteroatomen aus der Gruppe Sauerstoff, Schwefel und Stickstoff repräsentiert, wobei besagter Heterocyclus auch über eine C₁-C₆-Alkylenbrücke an das Sauerstoffatom in 5'-Position des Tetrahydrofuranringes gebunden sein kann.

6. Eine Verbindung der Formel I nach Anspruch 5, worin R⁵, R⁶ und R⁷ unabhängig voneinander für C₁-C₄-Alkyl, Benzyl oder Phenyl stehen; und R₃ C₁-C₅-Alkyl oder durch mindestens einen Substituenten der Gruppe Halogen, C₁-C₃-Alkoxy, C₂-C₆-Alkoxyalkoxy, C₃-C₉-Alkoxyalkoxyalkoxy, C₁-C₃-Alkylthio, C₃-C₇-Cycloalkyl, Hydroxy und Cᵢ-C₆-Acyl substituiertes C₁-C₅-Alkyl, wobei jeder der vorgängig genannten, eine Alkoxygruppe darstellenden oder enthaltenden Reste an einer endständigen Alkoxygruppe terminal durch Hydroxy, Halogen, Cᵢ-C₆-Acyl oder Cᵢ-C₆-Acyloxy substituiert sein kann, repräsentiert.

7. Eine Verbindung der Formel I nach Anspruch 5, worin R⁵, R⁶ und R⁷ unabhängig voneinander für C₁-C₄-Alkyl, Benzyl oder Phenyl stehen; und R₃ C₃-C₇-Cycloalkyl, durch mindestens einen Substituenten der Gruppe Fluor, Chlor, Brom und Methyl substituiertes C₃-C₇-Cycloalkyl, C₂-C₆-Alkenyl, C₂-C₆-Alkinyl, einen durch Fluor, Chlor, Brom, C₁-C₃-Alkoxy oder C₁-C₆-Acyloxy substituierten Rest aus der Gruppe C₂-C₆-Alkenyl und C₂-C₆-Alkinyl, Phenyl, Benzyl, a-Naphthyl, ß-Naphthyl, einen durch mindestens einen Substituenten der Gruppe Fluor, Chlor, Brom, Methyl, Methoxy, CF₃, CF₃0, CH₃S, Nitro und Cyano substituierten Rest aus der Gruppe Phenyl, Benzyl, a-Naphthyl und ß-Naphthyl, oder einen unsubstituierten oder durch mindestens einen Substituenten der Gruppe Fluor, Chlor, Brom, Methyl, Ethyl, CF₃, CH₃0, CF₃0, CH₃S, Nitro und Cyano substituiertem vier- bis sechsgliedrigen Heterocyclus mit ein bis drei Heteroatomen aus der Gruppe Sauerstoff, Schwefel und Stickstoff repräsentiert, wobei besagter Heterocyclus auch über eine C₁-C₆-Alkylenbrücke an das Sauerstoffatom in 5'-Position des Tetrahydrofuranringes gebunden sein kann.

8. Eine Verbindung der Formel I nach Anspruch 7, worin R₃ Phenyl, Benzyl, a-Naphthyl, β-Naphthyl, einen durch mindestens einen Substituenten der Gruppe Fluor, Chlor, Brom, Methyl, Methoxy, CF₃, CF₃0, CH₃S, Nitro und Cyano substituierten Rest aus der Gruppe Phenyl, Benzyl, a-Naphthyl und β-Naphthyl repräsentiert.

9. Verbindung der Formel I nach Anspruch 7, worin R₃ einen unsubstituierten oder durch mindestens einen Substituenten der Gruppe Fluor, Chlor, Brom, Methyl, Ethyl, CF₃, CH₃0, CF₃0, CH₃S, Nitro und Cyano substituierten vier- bis sechsgliedrigen Heterocyclus mit ein bis drei Heteroatomen aus der Gruppe Sauerstoff, Schwefel und Stickstoff repräsentiert, wobei besagter Heterocyclus auch über eine C₁-C₆-Alkylenbrücke an das Sauerstoffatom in 5'-Position des Tetrahydrofuranringes gebunden sein kann.

10. Eine Verbindung der Formel I nach Anspruch 9, worin R₃ für einen ungesättigten viergliedrigen Heterocyclus mit einem Heteroatom aus der Gruppe Sauerstoff, Stickstoff und Schwefel steht oder Furan, Thiophen, Pyrrol, Isoxazol, Isothiazol, Furazan, Imidazol, 1,2,4-Triazol, 1,2,3-Triazol, Pyrazol, Pyrrolin, Oxazol, Thiazol, Thiadiazole, Pyrazolin, Thiazolin, Pyrazolidin, Pyrrolidin, Oxazolidin, Thiazolidin, Oxadiazol, Imidazolin, Imidazolidin, Pyrazolidin, Tetrahydrofuran, Pyridin, Pyridazin, Pyrimidin, Pyrazin, Thiazin, Thiadiazine, Pyrane, Piperidin, Piperazin, Morpholin, Perhydrothiazin oder Dioxan repräsentiert, wobei besagter Heterocyclus auch über eine C₁-C₄-Alkylenbrücke an das Sauerstoffatom in 5'-Position des Tetrahydrofuranrings gebunden sein kann.

11. Eine Verbindung der Formel I nach Anspruch 9, worin R₃ für einen gesättigten viergliedrigen Heterocyclus mit einem Heteroatom aus der Gruppe Sauerstoff, Stickstoff und Schwefel steht, wobei besagter Heterocyclus auch über eine C₁-C₄-Alkylenbrücke an das Sauerstoffatom in 5'-Position des Tetrahydrofuranrings gebunden sein kann.

12. Eine Verbindung der Formel I nach Anspruch 1, worin X für -CH(OR1)- und R₁ für Wasserstoff steht; R₂ für Ethyl steht; und R₃ C₄-Cₛ-Alkyl, C₄-C₆-Cycloalkyl, über Methyl gebundenes C₄-C₆-Cycloalkyl, Phenyl, Benzyl oder a-Methylbenzyl repräsentiert.

13. Eine Verbindung der Formel I nach Anspruch 1, worin R₂ für Methyl oder Ethyl steht.

14. Eine Verbindung der Formel I nach Anspruch 13, worin R₂ für Ethyl steht.

15. Eine Verbindung der Formel I nach Anspruch 4, ausgewählt aus der Gruppe:
Milbemycin A₄-13-spiro-2'-[5'-(2"-ethoxy-ethoxy)-tetrahydrofuran],
Milbemycin A₄-13-spiro-2'-[5'-(2",2"-dimethyl-propoxy)-tetrahydrofuran],
Milbemycin A₄-13-spiro-2'-[5'-cyclohexyloxy-tetrahydrofuran],
Milbemycin A₄-13-spiro-2'-[5'-benzyloxy-tetrahydrofuran],
Milbemycin A₄-13-spiro-2'-[5'-{2"-(2"'-(methoxy-ethoxy)-ethoxyl-tetrahydrofuran],
Milbemycin A₄-13-spiro-2'-[5'-{2"-(2"'-(hydroxymethoxy)-ethoxy)-ethoxy}-tetrahydrofuran],
Milbemycin A₄-13-spiro-2'-[5'-{2"-(2"'-(2''''-(chloracetoxy)-ethoxy)-ethoxy)-ethoxy}-tetrahydrofuran],
Milbemycin A₄-13-spiro-2'-[5'-methoxy-tetrahydrofuran] und
Milbemycin A₄-13-spiro-2'-[5'-(2"-hydroxy-ethoxy)-tetrahydrofuran].

16. Eine Verbindung der Formel I nach Anspruch 12, ausgewählt aus der Gruppe Milbemycin A₄-13-spiro-2'-[5'-(2"-methyl-butoxy)-tetrahydrofuran] und Milbemycin A₄-13-spiro-2'-[5'-(1 "-methyl-propoxy)-tetrahydrofuran].

17. Eine Verbindung der Formel II 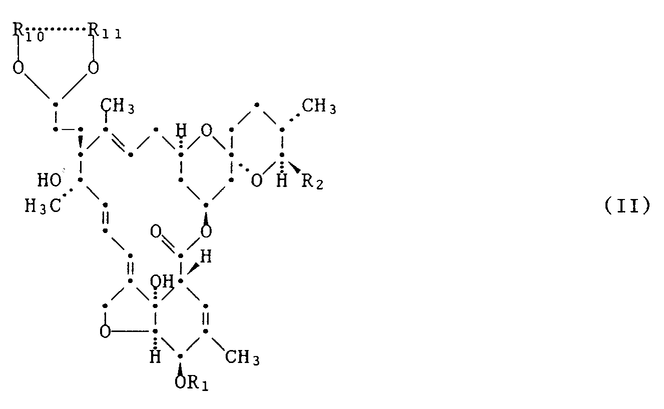 worin R₁ und R₂ wie unter Formel definiert sind und R₁₀ und R¹¹ unabhängig voneinander für Ci-C₆-Alkyl stehen oder zusammen eine C₂-C₁₀-Alkylenbrücke bilden.

18. Verfahren zur Herstellung einer Verbindung der Formel I nach Anspruch 1, dadurch gekennzeichnet, dass man eine Verbindung der Formel II nach Anspruch 17 in Gegenwart eines Säurekatalysators in einem reaktionsinerten Lösungsmittel mit einer Verbindung der Formel III 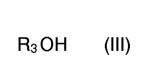 worin R₃ wie unter Formel I definiert ist, umsetzt, und gewünschtenfalls die so erhaltene Verbindung der Formel Ic 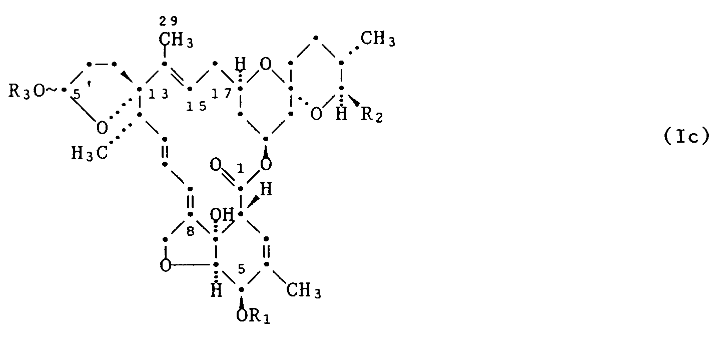 worin Ri, R₂ und R₃ die für Formel I angegebenen Bedeutungen haben, durch milde Oxydation in die entsprechende Verbindung der Formel Ib 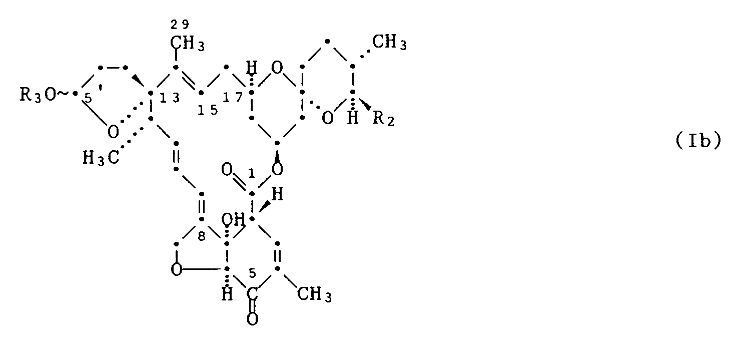 worin R₂ und R₃ die für Formel Ic angegebenen Bedeutungen haben, überführt und gewünschtenfalls die Verbindung der Formel Ib durch Umsetzung mit Hydroxylamin oder einem seiner Salze in die entsprechende Verbindung der Formel la 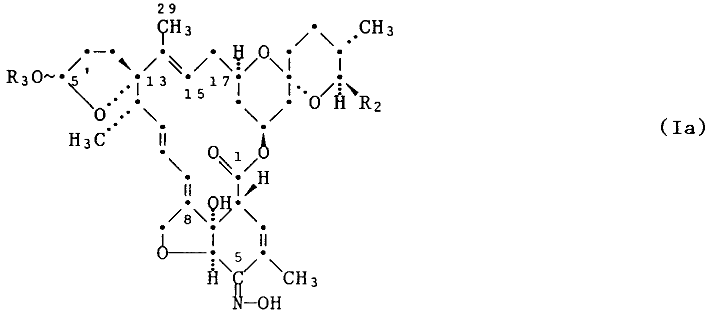 worin R₂ und R₃ die für Formel Ib angegebenen Bedeutungen haben, überführt.

19. Verfahren zur Herstellung einer Verbindung der Formel II nach Anspruch 17, dadurch gekennzeichnet, dass man eine Verbindung der Formel IV 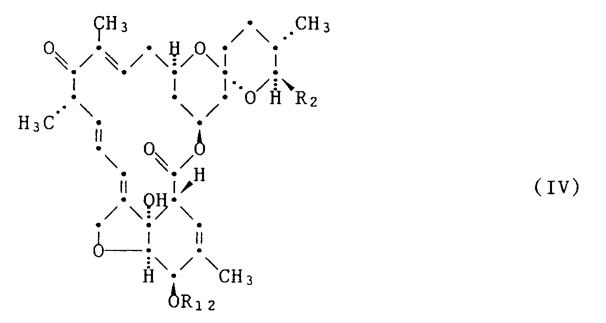 worin R₂ wie unter Formel I definiert ist und R₁₂ für Wasserstoff oder eine Silylgruppe steht, in einem reaktionsinerten Lösungsmittel mit einem Grignard-Reagenz der Formel V 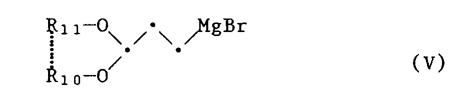 umsetzt.

20. Mittel zur Bekämpfung von Ekto- und Endoparasiten am Nutztier oder zur Bekämpfung von Schadinsekten, dadurch gekennzeichnet, dass es neben üblichen Trägerstoffen und Verteilungsmitteln eine Verbindung der Formel I nach Anspruch 1 enthält.

21. Mittel nach Anspruch 20, dadurch gekennzeichnet, dass es als Wirkstoff eine Verbindung der Formel I gemäss Anspruch 12 enthält.

## Patentansprüche (Patentansprüche für folgende(n) Vertragsstaat(en) : ES, GR)

1. Verfahren zur Herstellung einer Verbindung der Formel 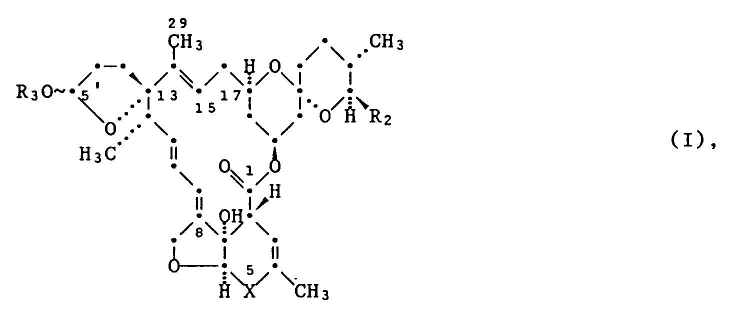 in welcher
X eine der Gruppen -CH(OR₁)-, -C( = O)- oder -C( = N-OH)- repräsentiert;
R₁ Wasserstoff oder eine OH-Schutzgruppe bedeutet;
R₂ für Methyl, Ethyl, Isopropyl oder sek.Butyl oder die Gruppe -C(CH3)=CH-A, worin A Methyl, Ethyl oder Isopropyl bedeutet, steht; und
R₃ Wasserstoff, C₁-C₁₀-Alkyl, durch mindestens einen Substituenten der Gruppe Halogen, Ci-C₆-Alkoxy, C₂-C₆-Alkoxyalkoxy, C₃-C₉-Alkoxyalkoxyalkoxy, C₁-C₆-Alkylthio, C₃-C₇-Cycloalkyl, durch C₁-C₃-Alkyl substituiertes C₃-C₇-Cyclaolkyl, Hydroxy, Benzyloxy, C₁-C₆-Acyl und C₁-C₆-Acyloxy substituiertes C₁-C₁₀-Alkyl, wobei jeder der vorgängig genannten, eine Alkoxygruppe darstellenden oder enthaltenden Reste an einer endständigen Alkoxygruppe terminal durch Hydroxy, Halogen, C₁-C₆-Acyl oder Cᵢ-C₆-Acyloxy substituiert sein kann, C₃-C₇-Cycloalkyl, durch mindestens einen Substituenten der Gruppe Halogen und C₁-C₃-Alkyl substituiertes C₃-C₇-Cycloalkyl, C₃-C₇-Cycloalkenyl, C₂-C₁₀-Alkenyl, C₂-C₁₀-Alkinyl, einen durch Halogen, C₁-C₆-Alkoxy oder C₁-C₆-Acyloxy substituierten Rest aus der Gruppe C₂-C₁₀-Alkenyl und C₂-C₁₀-Alkinyl, 1-Adamantylmethyl, Menthyl, Carveyl, Phenyl , Benzyl, Naphthyl, einen durch mindestens einen Substituenten der Gruppe Halogen, C₁-C₃-Alkyl, Ci-C₃-Haloalkyl, C₁-C₃-Alkoxy, C₁-C₃-Haloalkoxy, C₁-C₃-Alkylthio, Nitro und Cyano substituierten Rest aus der Gruppe Phenyl, Benzyl und Naphthyl, durch eine Phenoxygruppe substituiertes Benzyl, oder einen unsubstituierten oder durch mindestens einen Substituenten der Gruppe Halogen, C₁-C₃-Alkyl, C₁-C₃-Haloalkyl, C₁-C₃-Alkoxy, C₁-C₃-Haloalkoxy, C₁-C₃-Alkylthio, Nitro und Cyano substituierten vier- bis sechsgliedrigen Heterocyclus mit ein bis drei Heteroatomen aus der Gruppe Sauerstoff, Schwefel und Stickstoff repräsentiert, wobei besagter Heterocyclus auch über eine Cᵢ-C₆-Alkylenbrücke an das Sauerstoffatom in 5'-Position des Tetrahydrofuranrings gebunden sein kann, dadurch gekennzeichnet, dass man eine Verbindung der Formel 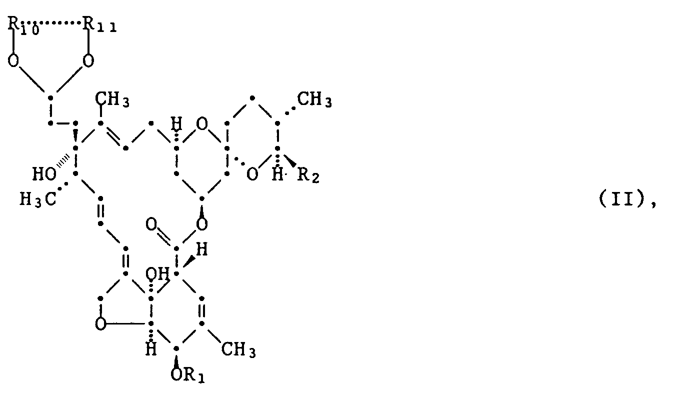 worin R₁ und R₂ wie unter Formel definiert sind und R₁₀ und R¹¹ unabhängig voneinander für Ci-C₆-Alkyl stehen oder zusammen eine C₂-C₁₀-Alkylenbrücke bilden, in Gegenwart eines Säurekatalysators in einem reaktionsinerten Lösungsmittel mit einer Verbindung der Formel 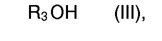 worin R₃ wie unter Formel I definiert ist, umsetzt, und gewünschtenfalls die so erhaltene Verbindung der Formel 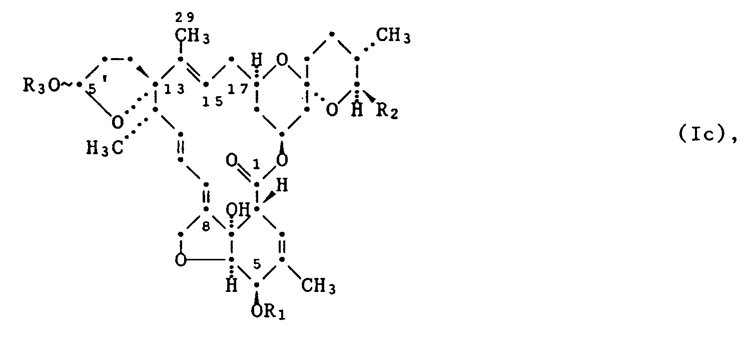 worin Ri, R₂ und R₃ die für Formel I angegebenen Bedeutungen haben, durch milde Oxydation in die entsprechende Verbindung der Formel 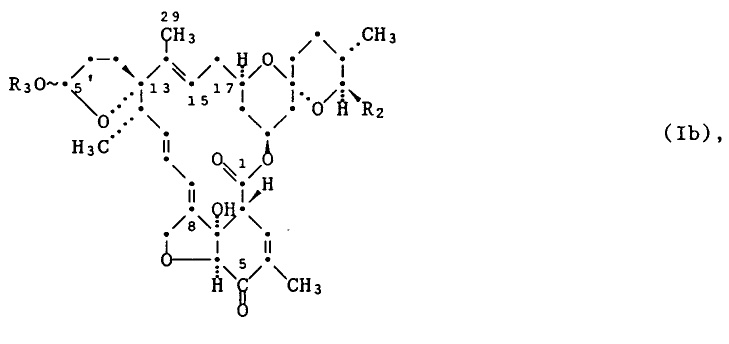 worin R₂ und R₃ die für Formel Ic angegebenen Bedeutungen haben, überführt und gewünschtenfalls die Verbindung der Formel Ib durch Umsetzung mit Hydroxylamin oder einem seiner Salze in die entsprechende Verbindung der Formel 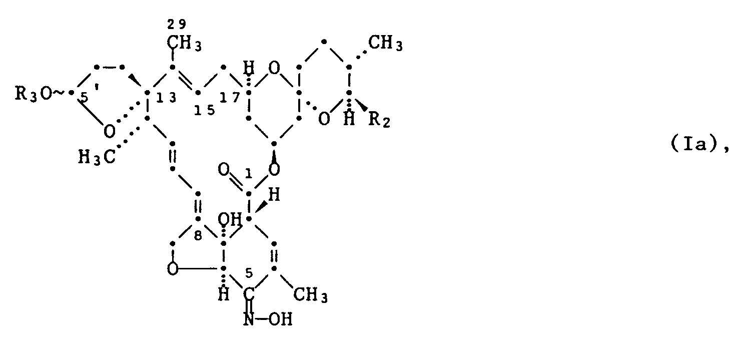 worin R₂ und R₃ die für Formel Ib angegebenen Bedeutungen haben, überführt.

2. Verfahren gemäss Anspruch 1 zur Herstellung einer Verbindung der Formel I, in welcher
X eine der Gruppen -CH(OR₁)- oder -C( = N-OH)- repräsentiert;
R₂ für Methyl, Ethyl, Isopropyl oder sek.Butyl steht; und
R₃ Wasserstoff, C₁-C₁₀-Alkyl, durch mindestens einen Substituenten der Gruppe Halogen, Ci-C₆-Alkoxy, C₂-C₆-Alkoxyalkoxy, C₃C₉-Alkoxyalkoxyalkoxy, C₁-C₆-Alkylthio, C₃-C₇-Cycloalkyl, Hydroxy und C₁-C₆-Acyl substituiertes C₁-C₁₀-Alkyl, wobei jeder der vorgängig genannten, eine Alkoxygruppe darstellenden oder enthaltenden Reste an einer endständigen Alkoxygruppe terminal durch Hydroxy, Halogen, C₁-C₆-Acyl oder C₁-C₆-Acyloxy substituiert sein kann, eine durch Benzyloxy substituierte Aethylgruppe, C₃-C₇-Cycloalkyl, durch mindestens einen Substituenten der Gruppe Halogen und C₁-C₃-Alkyl substituiertes C₃-C₇-Cycloalkyl, C₃-C₇-Cycloalkenyl, C₂-C₁₀-Alkenyl, C₂-C₁₀-Alkinyl, einen durch Halogen, C₁-C₆-Alkoxy oder Cᵢ-C₆-Acyloxy substituierten Rest aus der Gruppe C₂-C₁₀-Alkenyl und C₂-C₁₀-Alkinyl, 1-Adamantylmethyl, Menthyl, Carveyl, Phenyl , Benzyl, Naphthyl, einen durch mindestens einen Substituenten der Gruppe Halogen, C₁-C₃-Alkyl, C₁-C₃-Haloalkyl, C₁-C₃-Alkoxy, C₁-C₃-Halo- alkoxy, C₁-C₃-Alkylthio, Nitro und Cyano substituierten Rest aus der Gruppe Phenyl, Benzyl und Naphthyl, durch eine Phenoxygruppe substituiertes Benzyl, oder einen unsubstituierten oder durch mindestens einen Substituenten der Gruppe Halogen, C₁-C₃-Alkyl, C₁-C₃-Haloalkyl, C₁-C₃-Alkoxy, C₁-C₃-Haloalkoxy, C₁-C₃-Alkylthio, Nitro und Cyano substituierten vier- bis sechsgliedrigen Heterocyclus mit ein bis drei Heteroatomen aus der Gruppe Sauerstoff, Schwefel und Stickstoff repräsentiert, wobei besagter Heterocyclus auch über eine Ci-C₆-Alkylenbrücke an das Sauerstoffatom in 5'-Position des Tetrahydrofuranrings gebunden sein kann.

3. Verfahren gemäss Anspruch 2 zur Herstellung einer Verbindung der Formel I, worin X -CH(OR₁)-repräsentiert.

4. Verfahren gemäss Anspruch 1 zur Herstellung einer Verbindung der Formel I, worin X -CH(OR₁)-repräsentiert;
R₂ für Methyl, Ethyl, Isopropyl oder sek.Butyl steht; und
R₃ Wasserstoff, C₁-C₁₀-Alkyl, durch mindestens einen Substituenten der Gruppe Halogen, Ci-C₆-Alkoxy, C₂-C₆-Alkoxyalkoxy, C₃-C₉-Alkoxyalkoxyalkoxy, C₁-C₆-Alkylthio, C₃-C₇-Cycloalkyl, Hydroxy und C₁-C₆-Acyl substituiertes C₁-C₁₀-Alkyl, wobei jeder der vorgängig genannten, eine Alkoxygruppe darstellenden oder enthaltenden Reste an einer endständigen Alkoxygruppe terminal durch Hydroxy, Halogen, C₁-C₆-Acyl oder C₁-C₆-Acyloxy substituiert sein kann, C₃-C₇-Cycloalkyl, durch mindestens einen Substituenten der Gruppe Halogen und C₁-C₃-Alkyl substituiertes C₃-C₇-Cycloalkyl, C₃-C₇-Cycloalkenyl, C₂-C₁₀-Alkenyl, C₂-C₁₀-Alkinyl, einen durch Halogen, C₁-C₆-Alkoxy oder C₁-C₆-Acyloxy substituierten Rest aus der Gruppe C₂-C₁₀-Alkenyl und C₂-C₁₀-Alkinyl, 1-Adamantylmethyl, Menthyl, Carveyl, Phenyl , Benzyl, Naphthyl, einen durch mindestens einen Substituenten der Gruppe Halogen, C₁-C₃-Alkyl, Ci-C₃-Haloalkyl, C₁-C₃-Alkoxy, C₁-C₃-Haloalkoxy, C₁-C₃-Alkylthio, Nitro und Cyano substituierten Rest aus der Gruppe Phenyl, Benzyl und Naphthyl, oder einen unsubstituierten oder durch mindestens einen Substituenten der Gruppe Halogen, C₁-C₃-Alkyl, C₁-C₃-Haloalkyl, C₁-C₃-Alkoxy, C₁-C₃-Haloalkoxy, C₁-C₃-Alkylthio, Nitro und Cyano substituierten vier- bis sechsgliedrigen Heterocyclus mit ein bis drei Heteroatomen aus der Gruppe Sauerstoff, Schwefel und Stickstoff repräsentiert, wobei besagter Heterocyclus auch über eine C₁-C₆-Alkylenbrük- ke an das Sauerstoffatom in 5'-Position des Tetrahydrofuranrings gebunden sein kann.

5. Verfahren gemäss Anspruch 4 zur Herstellung einer Verbindung der Formel I, worin R₁ für Wasserstoff, R₄-C(O)- oder -Si(R₅)(R₆)(R₇) steht; wobei R₄ C₁-C₁₀-Alkyl, C₁-C₁₀-Haloalkyl oder einen unsubstituierten oder durch mindestens einen Substituenten der Gruppe Halogen, C₁-C₃-Alkyl, C₁-C₃-Haloalkyl, Ci-C₃-Alkoxy, C₁-C₃-Haloalkoxy, Cyano und Nitro substituierten Rest aus der Gruppe Phenyl und Benzyl bedeutet und Rₛ, R₆ und R₇ unabhängig voneinander für C₁-C₆-Alkyl, Benzyl oder Phenyl stehen; und R₃ Wasserstoff, C₁-C₅-Alkyl, durch mindestens einen Substituenten der Gruppe Halogen, C₁-C₃-Alkoxy, C₂-C₆-Alkoxyalkoxy, C₃-C₉-Alkoxyalkoxyalkoxy, C₁-C₃-Alkylthio, C₃-C₇-Cycloalkyl, Hydroxy und C₁-C₆-Acyl substituiertes C₁-C₅-Alkyl, wobei jeder der vorgängig genannten, eine Alkoxygruppe darstellenden oder enthaltenden Reste an einer endständigen Alkoxygruppe terminal durch Hydroxy, Halogen, C₁-C₆-Acyl oder C₁-C₆-Acyloxy substituiert sein kann; C₃-C₇-Cycloalkyl, durch mindestens einen Substituenten der Gruppe Fluor, Chlor, Brom und Methyl substituiertes C₃-C₇-Cycloalkyl, C₂-C₆-Alkenyl, C₂-C₆-Alkinyl, einen durch Fluor, Chlor, Brom, C₁-C₃-Alkoxy oder C₁-C₆-Acyloxy substituierten Rest aus der Gruppe C₂-C₆-Alkenyl und C₂-C₆-Alkinyl, Phenyl, Benzyl, a-Naphthyl, ß-Naphthyl, einen durch mindestens einen Substituenten der Gruppe Fluor, Chlor, Brom, Methyl, Methoxy, CF₃, CF₃0, CH₃S, Nitro und Cyano substituierten Rest aus der Gruppe Phenyl, Benzyl, a-Naphthyl und ß-Naphthyl, oder einen unsubstituierten oder durch mindestens einen Substituenten der Gruppe Fluor, Chlor, Brom, Methyl, Fthyl, CF₃, CH₃0, CF₃0, CH₃S, Nitro und Cyano substituierten vier- bis sechsgliedrigen Heterocyclus mit ein bis drei Heteroatomen aus der Gruppe Sauerstoff, Schwefel und Stickstoff repräsentiert, wobei besagter Heterocyclus auch über eine C₁-C₆-Alkylenbrücke an das Sauerstoffatom in 5'-Position des Tetrahydrofuranringes gebunden sein kann.

6. Verfahren gemäss Anspruch 5 zur Herstellung einer Verbindung der Formel I, worin R⁵, R⁶ und R⁷ unabhängig voneinander für C₁-C₄-Alkyl, Benzyl oder Phenyl stehen; und R₃ C₁-C₅-Alkyl oder durch mindestens einen Substituenten der Gruppe Halogen, C₁-C₃-Alkoxy, C₂-C₆-Alkoxyalkoxy, C₃-C₉-Alkoxy- alkoxyalkoxy, C₁-C₃-Alkylthio, C₃-C₇-Cycloalkyl, Hydroxy und C₁-C₆-Acyl substituiertes C₁-C₅-Alkyl, wobei jeder der vorgängig genannten, eine Alkoxygruppe darstellenden oder enthaltenden Reste an einer endständigen Alkoxygruppe terminal durch Hydroxy, Halogen, C₁-C₆-Acyl oder C₁-C₆-Acyloxy substituiert sein kann, repräsentiert.

7. Verfahren gemäss Anspruch 5 zur Herstellung einer Verbindung der Formel I, worin R₃ R⁵, R⁶ und R⁷ unabhängig voneinander für C₁-C₄-Alkyl, Benzyl oder Phenyl stehen; und C₃-C₇-Cycloalkyl, durch mindestens einen Substituenten der Gruppe Fluor, Chlor, Brom und Methyl substituiertes C₃-C₇-Cycloalkyl, C₂-C₆-Alkenyl, C₂-C₆-Alkinyl, einen durch Fluor, Chlor, Brom, C₁-C₃-Alkoxy oder C₁-C₆-Acyloxy substituierten Rest aus der Gruppe C₂-C₆-Alkenyl und C₂-C₆-Alkinyl, Phenyl, Benzyl, a-Naphthyl, β-Naphthyl, einen durch mindestens einen Substituenten der Gruppe Fluor, Chlor, Brom, Methyl, Methoxy, CF₃, CF₃0, CH₃S, Nitro und Cyano substituierten Rest aus der Gruppe Phenyl, Benzyl, a-Naphthyl und β-Naphthyl, oder einen unsubstituierten oder durch mindestens einen Substituenten der Gruppe Fluor, Chlor, Brom, Methyl, Ethyl, CF₃, CH₃0, CF₃0, CH₃S, Nitro und Cyano substituierten vier- bis sechsgliedrigen Heterocyclus mit ein bis drei Heteroatomen aus der Gruppe Sauerstoff, Schwefel und Stickstoff repräsentiert, wobei besagter Heterocyclus auch über eine Ci-C₆-Alkylenbrücke an das Sauerstoffatom in 5'-Position des Tetrahydrofuranringes gebunden sein kann.

8. Verfahren gemäss Anspruch 7 zur Herstellung einer Verbindung der Formel I, worin R₃ Phenyl, Benzyl, a-Naphthyl, β-Naphthyl, einen durch mindestens einen Substituenten der Gruppe Fluor, Chlor, Brom, Methyl, Methoxy, CF₃, CF₃0, CH₃S, Nitro und Cyano substituierten Rest aus der Gruppe Phenyl, Benzyl, a-Naphthyl und β-Naphthyl repräsentiert.

9. Verfahren gemäss Anspruch 7 zur Herstellung einer Verbindung der Formel I, worin R₃ einen unsubstituierten oder durch mindestens einen Substituenten der Gruppe Fluor, Chlor, Brom, Methyl, Ethyl, CF₃, CH₃0, CF₃O, CH₃S, Nitro und Cyano substituierten vier- bis sechsgliedrigen Heterocyclus mit ein bis drei Heteroatomen aus der Gruppe Sauerstoff, Schwefel und Stickstoff repräsentiert, wobei besagter Heterocyclus auch über eine C₁-C₆-Alkylenbrücke an das Sauerstoffatom in 5'-Position des Tetrahydrofuranringes gebunden sein kann.

10. Verfahren gemäss Anspruch 9 zur Herstellung einer Verbindung der Formel I, worin R₃ für einen ungesättigten viergliedrigen Heterocyclus mit einem Heteroatom aus der Gruppe Sauerstoff, Stickstoff und Schwefel steht oder Furan, Thiophen, Pyrrol, Isoxazol, Isothiazol, Furazan, Imidazol, 1,2,4-Triazol, 1,2,3-Triazol, Pyrazol, Pyrrolin, Oxazol, Thiazol, Thiadiazole, Pyrazolin, Thiazolin, Pyrazolidin, Pyrrolidin, Oxazolidin, Thiazolidin, Oxadiazol, Imidazolin, Imidazolidin, Pyrazolidin, Tetrahydrofuran, Pyridin, Pyridazin, Pyrimidin, Pyrazin, Thiazin, Thiadiazine, Pyrane, Piperidin, Piperazin, Morpholin, Perhydrothiazin oder Dioxan repräsentiert, wobei besagter Heterocyclus auch über eine C₁-C₄-Alkylenbrücke an das Sauerstoffatom in 5'-Position des Tetrahydrofuranrings gebunden sein kann.

11. Verfahren gemäss Anspruch 9 zur Herstellung einer Verbindung der Formel I, worin R₃ für einen gesättigten viergliedrigen Heterocyclus mit einem Heteroatom aus der Gruppe Sauerstoff, Stickstoff und Schwefel steht, wobei besagter Heterocyclus auch über eine C₁-C₄-Alkylenbrücke an das Sauerstoffatom in 5'-Position des Tetrahydrofuranrings gebunden sein kann.

12. Verfahren gemäss Anspruch 1 zur Herstellung einer Verbindung der Formel I, worin X für -CH(OR₁)-und R₁ für Wasserstoff steht; R₂ für Ethyl steht; und R₃ C₄-Cₛ-Alkyl, C₄-C₆-Cycloalkyl, über Methyl gebundenes C₄-C₆-Cycloalkyl, Phenyl, Benzyl oder a-Methylbenzyl repräsentiert.

13. Verfahren gemäss Anspruch 1 zur Herstellung einer Verbindung der Formel I, worin R₂ für Methyl oder Ethyl steht.

14. Verfahren gemäss Anspruch 13 zur Herstellung einer Verbindung der Formel I, worin R₂ für Ethyl steht.

15. Verfahren gemäss Anspruch 4 zur Herstellung einer Verbindung der Formel I, ausgewählt aus der
Gruppe von Verbindungen, bestehend aus
Milbemycin A₄-13-spiro-2'-[5'-(2"-ethoxy-ethoxy)-tetrahydrofuran],
Milbemycin A₄-13-spiro-2'-[5'-(2",2"-dimethyl-propoxy)-tetrahydrofuran],
Milbemycin A₄-13-spiro-2'-[5'-cyclohexyloxy-tetrahydrofuran],
Milbemycin A₄-13-spiro-2'-[5'-benzyloxy-tetrahydrofuran],
Milbemycin A₄-13-spiro-2'-[5'-{2"-(2"'-(methoxy-ethoxy)-ethoxyl-tetrahydrofuran],
Milbemycin A₄-13-spiro-2'-[5'-{2"-(2"'-(hydroxymethoxy)-ethoxy)-ethoxyl-tetrahydrofuran],
Milbemycin A4-13-spiro-2'-[5'-(2"-(2"'-(2''''(chloracetoxy)-ethoxy)-ethoxy)-ethoxyl-tetrahydrofuran],
Milbemycin A₄-13-spiro-2'-[5'-methoxy-tetrahydrofuran] und
Milbemycin A₄-13-spiro-2'-[5'-(2"-hydroxy-ethoxy)-tetrahydrofuran].

16. Verfahren gemäss Anspruch 12 zur Herstellung einer Verbindung der Formel I, ausgewählt aus der Gruppe von Verbindungen, bestehend aus Milbemycin A₄-13-spiro-2'-[5'-(2"-methyl-butoxy)-tetrahydrofuran] und Milbemycin A₄-13-spiro-2'-[5'-(1"-methyl-propoxy)-tetrahydrofuran].

17. Verfahren zur Herstellung einer Verbindung der Formel 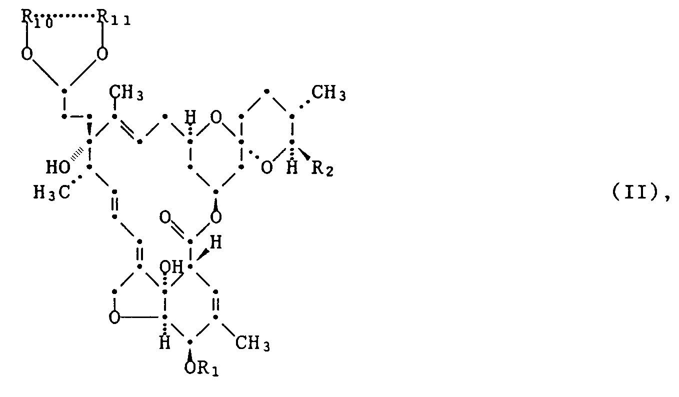 worin Ri, R₂, R₁₀ und R₁₁ die in Anspruch 1 angegebenen Bedeutungen haben, dadurch gekennzeichnet, dass man eine Verbindung der Formel 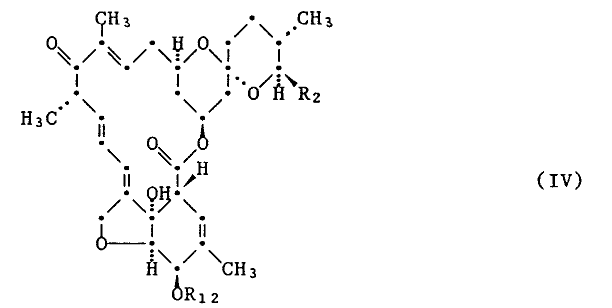 worin R₂ wie unter Formel I definiert ist und R₁₂ für Wasserstoff oder eine Silylgruppe steht, in einem reaktionsinerten Lösungsmittel mit einem Grignard-Reagenz der Formel 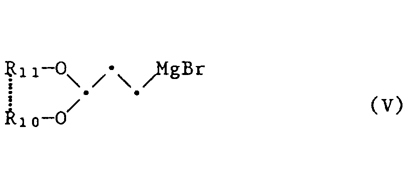 umsetzt.

18. Verfahren zur Herstellung eines Mittels zur Bekämpfung von Ektound Endoparasiten am Nutztier oder zur Bekämpfung von Schadinsekten, welches neben üblichen Trägerstoffen und Verteilungsmitteln als Wirkstoff eine nach Anspruch 1 erhältliche Verbindung der Formel I enthält, dadurch gekennzeichnet, dass man den Wirkstoff mit den Trägerstoffen und Verteilungsmitteln vermischt und/oder vermahlt.

19. Verfahren nach Anspruch 18, dadurch gekennzeichnet, dass das Mittel als Wirkstoff eine nach Anspruch 12 erhältliche Verbindung der Formel I enthält.

## Claims (Claims for the following Contracting State(s) : AT, BE, CH, DE, FR, GB, IT, LI, LU, NL, SE)

1. A compound of formula I 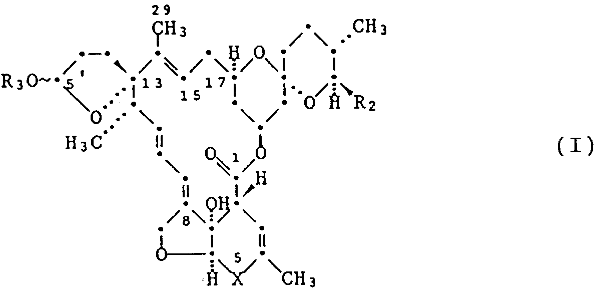 in which
X represents one of the groups -CH(OR₁)-, -C( = O)- and -C( = N-OH)-;
R₁ represents hydrogen or a OH-protecting group;
R₂ represents methyl, ethyl, isopropyl or sec-butyl or the group -C(CH₃) = CH-A in which A represents methyl, ethyl or isopropyl; and
R₃ represents hydrogen; C₁-C₁₀alkyl; C₁-C₁₀alkyl substituted by at least one substituent selected from the group consisting of halogen, C₁-C₆alkoxy, C₂-C₆alkoxyalkoxy, C₃-C^{g}alkoxyal- koxyalkoxy, C₁-C₆alkylthio, C₃-C₇cycloalkyl, C₁-C₃alkyl-substituted C₃-C₇cycloalkyl, hydroxy, benzyloxy, C₁-C₆acyl and C₁-C₆acyloxy, it being possible for each of the above-mentioned radicals representing or containing an alkoxy group to be terminally substituted at a terminal alkoxy group by hydroxy, halogen, C₁-C₆acyl or by Ci-C₆acyloxy; C₃-C₇cycloalkyl; C₃-C₇cycloalkyl substituted by at least one substituent selected from the group consisting of halogen and C₁-C₃alkyl; C₃-C₇cycloalkenyl; C₂-C₁oalkenyl; C₂-C₁oalkynyl; a radical selected from the group consisting of C₂-C₁ₒalkenyl and C₂-C₁ₒalkynyl, which radical is substituted by halogen, C₁-C₆alkoxy or by C₁-C₆acyloxy; 1-adamantylmethyl; menthyl; carveyl; phenyl; benzyl; naphthyl; a radical selected from the group consisting of phenyl, benzyl and naphthyl, which radical is substituted by at least one substituent selected from the group consisting of halogen, C₁-C₃alkyl, C₁-C₃haloalkyl, C₁-C₃alkoxy, C₁-C₃haloalkoxy, C₁-C₃alkylthio, nitro and cyano; benzyl substituted by a phenoxy group; or a four- to six-membered heterocyclic radical that has from one to three hetero atoms selected from the group consisting of oxygen, sulphur and nitrogen and that is unsubstituted or is substituted by at least one substituent selected from the group consisting of halogen, C₁-C₃alkyl, C₁-C₃haloalkyl, C₁-C₃alkoxy, Ci-C₃haloalkoxy, C₁-C₃alkylthio, nitro and cyano, it being possible for the said heterocyclic radical also to be bonded via a C₁-C₆alkylene bridge to the oxygen atom in the 5'-position of the tetrahydrofuran ring.

2. A compound of formula I according to claim 1, in which
X represents one of the groups -CH(OR₁)- and -C( = N-OH)-;
R₂ represents methyl, ethyl, isopropyl or sec-butyl; and
R₃ represents hydrogen; C₁-C₁₀alkyl; C₁-C₁₀alkyl substituted by at least one substituent selected from the group consisting of halogen, C₁-C₆alkoxy, C₂-C₆alkoxyalkoxy, C₃-C^{g}alkoxyal- koxyalkoxy, C₁-C₆alkylthio, C₃-C₇cycloalkyl, hydroxy and C₁-C₆acyl, it being possible for each of the above-mentioned radicals representing or containing an alkoxy group to be terminally substituted at a terminal alkoxy group by hydroxy, halogen, C₁-C₆acyl or by Ci-C₆acyloxy; an ethyl group substituted by benzyloxy; C₃-C₇cycloalkyl; C₃-C₇cycloalkyl substituted by at least one substituent selected from the group consisting of halogen and Ci-C₃alkyl; C₃-C₇cycloalkenyl; C₂-C₁oalkenyl; C₂-C₁oalkynyl; a radical selected from the group consisting of C₂-C₁₀alkenyl and C₂-C₁₀alkynyl, which radical is substituted by halogen, C₁-C₆alkoxy or by C₁-C₆ acyloxy; 1-adamantylmethyl; menthyl; carveyl; phenyl; benzyl; naphthyl; a radical selected from the group consisting of phenyl, benzyl and naphthyl, which radical is substituted by at least one substituent selected from the group consisting of halogen, Ci-C3alkyl, C₁-C₃haloalkyl, C₁-C₃alkoxy, C₁-C₃haloalkoxy,C₁-C₃alkylthio, nitro and cyano; benzyl substituted by a phenoxy group; or a four- to six-membered heterocyclic radical that has from one to three hetero atoms selected from the group consisting of oxygen, sulphur and nitrogen and that is unsubstituted or is substituted by at least one substituent selected from the group consisting of halogen, C₁-C₃alkyl, C₁-C₃haloalkyl, C₁-C₃alkoxy, C₁-C₃haloalkoxy,C₁-C₃alkylthio, nitro and cyano, it being possible for the said heterocyclic radical also to be bonded via a C₁-C₆alkylene bridge to the oxygen atom in the 5'-position of the tetrahydrofuran ring.

3. A compound of formula I according to claim 2, in which X represents -CH(OR₁)-.

4. A compound of formula I according to claim 1, in which
X represents -CH(OR₁)-;
R₂ represents methyl, ethyl, isopropyl or sec-butyl; and
R₃ represents hydrogen; C₁-C₁₀alkyl; C₁-C₁₀alkyl substituted by at least one substituent selected from the group consisting of halogen, C₁-C₆alkoxy, C₂-C₆alkoxyalkoxy, C₃-C^{g}alkoxyal- koxyalkoxy, C₁-C₆alkylthio, C₃-C₇cycloalkyl, hydroxy and C₁-C₆acyl, it being possible for each of the above-mentioned radicals representing or containing an alkoxy group to be terminally substituted at a terminal alkoxy group by hydroxy, halogen, C₁-C₆acyl or by Ci-C₆acyloxy; C₃-C₇cycloalkyl; C₃-C₇cycloalkyl substituted by at least one substituent selected from the group consisting of halogen and C₁-C₃alkyl; C₃-C₇cycloalkenyl; C₂-C₁oalkenyl; C₂-C₁₀alkynyl; a radical selected from the group consisting of C₂-C₁₀alkenyl and C₂-C₁oalkynyl, which radical is substituted by halogen, C₁-C₆alkoxy or by C₁-C₆acyloxy; 1-adamantylmethyl; menthyl; carveyl; phenyl; benzyl; naphthyl; a radical selected from the group consisting of phenyl, benzyl and naphthyl, which radical is substituted by at least one substituent selected from the group consisting of halogen, C₁-C₃alkyl, C₁-C₃haloalkyl, C₁-C₃alkoxy, C₁-C₃haloal- koxy, C₁-C₃alkylthio, nitro and cyano; or a four- to six-membered heterocyclic radical that has from one to three hetero atoms selected from the group consisting of oxygen, sulphur and nitrogen and that is unsubstituted or is substituted by at least one substituent selected from the group consisting of halogen, C₁-C₃alkyl, C₁-C₃haloalkyl, C₁-C₃alkoxy, C₁-C₃haloalkoxy, C₁-C₃alkylthio, nitro and cyano, it being possible for the said heterocyclic radical also to be bonded via a Ci-C₆-alkylene bridge to the oxygen atom in the 5'-position of the tetrahydrofuran ring.

5. A compound of formula I according to claim 4, in which
R₁ represents hydrogen, R4-C(O)- or -Si(R₅)(R₆)(R₇); wherein R₄ represents C₁-C₁₀alkyl, C₁-Cᵢₒhaloalkyl or a radical selected from the group consisting of phenyl and benzyl, which radical is unsubstituted or is substituted by at least one substituent selected from the group consisting of halogen, C₁-C₃alkyl, C₁-C₃-haloalkyl, C₁-C₃alkoxy, C₁-C₃haloalkoxy, cyano and nitro, and Rₛ, R₆ and R₇, independently of one another, represent Cᵢ-C₆alkyl, benzyl or phenyl; and
R₃ represents hydrogen; C₁-C₅alkyl; C₁-C₅alkyl substituted by at least one substituent selected from the group consisting of halogen, C₁-C₃alkoxy, C₂-C₆alkoxyalkoxy, C₃-Cgalkoxyalkoxyal- koxy, C₁-C₃alkylthio, C₃-C₇cycloalkyl, hydroxy and C₁-C₆acyl, it being possible for each of the above-mentioned radicals representing or containing an alkoxy group to be terminally substituted at a terminal alkoxy group by hydroxy, halogen, C₁-C₆acyl or by C₁-C₆acyloxy; C₃-C₇cycloalkyl; C₃-C₇cycloalkyl substituted by at least one substituent selected from the group consisting of fluorine, chlorine, bromine and methyl; C₂-C₆alkenyl; C₂-C₆alkynyl; a radical selected from the group consisting of C₂-C₆alkenyl and C₂-C₆alkynyl, which radical is substituted by fluorine, chlorine, bromine, C₁-C₃alkoxy or by C₁-C₆acyloxy; phenyl; benzyl; a-naphthyl; ß-naphthyl; a radical selected from the group consisting of phenyl, benzyl, a-naphthyl and ß-naphthyl, which radical is substituted by at least one substituent selected from the group consisting of fluorine, chlorine, bromine, methyl, methoxy, CF₃, CF₃0, CH₃S, nitro and cyano; or a four-to six-membered heterocyclic radical that has from one to three hetero atoms selected from the group consisting of oxygen, sulphur and nitrogen and that is unsubstituted or is substituted by at least one substituent selected from the group consisting of fluorine, chlorine, bromine, methyl, ethyl, CF₃, CH₃0, CF₃0, CH₃S, nitro and cyano, it being possible for the said heterocyclic radical also to be bonded via a C₁-C₆alkylene bridge to the oxygen atom in the 5'-position of the tetrahydrofuran ring.

6. A compound of formula I according to claim 5, in which
Rₛ, R₆ and R₇, independently of one another, represent C₁-C₄alkyl, benzyl or phenyl; and
R₃ represents Ci-Csalkyl, or Ci-Csalkyl substituted by at least one substituent selected from the group consisting of halogen, C₁-C₃alkoxy, C₂-C₆alkoxyalkoxy, C₃-C₉-alkoxyalkoxyalkoxy, C₁-C₃alkylthio, C₃-C₇cycloalkyl, hydroxy and Cᵢ-C₆acyl, it being possible for each of the above-mentioned radicals representing or containing an alkoxy group to be terminally substituted at a terminal alkoxy group by hydroxy, halogen, C₁-C₆acyl or by C₁-C₆acyloxy.

7. A compound of formula I according to claim 5, in which
Rₛ, R₆ and R₇, independently of one another, represent C₁-C₄alkyl, benzyl or phenyl; and
R₃ represents C₃-C₇cycloalkyl; C₃-C₇cycloalkyl substituted by at least one substituent selected from the group consisting of fluorine, chlorine, bromine and methyl; C₂-C₆alkenyl; C₂-C₆alkynyl; a radical selected from the group consisting of C₂-C₆alkenyl and C₂-C₆-alkynyl, which radical is substituted by fluorine, chlorine, bromine, C₁-C₃alkoxy or by C₁-C₆acyloxy; phenyl; benzyl; a-naphthyl; ß-naphthyl; a radical selected from the group consisting of phenyl, benzyl, a-naphthyl and β-naphthyl, which radical is substituted by at least one substituent selected from the group consisting of fluorine, chlorine, bromine, methyl, methoxy, CF₃, CF₃0, CH₃S, nitro and cyano; or a four- to six-membered heterocyclic radical that has from one to three hetero atoms selected from the group consisting of oxygen, sulphur and nitrogen and that is unsubstituted or is substituted by at least one substituent selected from the group consisting of fluorine, chlorine, bromine, methyl, ethyl, CF₃, CH₃0, CF₃0, CH₃S, nitro and cyano, it being possible for the said heterocyclic radical also to be bonded via a C₁-C₆alkylene bridge to the oxygen atom in the 5'-position of the tetrahydrofuran ring.

8. A compound of formula I according to claim 7, in which R₃ represents phenyl, benzyl, a-naphthyl, β-naphthyl or a radical selected from the group consisting of phenyl, benzyl, a-naphthyl and β-napthyl, which radical is substituted by at least one substituent selected from the group consisting of fluorine, chlorine, bromine, methyl, methoxy, CF₃, CF₃0, CH₃S, nitro and cyano.

9. A compound of formula I according to claim 7, in which R₃ represents a four- to six-membered heterocyclic radical that has from one to three hetero atoms selected from the group consisting of oxygen, sulphur and nitrogen and that is unsubstituted or is substituted by at least one substituent selected from the group consisting of fluorine, chlorine, bromine, methyl, ethyl, CF₃, CH₃0, CF₃0, CH₃S, nitro and cyano, it being possible for the said heterocyclic radical also to be bonded via a C₁-C₆-alkylene bridge to the oxygen atom in the 5'-position of the tetrahydrofuran ring.

10. A compound of formula I according to claim 9, in which R₃ represents an unsaturated four-membered heterocyclic radical having a hetero atom selected from the group consisting of oxygen, nitrogen and sulphur, or represents furan, thiophene, pyrrole, isoxazole, isothiazole, furazan, imidazole, 1,2,4-triazole, 1,2,3-triazole, pyrazole, pyrroline, oxazole, thiazole, thiadiazoles, pyrazoline, thiazoline, pyrazolidine, pyrrolidine, oxazolidine, thiazolidine, oxadiazole, imidazoline, imidazolidine, pyrazolidine, tetrahydrofuran, pyridine, pyridazine, pyrimidine, pyrazine, thiazine, thiadiazines, pyrans, piperidine, piperazine, morpholine, perhydrothiazine or dioxane, it being possible for the said heterocyclic radical also to be bonded via a C₁-C₄alkylene bridge to the oxygen atom in the 5'-position of the tetrahydrofuran ring.

11. A compound of formula I according to claim 9, in which R₃ represents a saturated four-membered heterocyclic radical having a hetero atom selected from the group consisting of oxygen, nitrogen and sulphur, it being possible for the said heterocyclic radical also to be bonded via a C₁-C₄alkylene bridge to the oxygen atom in the 5'-position of the tetrahydrofuran ring.

12. A compound of formula I according to claim 1, in which X represents -CH(OR₁)- and R₁ represents hydrogen; R₂ represents ethyl; and R₃ represents C₄-C₅alkyl, C4-C6-cycloalkyl, C4-C6cycloalkyl bonded via methyl, or phenyl, benzyl or a-methylbenzyl.

13. A compound of formula I according to claim 1 in which R₂ represents methyl or ethyl.

14. A compound of formula I according to claim 13, in which R₂ represents ethyl.

15. A compound of formula I according to claim 4, selected from the group:
milbemycin A4-13-spiro-2'-[5'-(2"-ethoxyethoxy)-tetrahydrofuran],
milbemycin A4-13-spiro-2'-[5'-(2",2"-dimethylpropoxy)-tetrahydrofuran],
milbemycin A4-13-spiro-2'-[5'-cyclohexyloxytetrahydrofuran],
milbemycin A4-13-spiro-2'-[5'-benzyloxytetrahydrofuran],
milbemycin A4-13-spiro-2'-[5'-{2"-(2"'-(methoxyethoxy)-ethoxy}-tetrahydrofuran],
milbemycin A₄-13-spiro-2'-[5'-{2"-(2'"-(hydroxymethoxy)-ethoxy)-ethoxy}-tetrahydrofuran],
milbemycin A₄-13-spiro-2'-[5'-{2"-(2'"-(2""-(chloroacetoxy)-ethoxy)-ethoxy)-ethoxy}-tetrahydrofuran],
milbemycin A4-13-spiro-2'-[5'-methoxytetrahydrofuran], and
milbemycin A4-13-spiro-2'-[5'-(2"-hydroxyethoxy)-tetrahydrofuran].

16. A compound of formula I according to claim 12, selected from the group: milbemycin A4-13-spiro-2'-[5'-(2"-methylbutoxy)-tetrahydrofuran] and milbemycin A4-13-spiro-2'-[5'-(1 "-methylpropoxy)-tetrahydrofuran].

17. A compound of formula II 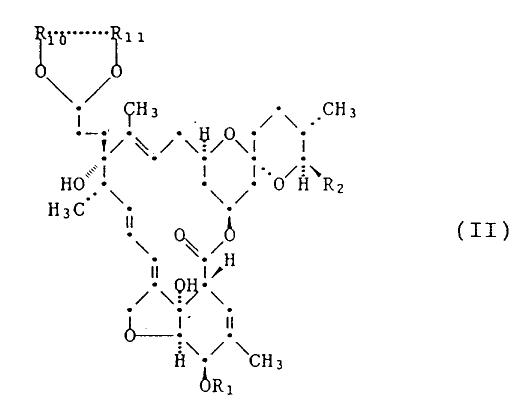 wherein R₁ and R₂ have the definitions given under formula I and R₁₀ and R₁₁, independently of one another, represent C₁-C₆alkyl or together form a C₂-C₁₀alkylene bridge.

18. A process for the preparation of a compound of formula I according to claim 1, characterised in that a compound of formula II according to claim 17 is reacted in the presence of an acid catalyst in an inert solvent with a compound of formula III 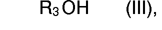 in which R₃ has the definition given under formula I, and, if desired, the resulting compound of formula Ic 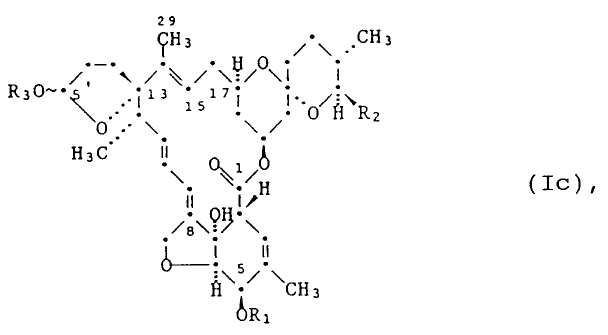 in which Ri, R₂ and R₃ have the meanings given for formula I, is converted by mild oxidation into a corresponding compound of formula Ib 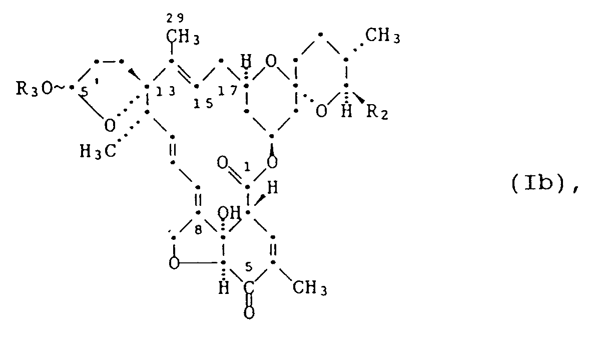 in which R₂ and R₃ have the meanings given for formula Ic, and, if desired, the compound of formula Ib is converted by reaction with hydroxylamine or a salt thereof into the corresponding compound of formula la 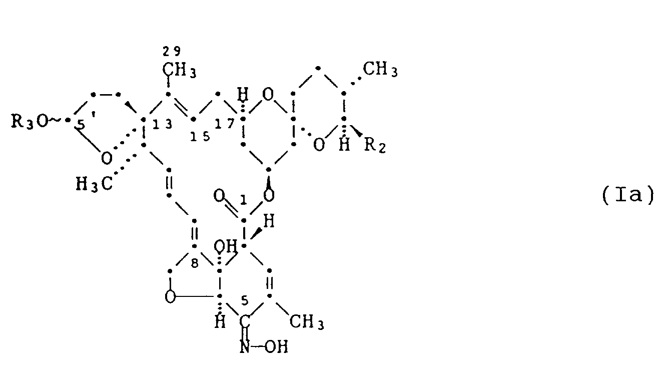 in which R₂ and R₃ have the meanings given for formula lb.

19. A process for the preparation of a compound of formula II according to claim 17, characterised in that a compound of formula IV 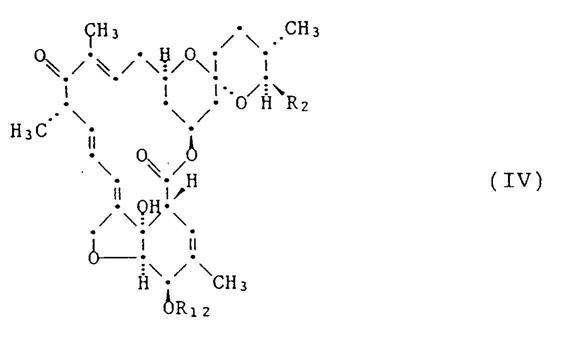 in which R₂ has the definition given under formula I and R₁₂ represents hydrogen or a silyl group, is reacted in an inert solvent with a Grignard reagent of formula V

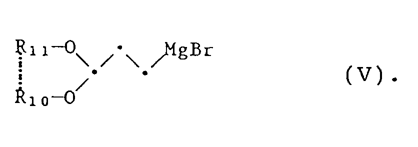

20. A composition for controlling ecto- and endo-parasites in productive livestock or for controlling pest insects, characterised in that, in addition to customary carriers and dispersing agents, it comprises a compound of formula I according to claim 1.

21. A composition according to claim 20, characterised in that it comprises as active ingredient a compound of formula I according to claim 12.

## Claims (Claims for the following Contracting State(s) : ES, GR)

1. A process for the preparation of a compound of formula 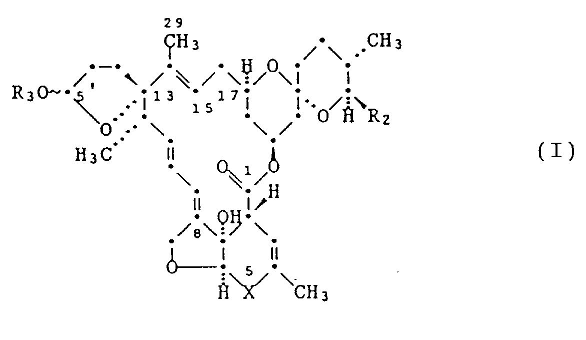 in which
X represents one of the groups -CH(OR₁)-, -C( = O)- and -C( = N-OH)-;
R₁ represents hydrogen or a OH-protecting group;
R₂ represents methyl, ethyl, isopropyl or sec-butyl or the group -C(CH₃) = CH-A in which A represents methyl, ethyl or isopropyl; and
R₃ represents hydrogen; C₁-C₁₀alkyl; C₁-C₁₀alkyl substituted by at least one substituent selected from the group consisting of halogen, C₁-C₆alkoxy, C₂-C₆alkoxyalkoxy, C₃-C^{g}alkoxyal- koxyalkoxy, C₁-C₆alkylthio, C₃-C₇cycloalkyl, C₁-C₃alkyl-substituted C₃-C₇cycloalkyl, hydroxy, benzyloxy, C₁-C₆acyl and C₁-C₆acyloxy, it being possible for each of the above-mentioned radicals representing or containing an alkoxy group to be terminally substituted at a terminal alkoxy group by hydroxy, halogen, Cᵢ-C₆acyl or by Ci-C₆acyloxy; C₃-C₇cycloalkyl; C₃-C₇cycloalkyl substituted by at least one substituent selected from the group consisting of halogen and C₁-C₃alkyl; C₃-C₇cycloalkenyl; C₂-C₁oalkenyl; C₂-C₁oalkynyl; a radical selected from the group consisting of C₂-C₁₀alkenyl and C₂-C₁₀-alkynyl, which radical is substituted by halogen, C₁-C₆alkoxy or by C₁-C₆acyloxy; 1-adamantylmethyl; menthyl; carveyl; phenyl; benzyl; naphthyl; a radical selected from the group consisting of phenyl, benzyl and naphthyl, which radical is substituted by at least one substituent selected from the group consisting of halogen, C₁-C₃alkyl, C₁-C₃haloalkyl, C₁-C₃alkoxy, C₁-C₃haloalkoxy, C₁-C₃alkylthio, nitro and cyano; benzyl substituted by a phenoxy group; or a four- to six-membered heterocyclic radical that has from one to three hetero atoms selected from the group consisting of oxygen, sulphur and nitrogen and that is unsubstituted or is substituted by at least one substituent selected from the group consisting of halogen, C₁-C₃-alkyl, C₁-C₃haloalkyl, C₁-C₃alkoxy, Ci-C₃haloalkoxy, C₁-C₃alkylthio, nitro and cyano, it being possible for the said heterocyclic radical also to be bonded via a C₁-C₆alkylene bridge to the oxygen atom in the 5'-position of the tetrahydrofuran ring,
characterised in that a compound of formula 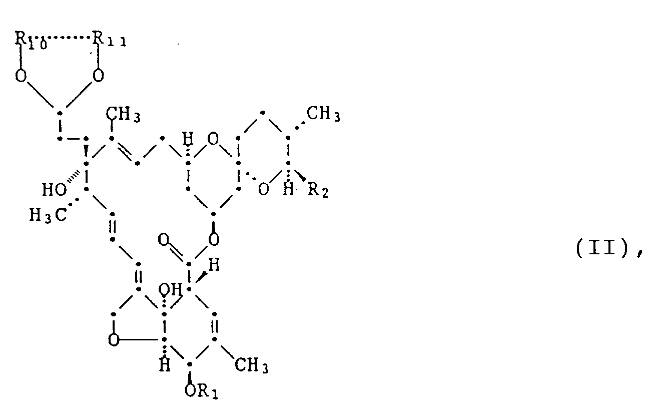 wherein R₁ and R₂ have the definitions given under formula I and R₁₀ and R₁₁, independently of one another, represent C₁-C₆alkyl or together form a C₂-C₁₀alkylene bridge, is reacted in the presence of an acid catalyst in an inert solvent with a compound of formula 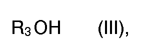 in which R₃ has the definition given under formula I, and, if desired, the resulting compound of formula 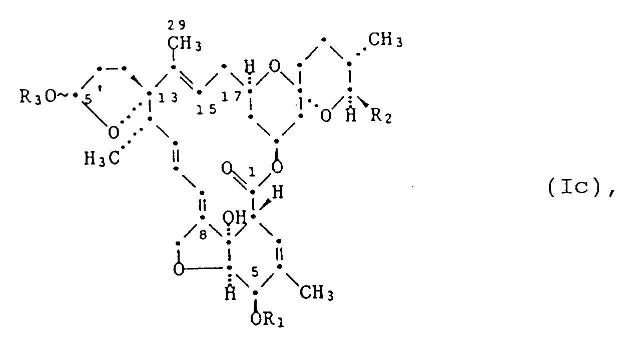 in which Ri, R₂ and R₃ have the meanings given for formula I, is converted by mild oxidation into a corresponding compound of formula 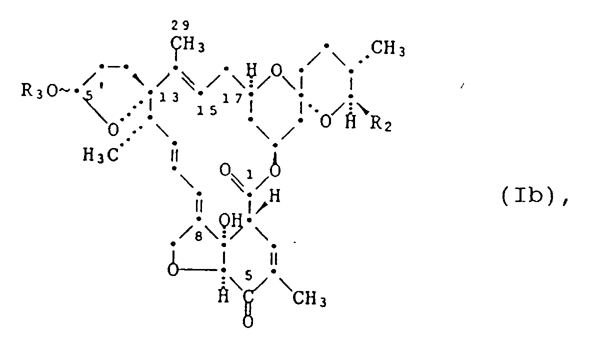 in which R₂ and R₃ have the meanings given for formula Ic, and, if desired, the compound of formula Ib is converted by reaction with hydroxylamine or a salt thereof into the corresponding compound of formula 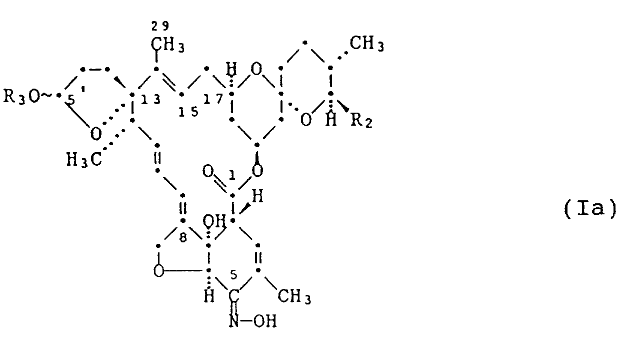 in which R₂ and R₃ have the meanings given for formula lb.

2. A process according to claim 1 for the preparation of a compound of formula I, in which
X represents one of the groups -CH(OR₁)- and -C( = N-OH)-;
R₂ represents methyl, ethyl, isopropyl or sec-butyl; and
R₃ represents hydrogen; C₁-C₁₀alkyl; C₁-C₁₀alkyl substituted by at least one substituent selected from the group consisting of halogen, C₁-C₆alkoxy, C₂-C₆alkoxyalkoxy, C₃-C^{g}alkoxyal- koxyalkoxy, C₁-C₆alkylthio, C₃-C₇cycloalkyl, hydroxy and C₁-C₆acyl, it being possible for each of the above-mentioned radicals representing or containing an alkoxy group to be terminally substituted at a terminal alkoxy group by hydroxy, halogen, C₁-C₆acyl or by Ci-C₆acyloxy; an ethyl group substituted by benzyloxy; C₃-C₇cycloalkyl; C₃-C₇cycloalkyl substituted by at least one substituent selected from the group consisting of halogen and Ci-C₃alkyl; C₃-C₇cycloalkenyl; C₂-C₁oalkenyl; C₂-C₁oalkynyl; a radical selected from the group consisting of C₂-C₁₀alkenyl and C₂-C₁₀alkynyl, which radical is substituted by halogen, C₁-C₆alkoxy or by C₁-C₆acyloxy; 1-adamantylmethyl; menthyl; carveyl; phenyl; benzyl; naphthyl; a radical selected from the group consisting of phenyl, benzyl and naphthyl, which radical is substituted by at least one substituent selected from the group consisting of halogen, C₁-C₃alkyl, C₁-C₃haloalkyl, C₁-C₃alkoxy, C₁-C₃haloalkoxy, C₁-C₃alkylthio, nitro and cyano; benzyl substituted by a phenoxy group; or a four- to six-membered heterocyclic radical that has from one to three hetero atoms selected from the group consisting of oxygen, sulphur and nitrogen and that is unsubstituted or is substituted by at least one substituent selected from the group consisting of halogen, C₁-C₃-alkyl, C₁-C₃haloalkyl, C₁-C₃alkoxy, C₁-C₃haloalkoxy, C₁-C₃alkylthio, nitro and cyano, it being possible for the said heterocyclic radical also to be bonded via a C₁-C₆-alkylene bridge to the oxygen atom in the 5'-position of the tetrahydrofuran ring.

3. A process according to claim 2 for the preparation of a compound of formula I in which X represents -CH(OR₁)-.

4. A process according to claim 1 for the preparation of a compound of formula I, in which
X represents -CH(OR₁)-;
R₂ represents methyl, ethyl, isopropyl or sec-butyl; and
R₃ represents hydrogen; C₁-C₁₀alkyl; C₁-C₁₀alkyl substituted by at least one substituent selected from the group consisting of halogen, C₁-C₆alkoxy, C₂-C₆alkoxyalkoxy, C₃-C^{g}alkoxyal- koxyalkoxy, C₁-C₆alkylthio, C₃-C₇cycloalkyl, hydroxy and C₁-C₆acyl, it being possible for each of the above-mentioned radicals representing or containing an alkoxy group to be terminally substituted at a terminal alkoxy group by hydroxy, halogen, C₁-C₆acyl or by Ci-C₆acyloxy; C₃-C₇cycloalkyl; C₃-C₇cycloalkyl substituted by at least one substituent selected from the group consisting of halogen and C₁-C₃alkyl; C₃-C₇cycloalkenyl; C₂-C₁oalkenyl; C₂-C₁₀alkynyl; a radical selected from the group consisting of C₂-C₁₀alkenyl and C₂-C₁₀-alkynyl, which radical is substituted by halogen, C₁-C₆alkoxy or by C₁-C₆acyloxy; 1-adamantylmethyl; menthyl; carveyl; phenyl; benzyl; naphthyl; a radical selected from the group consisting of phenyl, benzyl and naphthyl, which radical is substituted by at least one substituent selected from the group consisting of halogen, C₁-C₃alkyl, C₁-C₃haloalkyl, C₁-C₃alkoxy, C₁-C₃haloal- koxy, C₁-C₃alkylthio, nitro and cyano; or a four- to six-membered heterocyclic radical that has from one to three hetero atoms selected from the group consisting of oxygen, sulphur and nitrogen and that is unsubstituted or is substituted by at least one substituent selected from the group consisting of halogen, C₁-C₃alkyl, C₁-C₃haloalkyl, C₁-C₃alkoxy, C₁-C₃haloalkoxy, C₁-C₃alkylthio, nitro and cyano, it being possible for the said heterocyclic radical also to be bonded via a Ci-C₆alkylene bridge to the oxygen atom in the 5'-position of the tetrahydrofuran ring.

5. A process according to claim 4 for the preparation of a compound of formula I, in which
R₁ represents hydrogen, R4-C(O)- or -Si(R₅)(R₆)(R₇); wherein R₄ represents C₁-C₁₀alkyl, C₁-Cᵢₒhaloalkyl or a radical selected from the group consisting of phenyl and benzyl, which radical is unsubstituted or is substituted by at least one substituent selected from the group consisting of halogen, C₁-C₃alkyl, C₁-C₃haloalkyl, C₁-C₃alkoxy, C₁-C₃haloalkoxy, cyano and nitro, and Rₛ, R₆ and R₇, independently of one another, represent Cᵢ-C₆alkyl, benzyl or phenyl; and
R₃ represents hydrogen, C₁-C₅alkyl; C₁-C₅alkyl substituted by at least one substituent selected from the group consisting of halogen, C₁-C₃alkoxy, C₂-C₆alkoxyalkoxy, C₃-Cgalkoxyalkoxyal- koxy, C₁-C₃alkylthio, C₃-C₇cycloalkyl, hydroxy and C₁-C₆acyl, it being possible for each of the above-mentioned radicals representing or containing an alkoxy group to be terminally substituted at a terminal alkoxy group by hydroxy, halogen, C₁-C₆acyl or by C₁-C₆acyloxy; C₃-C₇cycloalkyl; C₃-C₇cycloalkyl substituted by at least one substituent selected from the group consisting of fluorine, chlorine, bromine and methyl; C₂-C₆-alkenyl; C₂-C₆alkynyl; a radical selected from the group consisting of C₂-C₆alkenyl and C₂-C₆alkynyl, which radical is substituted by fluorine, chlorine, bromine, C₁-C₃alkoxy or by C₁-C₆acyloxy; phenyl; benzyl; a-naphthyl; β-naphthyl; a radical selected from the group consisting of phenyl, benzyl, a-naphthyl and β-naphthyl, which radical is substituted by at least one substituent selected from the group consisting of fluorine, chlorine, bromine, methyl, methoxy, CF₃, CF₃0, CH₃S, nitro and cyano; or a four-to six-membered heterocyclic radical that has from one to three hetero atoms selected from the group consisting of oxygen, sulphur and nitrogen and that is unsubstituted or is substituted by at least one substituent selected from the group consisting of fluorine, chlorine, bromine, methyl, ethyl, CF₃, CH₃0, CF₃0, CH₃S, nitro and cyano, it being possible for the said heterocyclic radical also to be bonded via a C₁-C₆-alkylene bridge to the oxygen atom in the 5'-position of the tetrahydrofuran ring.

6. A process according to claim 5 for the preparation of a compound of formula I, in which
Rₛ, R₆ and R₇, independently of one another, represent C₁-C₄alkyl, benzyl or phenyl; and
R₃ represents C₁-C₅alkyl, or C₁-C₅alkyl substituted by at least one substituent selected from the group consisting of halogen, C₁-C₃alkoxy, C₂-C₆alkoxyalkoxy, C₃-Cgalkoxyalkoxyalkoxy, C₁-C₃alkylthio, C₃-C₇cycloalkyl, hydroxy and Cᵢ-C₆acyl, it being possible for each of the above-mentioned radicals representing or containing an alkoxy group to be terminally substituted at a terminal alkoxy group by hydroxy, halogen, C₁-C₆acyl or by C₁-C₆acyloxy.

7. A process according to claim 5 for the preparation of a compound of formula I, in which
Rₛ, R₆ and R₇, independently of one another, represent C₁-C₄alkyl, benzyl or phenyl; and
R₃ represents C₃-C₇cycloalkyl; C₃-C₇cycloalkyl substituted by at least one substituent selected from the group consisting of fluorine, chlorine, bromine and methyl; C₂-C₆alkenyl; C₂-C₆alkynyl; a radical selected from the group consisting of C₂-C₆alkenyl and C₂-C₆alkynyl, which radical is substituted by fluorine, chlorine, bromine, C₁-C₃alkoxy or by C₁-C₆acyloxy; phenyl; benzyl; a-naphthyl; ß-naphthyl; a radical selected from the group consisting of phenyl, benzyl, a-naphthyl and β-naphthyl, which radical is substituted by at least one substituent selected from the group consisting of fluorine, chlorine, bromine, methyl, methoxy, CF₃, CF₃0, CH₃S, nitro and cyano; or a four- to six-membered heterocyclic radical that has from one to three hetero atoms selected from the group consisting of oxygen, sulphur and nitrogen and that is unsubstituted or is substituted by at least one substituent selected from the group consisting of fluorine, chlorine, bromine, methyl, ethyl, CF₃, CH₃O, CF₃O, CH₃S, nitro and cyano, it being possible for the said heterocyclic radical also to be bonded via a C₁-C₆alkylene bridge to the oxygen atom in the 5'-position of the tetrahydrofuran ring.

8. A process according to claim 7 for the preparation of a compound of formula I, in which R₃ represents phenyl, benzyl, a-naphthyl, ß-naphthyl or a radical selected from the group consisting of phenyl, benzyl, a-naphthyl and ß-napthyl, which radical is substituted by at least one substituent selected from the group consisting of fluorine, chlorine, bromine, methyl, methoxy, CF₃, CF₃0, CH₃S, nitro and cyano.

9. A process according to claim 7 for the preparation of a compound of formula I, in which R₃ represents a four-to six-membered heterocyclic radical that has from one to three hetero atoms selected from the group consisting of oxygen, sulphur and nitrogen and that is unsubstituted or is substituted by at least one substituent selected from the group consisting of fluorine, chlorine, bromine, methyl, ethyl, CF₃, CH₃0, CF₃0, CH₃S, nitro and cyano, it being possible for the said heterocyclic radical also to be bonded via a C₁-C₆alkylene bridge to the oxygen atom in the 5'-position of the tetrahydrofuran ring.

10. A process according to claim 9 for the preparation of a compound of formula I, in which R₃ represents an un-saturated four-membered heterocyclic radical having a hetero atom selected from the group consisting of oxygen, nitrogen and sulphur, or represents furan, thiophene, pyrrole, isoxazole, isothiazole, furazan, imidazole, 1,2,4-triazole, 1,2,3-triazole, pyrazole, pyrroline, oxazole, thiazole, thiadiazoles, pyrazoline, thiazoline, pyrazolidine, pyrrolidine, oxazolidine, thiazolidine, oxadiazole, imidazoline, imidazolidine, pyrazolidine, tetrahydrofuran, pyridine, pyridazine, pyrimidine, pyrazine, thiazine, thiadiazines, pyrans, piperidine, piperazine, morpholine, perhydrothiazine or dioxane, it being possible for the said heterocyclic radical also to be bonded via a C1-C4alkylene bridge to the oxygen atom in the 5'-position of the tetrahydrofuran ring.

11. A process according to claim 9 for the preparation of a compound of formula I, in which R₃ represents a saturated four-membered heterocyclic radical having a hetero atom selected from the group consisting of oxygen, nitrogen and sulphur, it being possible for the said heterocyclic radical also to be bonded via a C₁-C₄-alkylene bridge to the oxygen atom in the 5'-position of the tetrahydrofuran ring.

12. A process according to claim 1 for the preparation of a compound of formula I, in which X represents -CH(OR₁)-and R₁ represents hydrogen; R₂ represents ethyl; and R₃ represents C₄-C₅alkyl, C₄-C₆cycloalkyl, C4-C6cycloalkyl bonded via methyl, or phenyl, benzyl or a-methylbenzyl.

13. A process according to claim 1 for the preparation of a compound of formula I in which R₂ represents methyl or ethyl.

14. A process according to claim 13 for the preparation of a compound of formula I in which R₂ represents ethyl.

15. A process according to claim 4 for the preparation of a compound of formula I selected from the group of compounds:
milbemycin A4-13-spiro-2'-[5'-(2"-ethoxyethoxy)-tetrahydrofuran],
milbemycin A4-13-spiro-2'-[5'-(2",2"-dimethylpropoxy)-tetrahydrofuran],
milbemycin A4-13-spiro-2'-[5'-cyclohexyloxytetrahydrofuran],
milbemycin A4-13-spiro-2'-[5'-benzyloxytetrahydrofuran],
milbemycin A4-13-spiro-2'-[5'-{2"-(2'''-(methoxyethoxy)-ethoxy}-tetrahydrofuran],
milbemycin A4-13-spiro-2'-[5'-{2"-(2'"-(hydroxymethoxy)-ethoxy)-ethoxy}-tetrahydrofuran],
milbemycin A4-13-spiro-2'-[5'-{2"-(2'"-(2""-(chloroacetoxy)-ethoxy)-ethoxy)-ethoxy}-tetrahydrofuran],
milbemycin A4-13-spiro-2'-[5'-methoxytetrahydrofuran], and
milbemycin A4-13-spiro-2'-[5'-(2"-hydroxyethoxy)-tetrahydrofuran].

16. A process according to claim 12 for the preparation of a compound of formula I selected from the group of compounds consisting of: milbemycin A4-13-spiro-2'-[5'-(2"-methylbutoxy)-tetrahydrofuran] and milbemycin A4-13-spiro-2'-[5'-(1 "-methylpropoxy)-tetrahydrofuran].

17. A process for the preparation of a compound of formula 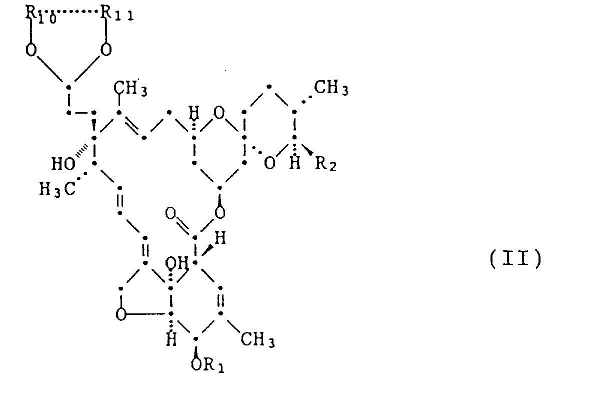 wherein R₁, R₂, R₁₀ and R₁₁ have the definitions given in claim 1, characterised in that a compound of formula 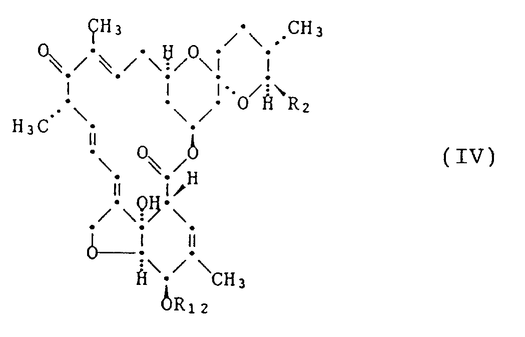 in which R₂ has the definition given under formula I and R₁₂ represents hydrogen or a silyl group, is reacted in an inert solvent with a Grignard reagent of formula

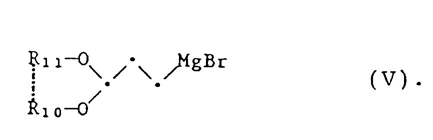

18. A process for the preparation of a composition for controlling ecto- and endo-parasites in productive livestock or for controlling pest insects, which in addition to customary carriers and dispersing agents, comprises as active ingredient a compound of formula I obtainable according to claim 1, characterised in that the active ingredient is mixed and/or ground with the carriers and dispersing agents.

19. A process according to claim 18, characterised in that the composition comprises as active ingredient a compound of formula I obtainable according to claim 12.

## Revendications (Revendications pour l'(les) Etat(s) contractant(s) suivant(s) : suivants : AT, BE, CH, DE, FR, GB, IT, LI, LU, NL, SE)

1. Un composé de formule 1 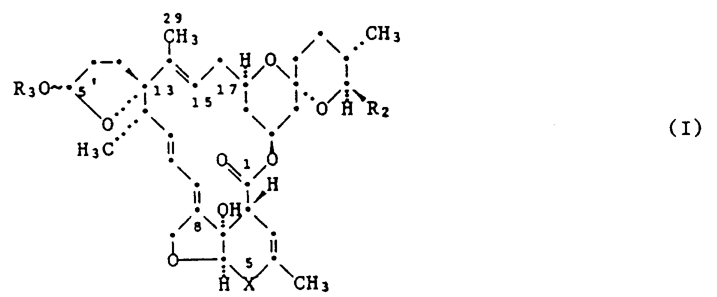 dans laquelle
X représente l'un des groupes -CH(OR,)-, -C( = O)- ou -C( = N-OH)-;
R₁ représente l'hydrogène ou un groupe protecteur du groupe OH;
R₂ représente un groupe méthyle, éthyle, isopropyle ou sec.-butyle ou le groupe -C(CH₃) = CH-A dans lequel A représente un groupe méthyle, éthyle ou isopropyle, et
R₃ représente l'hydrogène, un groupe alkyle en C 1-C 10, un groupe alkyle en C 1-C 10 portant au moins un substituant choisi parmi les halogènes, les groupes alcoxy en C 1-C 6, alcoxyalcoxy en C 2-C 6, alcoxyalcoxyalcoxy en C 3-C 9, alkylthio en C 1-C 6, cycloalkyle en C 3-C 7, cycloalkyle en C 3-C 7 à substituant alkyle en C 1-C 3, hydroxy, benzyloxy, acyle en C 1-C 6 et acyloxy en C 1-C 6, chacun des groupes mentionnés ci-dessus consistant en un groupe alcoxy ou contenant un groupe alcoxy pouvant être substitué en position terminale sur un groupe alcoxy terminal par un groupe hydroxy, un halogène, un groupe acyle en C 1-C 6 ou acyloxy en C 1-C 6, un groupe cycloalkyle en C 3-C 7, un groupe cycloalkyle en C 3-C 7 portant au moins un substituant choisi parmi les halogènes et les groupes alkyle en C 1-C 3, un groupe cycloalcényle en C 3-C 7, alcényle en C 2-C 10, alcynyle en C 2-C 10, un groupe, substitué par des halogènes, des groupes alcoxy en C 1-C 6 ou acyloxy en C 1-C 6, lui-même choisi parmi les groupes alcényle en C 2-C 10 et alcynyle en C 2-C 10, un groupe 1-adamantylméthyle, menthyle, carvéyle, phényle, benzyle, naphtyle, un groupe, portant au moins un substituant choisi parmi les halogènes, les groupes alkyle en C 1-C 3, halogénoalkyle en C 1-C 3, alcoxy en C 1-C 3, halogénoalcoxy en C 1-C 3, alkylthio en C 1-C 3, nitro et cyano, et choisi lui-même parmi les groupes phényle, benzyle et naphtyle, un groupe benzyle substitué par un groupe phénoxy ou un hétérocycle de 4 à 6 chaînons, non substitué ou portant au moins un substituant choisi parmi les halogènes, les groupes alkyle en C 1-C 3, halogénoalkyle en C 1-C 3, alcoxy en C 1-C 3, halogénoalcoxy en C 1-C 3, alkylthio en C 1-C 3, nitro et cyano, et contenant 1 à 3 hétéroatomes choisis parmi l'oxygène, le soufre et l'azote, cet hétérocycle pouvant également être relié par un pont alkylène en C 1-C 6 à l'atome d'oxygène en position 5' du cycle tétrahydrofuranne.

2. Un composé de formule 1 selon revendication 1, pour lequel
X représente l'un des groupes -CH(OR₁ )- ou -C( = N-OH)-;
R₂ représente un groupe méthyle, éthyle, isopropyle ou sec-butyle; et
R₃ représente l'hydrogène, un groupe alkyle en C 1-C 10, un groupe alkyle en C 1-C 10 portant au moins un substituant choisi parmi les halogènes, les groupes alcoxy en C 1-C 6, alcoxyalcoxy en C 2-C 6, alcoxyalcoxyalcoxy en C 3-C 9, alkylthio en C 1-C 6, cycloalkyle en C 3-C 7, hydroxy ou acyle en C 1-C 6, chacun des groupes mentionnés ci-dessus consistant en un groupe alcoxy ou contenant un groupe alcoxy pouvant être substitué en position terminale, sur un groupe alcoxy terminal, par un groupe hydroxy, un halogène, un groupe acyle en C 1-C 6 ou acyloxy en C 1-C 6, un groupe éthyle substitué par un groupe benzyloxy, un groupe cycloalkyle en C 3-C 7, un groupe cycloalkyle en C 3-C 7 portant au moins un substituant choisi parmi les halogènes et les groupes alkyle en C 1-C 3, un groupe cycloalcényle en C 3-C 7, alcényle en C 2-C 10, alcynyle en C 2-C 10, un groupe, substitué par des halogènes, des groupes alcoxy en C 1-C 6 ou acyloxy en C 1-C 6 et lui-même choisi parmi les groupes alcényle en C 2-C 10 et alcynyle en C 2-C 10, 1-adamantylméthyle, menthyle, carvéyle, phényle, benzyle, naphtyle, un groupe, portant au moins un substituant choisi parmi les halogènes, les groupes alkyle en C 1-C 3, halogénoalkyle en C 1-C 3, alcoxy en C 1-C 3, halogénoalcoxy en C 1-C 3, alkylthio en C 1-C 3, nitro et cyano, et choisi lui-même parmi les groupes phényle, benzyle et naphtyle, un groupe benzyle substitué par un groupe phénoxy, ou un hétérocycle de 4 à 6 chaînons non substitué ou portant au moins un substituant choisi parmi les halogènes, les groupes alkyle en C 1-C 3, halogénoalkyle en C 1-C 3, alcoxy en C 1-C 3, halogénoalcoxy en C 1-C 3, alkylthio en C 1-C 3, nitro et cyano, et qui contient 1 à 3 hétéroatomes choisis parmi l'oxygène, le soufre et l'azote, cet hétérocycle pouvant également être relié par l'intermédiaire d'un pont alkylène en C 1-C 6 à l'atome d'oxygène en position 5' du cycle tétrahydrofuranne.

3. Un composé de formule 1 selon revendication 2, pour lequel X représente -CH(OR₁ )-.

4. Un composé de formule 1 selon revendication 1, pour lequel X représente -CH(OR₁ )-;
R₂ représente un groupe méthyle, éthyle, isopropyle ou sec-butyle; et
R₃ représente l'hydrogène, un groupe alkyle en C 1-C 10, un groupe alkyle en C 1-C 10 portant au moins un substituant choisi parmi les halogènes, les groupes alcoxy en C 1-C 6, alcoxyalcoxy en C 2-C 6, alcoxyalcoxyalcoxy en C 3-C 9, alkylthio en C 1-C 6, cycloalkyle en C 3-C 7, hydroxy et acyle en C 1-C 6, chacun des groupes mentionnés consistant en un groupe alcoxy ou contenant un groupe alcoxy pouvant être substitué en position terminale, sur un groupe alcoxy terminal, par un groupe hydroxy, un halogène, un groupe acyle en C 1-C 6 ou acyloxy en C 1-C 6, un groupe cycloalkyle en C 3-C 7, un groupe cycloalkyle en C 3-C 7 portant au moins un substituant choisi parmi les halogènes et les groupes alkyle en C 1-C 3, un groupe cycloalcényle en C 3-C 7, alcényle en C 2-C 10, alcynyle en C 2-C 10, un groupe, substitué par des halogènes, des groupes alcoxy en C 1-C 6 ou acyloxy en C 1-C 6 et lui-même choisi parmi les groupes alcényle en C 2-C 10 et alcynyle en C 2-C 10, 1- adamantylméthyle, menthyle, carvéyle, phényle, benzyle, naphtyle, un groupe, portant au moins un substituant choisi parmi les halogènes, les groupes alkyle en C 1-C 3, halogénoalkyle en C 1-C 3, alcoxy en C 1-C 3, halogénoalcoxy en C 1-C 3, alkylthio en C 1-C 3, nitro et cyano, et lui-même choisi parmi les groupes phényle, benzyle et naphtyle, ou un hétérocycle de 4 à 6 chaînons non substitué ou portant au moins un substituant choisi parmi les halogènes, les groupes alkyle en C 1-C 3, halogénoalkyle en C 1-C 3, alcoxy en C 1-C 3, halogénoalcoxy en C 1-C 3, alkylthio en C 1-C 3, nitro et cyano, et qui contient de 1 à 3 hétéroatomes choisis parmi l'oxygène, le soufre et l'azote, cet hétérocycle pouvant également être relié par l'intermédiaire d'un pont alkylène en C 1-C 6 à l'atome d'oxygène en position 5' du cycle tétrahydrofuranne.

5. Un composé de formule 1 selon revendication 4, pour lequel R₁ représente l'hydrogène, un groupe R₄-C(O)- ou -Si(R₅)(R₆)(R₇); R₄ représente un groupe alkyle en C 1-C 10, halogénoalkyle en C 1-C 10 ou un groupe, non substitué ou portant au moins un substituant choisi parmi les halogènes, les groupes alkyle en C 1-C 3, halogénoalkyle en C 1-C 3, alcoxy en C 1-C 3, halogénoalcoxy en C 1-C 3, cyano et nitro, et lui-même choisi parmi les groupes phényle et benzyle, et Rₛ, R₆ et R₇ représentent chacun, indépendamment les uns des autres, un groupe alkyle en C 1-C 6, benzyle ou phényle; R₂ représente un groupe méthyle, éthyle, isopropyle ou secbutyle; et R₃ représente l'hydrogène, un groupe alkyle en C 1-C 5, un groupe alkyle en C 1-C 5 portant au moins un substituant choisi parmi les halogènes, les groupes alcoxy en C 1-C 3, alcoxyalcoxy en C 2-C 6, alcoxyalcoxyalcoxy en C 3-C 9, alkylthio en C 1-C 3, cycloalkyle en C 3-C 7, hydroxy et acyle en C 1-C 6, chacun des groupes mentionnés consistant en un groupe alcoxy ou contenant un groupe alcoxy pouvant être substitué en position terminale sur un groupe alcoxy terminal par un groupe hydroxy, un halogène, un groupe acyle en C 1-C 6 ou acyloxy en C 1-C 6; un groupe cycloalkyle en C 3-C 7, un groupe cycloalkyle en C 3-C 7 portant au moins un substituant choisi parmi le fluor, le chlore, le brome et les groupes méthyle, un groupe alcényle en C 2-C 6, un groupe alcynyle en C 2-C 6, un groupe, substitué par le fluor, le chlore, le brome, des groupes alcoxy en C 1-C 3 ou acyloxy en C 1-C 6, et lui-même choisi parmi les groupes alcényle en C 2-C 6 et alcynyle en C 2-C 6, phényle, benzyle, alpha-naphtyle, bêta-naphtyle, un groupe, portant au moins un substituant choisi parmi le fluor, le chlore, le brome, les groupes méthyle, méthoxy, CF₃, CF₃0, CH₃S, nitro et cyano, et lui-même choisi parmi les groupes phényle, benzyle, alpha-naphtyle et bêta-naphtyle, ou un hétérocycle de 4 à 6 chaînons, non substitué ou portant au moins un substituant choisi parmi le fluor, le chlore, le brome, les groupes méthyle, éthyle, CF₃, CF₃0, CF₃S, nitro et cyano, et contenant 1 à 3 hétéroatomes choisis parmi l'oxygène, le soufre et l'azote, cet hétérocycle pouvant également être relié par un pont alkylène en C 1-C 6 à l'atome d'oxygène en position 5' du cycle tétrahydrofuranne.

6. Un composé de formule 1 selon revendication 5, pour lequel Rs, R₆ et R₇ représentent chacun, indépendamment les uns des autres, un groupe alkyle en C 1-C 4, benzyle ou phényle; et R₃ représente un groupe alkyle en C 1-C 5 ou un groupe alkyle en C 1-C 5 portant au moins un substituant choisi parmi les halogènes, les groupes alcoxy en C 1-C 3, alcoxyalcoxy en C 2-C 6, alcoxyalcoxyalcoxy en C 3-C 9, alkylthio en C 1-C 3, cycloalkyle en C 3-C 7, hydroxy et acyle en C 1-C 6, chacun des groupes qu'on vient de mentionner consistant en un groupe alcoxy ou contenant un groupe alcoxy pouvant être substitué en position terminale sur un groupe alcoxy terminal par un groupe hydroxy, un halogène, un groupe acyle en C 1-C 6 ou acyloxy en C 1-C 6.

7. Un composé de formule 1 selon revendication 5, pour lequel Rs, R₆ et R₇ représentent chacun, indépendamment les uns des autres, un groupe alkyle en C 1-C 4, benzyle ou phényle, et R₃ représente un groupe cycloalkyle en C 3-C 7, un groupe cycloalkyle en C 3-C 7 portant au moins un substituant choisi parmi le fluor, le chlore, le brome et les groupes méthyle, un groupe alcényle en C 2-C 6, alcynyle en C 2-C 6, un groupe, substitué par le fluor, le chlore, le brome, des groupes alcoxy en C 1-C 3 ou acyloxy en C 1-C 6, et lui-même choisi parmi les groupes alcényle en C 2-C 6 et alcynyle en C 2-C 6, un groupe phényle, benzyle, alpha-naphtyle, bêta-naphtyle, un groupe, portant au moins un substituant choisi parmi le fluor, le chlore, le brome, les groupes méthyle, méthoxy, CF₃, CF₃0, CH₃S, nitro et cyano et lui-même choisi parmi les groupes phényle, benzyle, alpha-naphtyle et bêta-naphtyle, ou bien un hétérocycle de 4 à 6 chaînons non substitué ou portant au moins un substituant choisi parmi le fluor, le chlore, le brome, les groupes méthyle, éthyle, CF₃, CH₃0, CF₃0, CH₃S, nitro et cyano, qui contient 1 à 3 hétéroatomes choisis parmi l'oxygène, le soufre et l'azote et qui peut être relié par un pont alkylène en C 1-C 6 à l'atome d'oxygène en position 5' du cycle tétrahydrofuranne.

8. Un composé de formule 1 selon revendication 7, pour lequel R₃ représente un groupe phényle, benzyle, alpha-naphtyle, bêta-naphtyle, un groupe, portant au moins un substituant choisi parmi le fluor, le chlore, le brome, les groupes méthyle, méthoxy, CF₃, CF₃0, CH₃S, nitro et cyano, et lui-même choisi parmi les groupes phényle, benzyle, alpha-naphtyle et bêta-naphtyle.

9. Composé de formule 1 selon revendication 7, pour lequel R₃ représente un hétérocycle de 4 à 6 chaînons non substitué ou portant au moins un substituant choisi parmi le fluor, le chlore, le brome, les groupes méthyle, éthyle, CF₃, CH₃0, CF₃0, CH₃S, nitro et cyano, et qui contient 1 à 3 hétéroatomes choisis parmi l'oxygène, le soufre et l'azote, cet hétérocycle pouvant également être relié par un pont alkylène en C 1-C 6 à l'atome d'oxygène en position 5' du cycle tétrahydrofuranne.

10. Un composé de formule 1 selon revendication 9, pour lequel R₃ représente un hétérocycle insaturé à 4 chaînons contenant un hétéroatome choisi parmi l'oxygène, l'azote ou le soufre, ou un cycle furanne, thiophène, pyrrole, isoxazole, isothiazole, furazanne, imidazole, 1,2,4-triazole, 1,2,3-triazole, pyrazole, pyrroline, oxazole, thiazole, thiadiazole, pyrazoline, thiazoline, pyrazolidine, pyrrolidine, oxazolidine, thiazolidine, oxadiazole, imidazoline, imidazolidine, pyrazolidine, tétrahydrofuranne, pyridine, pyridazine, pyrimidine, pyrazine, thiazine, thiadiazine, pyranne, pipéridine, pipérazine, morpholine, perhydrothiazine ou dioxanne, cet hétérocycle pouvant également être relié par un pont alkylène en C 1-C 4 à l'atome d'oxygène en position 5' du cycle tétrahydrofuranne.

11. Un composé de formule 1 selon revendication 9, pour lequel R₃ représente un hétérocycle saturé de 4 chaînons contenant un hétéroatome choisi parmi l'oxygène, l'azote et le soufre, cet hétérocycle pouvant également être relié par un pont alkylène en C 1-C 4 à l'atome d'oxygène en position 5' du cycle tétrahydrofuranne.

12. Un composé de formule 1 selon revendication 1, pour lequel X représente -CH(OR1)- et R₁ représente l'hydrogène; R₂ un groupe éthyle; et R₃ un groupe alkyle en C 4-C 5, cycloalkyle en C 4-C 6, cycloalkyle en C 4-C 6 relié par un groupe méthyle, phényle, benzyle ou alpha-méthylbenzyle.

13. Un composé de formule 1 selon revendication 1, pour lequel R₂ représente un groupe méthyle ou éthyle.

14. Un composé de formule 1 selon revendication 13, pour lequel R₂ représente un groupe éthyle.

15. Un composé de formule 1 selon revendication 4, choisi dans le groupe des suivants :
le milbémycine A₄-13-spiro-2'-[5'-(2"-éthoxy-éthoxy)-tétrahydrofuranne],
le milbémycine A₄-13-spiro-2'-[5'-(2",2"-diméthyl-propoxy)-tétrahydrofuranne],
le milbémycine A₄-13-spiro-2'-[5'-cyclohexyloxy-tétrahydrofuranne] ,
le milbémycine A₄-13-spiro-2'-[5'-benzyloxy-tétrahydrofuranne],
le milbémycine A₄-13-spiro-2'-[5'-{2"-(2"'-méthoxy-éthoxy)-éthoxyl-tétrahydrofuranne],
le milbémycine A₄-13-spiro-2'-[5'-{2"-(2"'-(hydroxy-méthoxy)-éthoxy)-éthoxyl-tétrahydrofuranne],
le milbémycine A₄-13-spiro-2'-[5'-{2"-(2"'-(2""-(chloracétoxy)-éthoxy)-éthoxy)-éthoxyl-tétrahydrofuranne],
le milbémycine A₄-13-spiro-2'-[5'-méthoxy-tétrahydrofuranne] et
le milbémycine A₄-13-spiro-2'-[5'-(2"-hydroxy-éthoxy)-tétrahydrofuranne].

16. Un composé de formule 1 selon revendication 12, choisi dans le groupe formé par le milbémycine A₄-13-spiro-2'-[5'-(2"-méthyl-butoxy)-tétrahydrofuranne] et le milbémycine A₄-13-spiro-2'-[5'-(1"-méthyl- propoxy)-tétrahydrofuranne].

17. Un composé de formule II 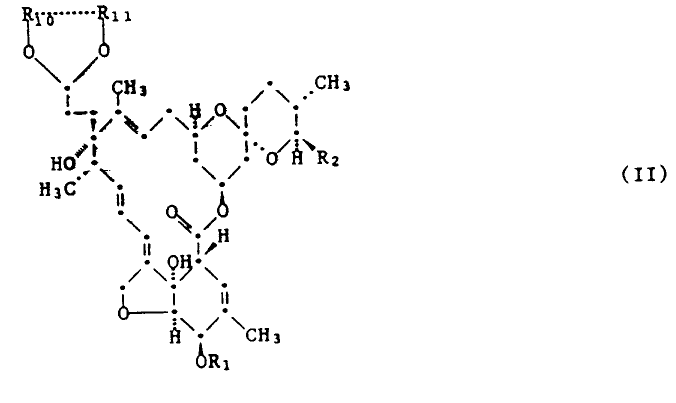 dans laquelle R₁ et R₂ ont les significations indiquées en référence à la formule I et R_{1 10} et R₁₁ représentent chacun, indépendamment l'un de l'autre, un groupe alkyle en C 1-C 6 ou forment ensemble un pont alkylène en C 2-C 10.

18. Procédé de préparation d'un composé de formule I selon revendication 1, caractérisé en ce que l'on fait réagir un composé de formule II de la revendication 17, en présence d'un catalyseur acide, dans un solvant inerte dans la réaction, avec un composé de formule III 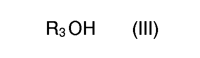 dans laquelle R₃ a les significations indiquées en référence à la formule I, et si on le désire, on convertit le composé ainsi obtenu, répondant à la formule Ic 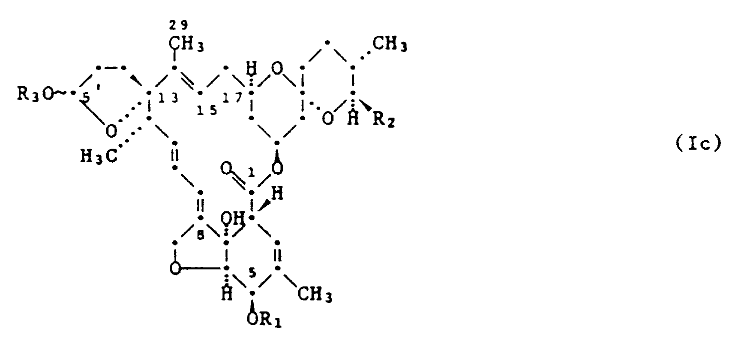 dans laquelle Ri, R₂ et R₃ ont les significations indiquées en référence à la formule I, par oxydation ménagée, en le composé correspondant de formule Ib 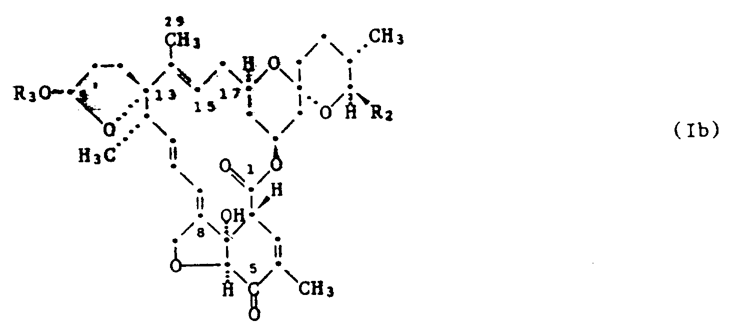 dans laquelle R₂ et R₃ ont les significations indiquées en référence à la formule Ic et si on le désire, on convertit le composé de formule Ib, par réaction avec l'hydroxylamine ou l'un de ses sels, en le composé correspondant de formule la 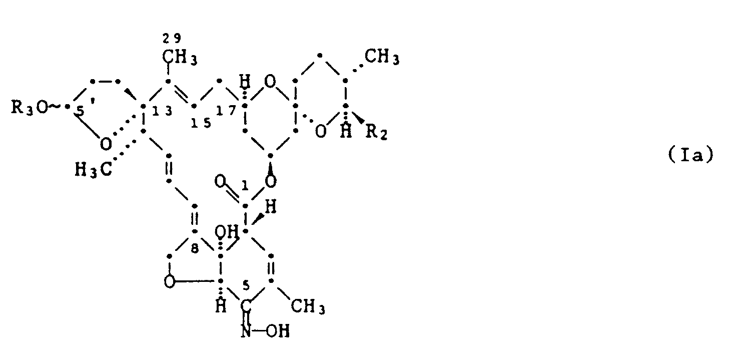 dans laquelle R₂ et R₃ ont les significations indiquées en référence à la formule Ib.

19. Procédé de préparation d'un composé de formule II selon revendication 17, caractérisé en ce que l'on fait réagir un composé de formule IV 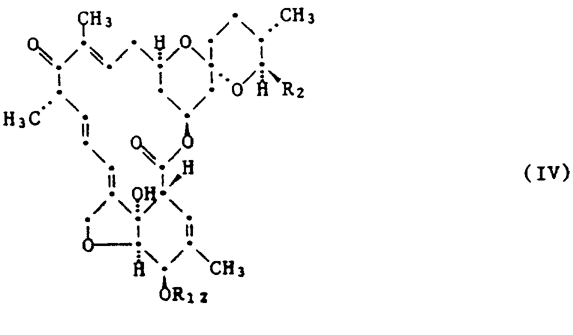 dans laquelle R₂ a les significations indiquées en référence à la formule I, et R₁₂ représente l'hydrogène ou un groupe silyle, dans un solvant inerte dans la réaction, avec un réactif de Grignard de formule V

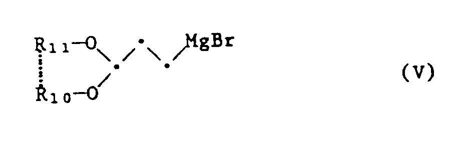

20. Produit pour combattre les ecto- et endo-parasites des animaux utiles ou pour combattre les insectes nuisibles, caractérisé en ce qu'il contient, avec des véhicules et des diluants usuels, un composé de formule 1 selon revendication 1.

21. Produit selon revendication 20, caractérisé en ce qu'il contient en tant que substance active un composé de formule I de la revendication 12.

## Revendications (Revendications pour l'(les) Etat(s) contractant(s) suivant(s) : suivants : ES, GR)

1. Procédé de préparation d'un composé de formule 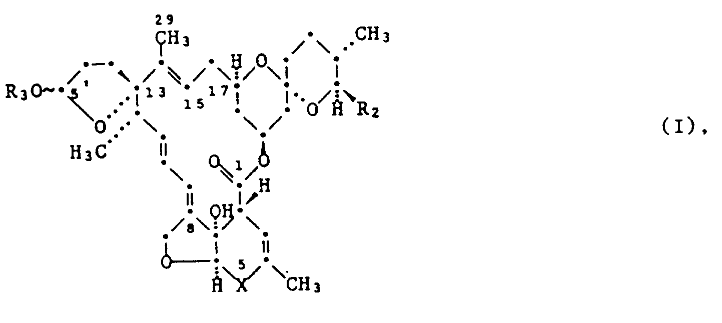 dans laquelle
X représente l'un des groupes -CH(OR₁ )-, -C( = O)- ou -C( = N-OH)-;
R₁ représente l'hydrogène ou un groupe protecteur du groupe OH;
R₂ représente un groupe méthyle, éthyle, isopropyle ou sec.-butyle ou le groupe -C(CH₃) = CH-A dans lequel A représente un groupe méthyle, éthyle ou isopropyle, et
R₃ représente l'hydrogène, un groupe alkyle en C 1-C 10, un groupe alkyle en C 1-C 10 portant au moins un substituant choisi parmi les halogènes, les groupes alcoxy en C 1-C 6, alcoxyalcoxy en C 2-C 6, alcoxyalcoxyalcoxy en C 3-C 9, alkylthio en C 1-C 6, cycloalkyle en C 3-C 7, cycloalkyle en C 3-C 7 à substituant alkyle en C 1-C 3, hydroxy, benzyloxy, acyle en C 1-C 6 et acyloxy en C 1-C 6, chacun des groupes mentionnés ci-dessus consistant en un groupe alcoxy ou contenant un groupe alcoxy pouvant être substitué en position terminale sur un groupe alcoxy terminal par un groupe hydroxy, un halogène, un groupe acyle en C 1-C 6 ou acyloxy en C 1-C 6, un groupe cycloalkyle en C 3-C 7, un groupe cycloalkyle en C 3-C 7 portant au moins un substituant choisi parmi les halogènes et les groupes alkyle en C 1-C 3, un groupe cycloalcényle en C 3-C 7, alcényle en C 2-C 10, alcynyle en C 2-C 10, un groupe, substitué par des halogènes, des groupes alcoxy en C 1-C 6 ou acyloxy en C 1-C 6, lui-même choisi parmi les groupes alcényle en C 2-C 10 et alcynyle en C 2-C 10, un groupe 1-adamantylméthyle, menthyle, carvéyle, phényle, benzyle, naphtyle, un groupe, portant au moins un substituant choisi parmi les halogènes, les groupes alkyle en C 1-C 3, halogénoalkyle en C 1-C 3, alcoxy en C 1-C 3, halogénoalcoxy en C 1-C 3, alkylthio en C 1-C 3, nitro et cyano, et choisi lui-même parmi les groupes phényle, benzyle et naphtyle, un groupe benzyle substitué par un groupe phénoxy ou un hétérocycle de 4 à 6 chaînons, non substitué ou portant au moins un substituant choisi parmi les halogènes, les groupes alkyle en C 1-C 3, halogénoalkyle en C 1-C 3, alcoxy en C 1-C 3, halogénoalcoxy en C 1-C 3, alkylthio en C 1-C 3, nitro et cyano, et contenant 1 à 3 hétéroatomes choisis parmi l'oxygène, le soufre et l'azote, cet hétérocycle pouvant également être relié par un pont alkylène en C 1-C 6 à l'atome d'oxygène en position 5' du cycle tétrahydrofuranne,
caractérisé en ce que l'on fait réagir un composé de formule Il 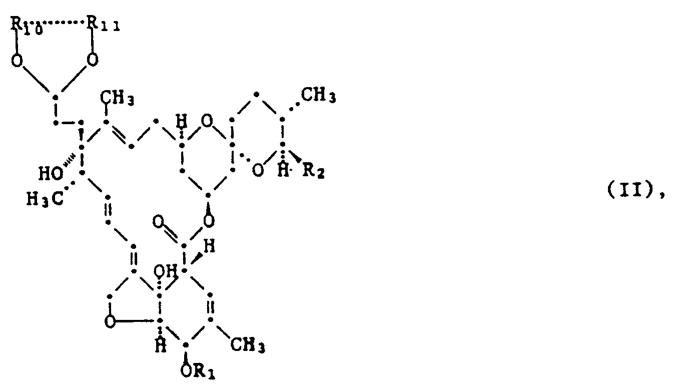 dans laquelle R₁ et R₂ ont les significations indiquées en référence à la formule I et R₁₀ et R₁₁ représentent chacun, indépendamment l'un de l'autre, un groupe alkyle en C 1-C 6 ou forment ensemble un pont alkylène en C 2-C 10, en présence d'un catalyseur acide, dans un solvant inerte dans la réaction, avec un composé de formule III 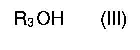 dans laquelle R₃ a les significations indiquées en référence à la formule I, et si on le désire, on convertit le composé ainsi obtenu, répondant à la formule Ic 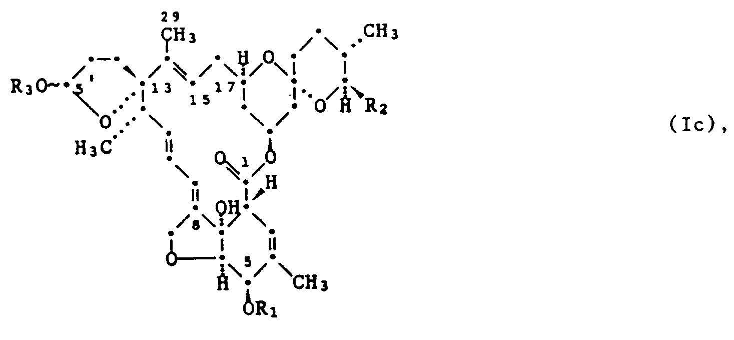 dans laquelle Ri, R₂ et R₃ ont les significations indiquées en référence à la formule I, par oxydation ménagée, en le composé correspondant de formule Ib 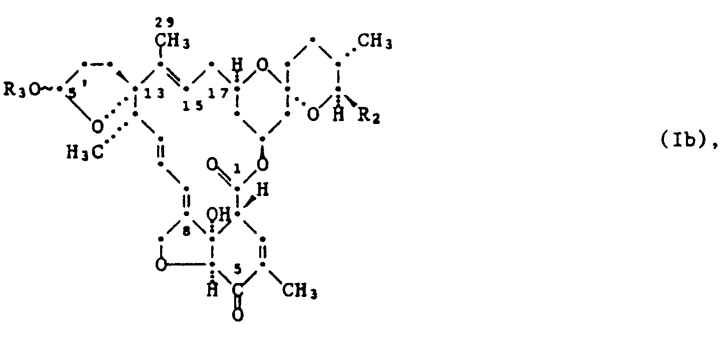 dans laquelle R₂ et R₃ ont les significations indiquées en référence à la formule Ic et si on le désire, on convertit le composé de formule Ib, par réaction avec l'hydroxylamine ou l'un de ses sels, en le composé correspondant de formule la 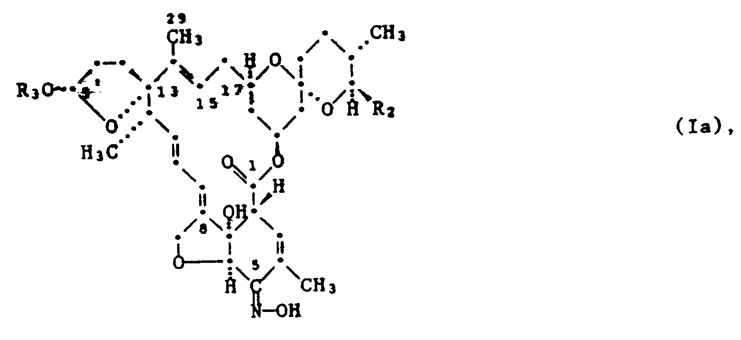 dans laquelle R₂ et R₃ ont les significations indiquées en référence à la formule Ib.

2. Procédé selon revendication 1, pour préparer un composé de formule I dans laquelle
X représente l'un des groupes -CH(OR₁)- ou -C( = N-OH)-;
R₂ représente un groupe méthyle, éthyle, isopropyle ou sec-butyle; et
R₃ représente l'hydrogène, un groupe alkyle en C 1-C 10, un groupe alkyle en C 1-C 10 portant au moins un substituant choisi parmi les halogènes, les groupes alcoxy en C 1-C 6, alcoxyalcoxy en C 2-C 6, alcoxyalcoxyalcoxy en C 3-C 9, alkylthio en C 1-C 6, cycloalkyle en C 3-C 7, hydroxy ou acyle en C 1-C 6, chacun des groupes mentionnés ci-dessus consistant en un groupe alcoxy ou contenant un groupe alcoxy pouvant être substitué en position terminale, sur un groupe alcoxy terminal, par un groupe hydroxy, un halogène, un groupe acyle en C 1-C 6 ou acyloxy en C 1-C 6, un groupe éthyle substitué par un groupe benzyloxy, un groupe cycloalkyle en C 3-C 7, un groupe cycloalkyle en C 3-C 7 portant au moins un substituant choisi parmi les halogènes et les groupes alkyle en C 1-C 3, un groupe cycloalcényle en C 3-C 7, alcényle en C 2-C 10, alcynyle en C 2-C 10, un groupe, substitué par des halogènes, des groupes alcoxy en C 1-C 6 ou acyloxy en C 1-C 6 et lui-même choisi parmi les groupes alcényle en C 2-C 10 et alcynyle en C 2-C 10, 1-adamantylméthyle, menthyle, carvéyle, phényle, benzyle, naphtyle, un groupe, portant au moins un substituant choisi parmi les halogènes, les groupes alkyle en C 1-C 3, halogénoalkyle en C 1-C 3, alcoxy en C 1-C 3, halogénoalcoxy en C 1-C 3, alkylthio en C 1-C 3, nitro et cyano, et choisi lui-même parmi les groupes phényle, benzyle et naphtyle, un groupe benzyle substitué par un groupe phénoxy, ou un hétérocycle de 4 à 6 chaînons non substitué ou portant au moins un substituant choisi parmi les halogènes, les groupes alkyle en C 1-C 3, halogénoalkyle en C 1-C 3, alcoxy en C 1-C 3, halogénoalcoxy en C 1-C 3, alkylthio en C 1-C 3, nitro et cyano, et qui contient 1 à 3 hétéroatomes choisis parmi l'oxygène, le soufre et l'azote, cet hétérocycle pouvant également être relié par l'intermédiaire d'un pont alkylène en C 1-C 6 à l'atome d'oxygène en position 5' du cycle tétrahydrofuranne.

3. Procédé selon revendication 2, pour préparer un composé de formule dans laquelle X représente -CH(ORi)-.

4. Procédé selon revendication 1, pour préparer un composé de formule dans laquelle X représente -CH(OR1 )-;
R₂ représente un groupe méthyle, éthyle, isopropyle ou sec-butyle; et
R₃ représente l'hydrogène, un groupe alkyle en C 1-C 10, un groupe alkyle en C 1-C 10 portant au moins un substituant choisi parmi les halogènes, les groupes alcoxy en C 1-C 6, alcoxyalcoxy en C 2-C 6, alcoxyalcoxyalcoxy en C 3-C 9, alkylthio en C 1-C 6, cycloalkyle en C 3-C 7, hydroxy et acyle en C 1-C 6, chacun des groupes mentionnés consistant en un groupe alcoxy ou contenant un groupe alcoxy pouvant être substitué en position terminale, sur un groupe alcoxy terminal, par un groupe hydroxy, un halogène, un groupe acyle en C 1-C 6 ou acyloxy en C 1-C 6, un groupe cycloalkyle en C 3-C 7, un groupe cycloalkyle en C 3-C 7 portant au moins un substituant choisi parmi les halogènes et les groupes alkyle en C 1-C 3, un groupe cycloalcényle en C 3-C 7, alcényle en C 2-C 10, alcynyle en C 2-C 10, un groupe, substitué par des halogènes, des groupes alcoxy en C 1-C 6 ou acyloxy en C 1-C 6 et lui-même choisi parmi les groupes alcényle en C 2-C 10 et alcynyle en C 2-C 10, 1- adamantylméthyle, menthyle, carvéyle, phényle, benzyle, naphtyle, un groupe, portant au moins un substituant choisi parmi les halogènes, les groupes alkyle en C 1-C 3, halogénoalkyle en C 1-C 3, alcoxy en C 1-C 3, halogénoalcoxy en C 1_C 3, alkylthio en C 1-C 3, nitro et cyano, et lui-même choisi parmi les groupes phényle, benzyle et naphtyle, ou un hétérocycle de 4 à 6 chaînons non substitué ou portant au moins un substituant choisi parmi les halogènes, les groupes alkyle en C 1-C 3, halogénoalkyle en C 1-C 3, alcoxy en C 1-C 3, halogénoalcoxy en C 1-C 3, alkylthio en C 1-C 3, nitro et cyano, et qui contient de 1 à 3 hétéroatomes choisis parmi l'oxygène, le soufre et l'azote, cet hétérocycle pouvant également être relié par l'intermédiaire d'un pont alkylène en C 1-C 6 à l'atome d'oxygène en position 5' du cycle tétrahydrofuranne.

5. Procédé selon revendication 4, pour préparer un composé de formule dans laquelle R₁ représente l'hydrogène, un groupe R₄-C(O)- ou -Si(R₅)(R₆)(R₇); R₄ représente un groupe alkyle en C 1-C 10, halogénoalkyle en C 1-C 10 ou un groupe, non substitué ou portant au moins un substituant choisi parmi les halogènes, les groupes alkyle en C 1-C 3, halogénoalkyle en C 1-C 3, alcoxy en C 1-C 3, halogénoalcoxy en C 1-C 3, cyano et nitro, et lui-même choisi parmi les groupes phényle et benzyle, et Rs, R₆ et R₇ représentent chacun, indépendamment les uns des autres, un groupe alkyle en C 1-C 6, benzyle ou phényle; R₂ représente un groupe méthyle, éthyle, isopropyle ou sec-butyle; et R₃ représente l'hydrogène, un groupe alkyle en C 1-C 5, un groupe alkyle en C 1-C 5 portant au moins un substituant choisi parmi les halogènes, les groupes alcoxy en C 1-C 3, alcoxyalcoxy en C 2-C 6, alcoxyalcoxyalcoxy en C 3-C 9, alkylthio en C 1-C 3, cycloalkyle en C 3-C 7, hydroxy et acyle en C 1-C 6, chacun des groupes mentionnés consistant en un groupe alcoxy ou contenant un groupe alcoxy pouvant être substitué en position terminale sur un groupe alcoxy terminal par un groupe hydroxy, un halogène, un groupe acyle en C 1-C 6 ou acyloxy en C 1-C 6; un groupe cycloalkyle en C 3-C 7, un groupe cycloalkyle en C 3-C 7 portant au moins un substituant choisi parmi le fluor, le chlore, le brome et les groupes méthyle, un groupe alcényle en C 2-C 6, un groupe alcynyle en C 2-C 6, un groupe, substitué par le fluor, le chlore, le brome, des groupes alcoxy en C 1-C 3 ou acyloxy en C 1-C 6, et lui-même choisi parmi les groupes alcényle en C 2-C 6 et alcynyle en C 2-C 6, phényle, benzyle, alpha-naphtyle, bêta-naphtyle, un groupe, portant au moins un substituant choisi parmi le fluor, le chlore, le brome, les groupes méthyle, méthoxy, CF₃, CF₃0, CH₃S, nitro et cyano, et lui-même choisi parmi les groupes phényle, benzyle, alpha-naphtyle et bêta-naphtyle, ou un hétérocycle de 4 à 6 chaînons, non substitué ou portant au moins un substituant choisi parmi le fluor, le chlore, le brome, les groupes méthyle, éthyle, CF₃, CF₃0, CF₃S, nitro et cyano, et contenant 1 à 3 hétéroatomes choisis parmi l'oxygène, le soufre et l'azote, cet hétérocycle pouvant également être relié par un pont alkylène en C 1-C 6 à l'atome d'oxygène en position 5' du cycle tétrahydrofuranne.

6. Procédé selon revendication 5, pour préparer un composé de formule dans laquelle Rs, R₆ et R₇ représentent chacun, indépendamment les uns des autres, un groupe alkyle en C 1-C 4, benzyle ou phényle; et R₃ représente un groupe alkyle en C 1-C 5 ou un groupe alkyle en C 1-C 5 portant au moins un substituant choisi parmi les halogènes, les groupes alcoxy en C 1-C 3, alcoxyalcoxy en C 2-C 6, alcoxyalcoxyalcoxy en C 3-C 9, alkylthio en C 1-C 3, cycloalkyle en C 3-C 7, hydroxy et acyle en C 1-C 6, chacun des groupes qu'on vient de mentionner consistant en un groupe alcoxy ou contenant un groupe alcoxy pouvant être substitué en position terminale sur un groupe alcoxy terminal par un groupe hydroxy, un halogène, un groupe acyle en C 1-C 6 ou acyloxy en C 1-C 6.

7. Procédé selon revendication 5, pour préparer un composé de formule dans laquelle Rs, R₆ et R₇ représentent chacun, indépendamment les uns des autres, un groupe alkyle en C 1-C 4, benzyle ou phényle; et R₃ représente un groupe cycloalkyle en C 3-C 7, un groupe cycloalkyle en C 3-C 7 portant au moins un substituant choisi parmi le fluor, le chlore, le brome et les groupes méthyle, un groupe alcényle en C 2-C 6, alcynyle en C 2-C 6, un groupe, substitué par le fluor, le chlore, le brome, des groupes alcoxy en C 1-C 3 ou acyloxy en C 1-C 6, et lui-même choisi parmi les groupes alcényle en C 2-C 6 et alcynyle en C 2-C 6, un groupe phényle, benzyle, alpha-naphtyle, bêta-naphtyle, un groupe, portant au moins un substituant choisi parmi le fluor, le chlore, le brome, les groupes méthyle, méthoxy, CF₃, CF₃0, CH₃S, nitro et cyano et lui-même choisi parmi les groupes phényle, benzyle, alpha-naphtyle et bêta-naphtyle, ou bien un hétérocycle de 4 à 6 chaînons non substitué ou portant au moins un substituant choisi parmi le fluor, le chlore, le brome, les groupes méthyle, éthyle, CF₃, CH₃0, CF₃0, CH₃S, nitro et cyano, qui contient 1 à 3 hétéroatomes choisis parmi l'oxygène, le soufre et l'azote et qui peut être relié par un pont alkylène en C 1-C 6 à l'atome d'oxygène en position 5' du cycle tétrahydrofuranne.

8. Procédé selon revendication 7, pour préparer un composé de formule dans laquelle R₃ représente un groupe phényle, benzyle, alpha-naphtyle, bêta-naphtyle, un groupe, portant au moins un substituant choisi parmi le fluor, le chlore, le brome, les groupes méthyle, méthoxy, CF₃, CF₃0, CH₃S, nitro et cyano, et lui-même choisi parmi les groupes phényle, benzyle, alpha-naphtyle et bêta-naphtyle.

9. Procédé selon revendication 7, pour préparer un composé de formule dans laquelle R₃ représente un hétérocycle de 4 à 6 chaînons non substitué ou portant au moins un substituant choisi parmi le fluor, le chlore, le brome, les groupes méthyle, éthyle, CF₃, CH₃0, CF₃0, CH₃S, nitro et cyano, et qui contient 1 à 3 hétéroatomes choisis parmi l'oxygène, le soufre et l'azote, cet hétérocycle pouvant également être relié par un pont alkylène en C 1-C 6 à l'atome d'oxygène en position 5' du cycle tétrahydrofuranne.

10. Procédé selon revendication 9, pour préparer un composé de formule dans laquelle R₃ représente un hétérocycle insaturé à 4 chaînons contenant un hétéroatome choisi parmi l'oxygène, l'azote ou le soufre, ou un cycle furanne, thiophène, pyrrole, isoxazole, isothiazole, furazanne, imidazole, 1,2,4-triazole, 1,2,3-triazole, pyrazole, pyrroline, oxazole, thiazole, thiadiazole, pyrazoline, thiazoline, pyrazolidine, pyrrolidine, oxazolidine, thiazolidine, oxadiazole, imidazoline, imidazolidine, pyrazolidine, tétrahydrofuranne, pyridine, pyridazine, pyrimidine, pyrazine, thiazine, thiadiazine, pyranne, pipéridine, pipérazine, morpholine, perhydrothiazine ou dioxanne, cet hétérocycle pouvant également être relie par un pont alkylène en C 1-C 4 à l'atome d'oxygène en position 5' du cycle tétrahydrofuranne.

11. Procédé selon revendication 9, pour préparer un composé de formule I dans laquelle R₃ représente un hétérocycle saturé de 4 chaînons contenant un hétéroatome choisi parmi l'oxygène, l'azote et le soufre, cet hétérocycle pouvant également être relié par un pont alkylène en C 1-C 4 à l'atome d'oxygène en position 5' du cycle tétrahydrofuranne.

12. Procédé selon revendication 1, pour préparer un composé de formule I dans laquelle X représente -CH(OR₁)- et R₁ représente l'hydrogène, R₂ représente un groupe éthyle; et R₃ un groupe alkyle en C 4-C 5, cycloalkyle en C 4-C 6, cycloalkyle en C 4-C 6 relié par un groupe méthyle, phényle, benzyle ou alpha-méthylbenzyle.

13. Procédé selon revendication 1, pour préparer un composé de formule I dans laquelle R₂ représente un groupe méthyle ou éthyle.

14. Procédé selon revendication 13, pour préparer un composé de formule I dans laquelle R₂ représente un groupe éthyle.

15. Procédé selon revendication 4, pour préparer un composé de formule 1 choisi dans le groupe formé par les suivants :
le milbémycine A₄-13-spiro-2'-[5'-(2"-éthoxy-éthoxy)-tétrahydrofuranne],
le milbémycine A₄-13-spiro-2'-[5'-(2",2"-diméthylpropoxy)-tétrahydrofuranne],
le milbémycine A₄-13-spiro-2'-[5'-cyclohexyloxy-tétrahydrofuranne],
le milbémycine A₄-13-spiro-2'-[5'-benzyloxy-tétrahydrofuranne],
le milbémycine A₄-13-spiro-2'-[5'-{2"-(2"'-méthoxy-éthoxy)-éthoxyl-tétrahydrofuranne],
le milbémycine A₄-13-spiro-2'-[5'-{2"-(2"'-(hydroxy-méthoxy)-éthoxy)-éthoxyl-tétrahydrofuranne],
le milbémycine A₄-13-spiro-2'-[5'-{2"-(2"'-(2""-(chloracétoxy)-éthoxy)-éthoxy)-éthoxyl-tétrahydrofuranne], le milbémycine A₄-13-spiro-2'-[5'-méthoxy-tétrahydro
furanne] et
le milbémycine A₄-13-spiro-2'-[5'-(2"-hydroxy-éthoxy)-tétrahydrofuranne].

16. Procédé selon revendication 12, pour préparer un composé de formule 1 choisi dans le groupe formé par le milbémycine A₄-13-spiro-2'-[5'-(2"-méthyl-butoxy)-tétrahydrofuranne] et le milbémycine A₄-13- spiro-2'-[5'-(1 "-méthyl-propoxy)-tétrahydrofuranne].

17. Procédé de préparation d'un composé de formule 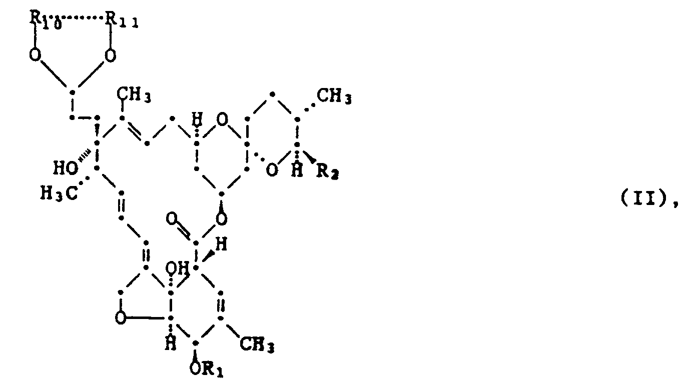 dans laquelle R₁ et R₂, R₁ o et R₁₁ ont les significations indiquées dans la revendication 1, caractérisé en ce que l'on fait réagir un composé de formule 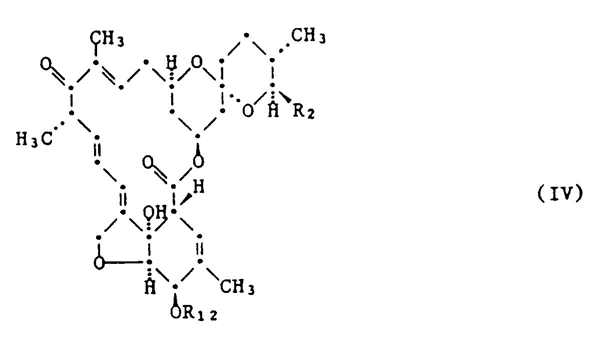 dans laquelle R₂,a les significations indiquées en référence à la formule I et R₁₂ représente l'hydrogène ou un groupe silyle, dans un solvant inerte dans la réaction, avec un réactif de Grignard de formule

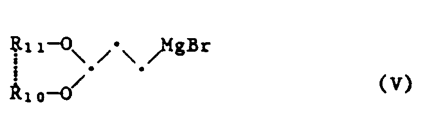

18. Procédé de préparation d'un produit pour combattre les ecto- et endo-parasites des animaux utiles ou pour combattre les insectes nuisibles, contenant en tant que substance active, avec des véhicules e1 diluants usuels, un composé de formule 1 obtenu selon revendication 1, caractérisé en ce que l'on mélange et/ou on broie la substance active avec les véhicules et diluants.

19. Procédé selon revendication 18, caractérisé en ce que le produit contient en tant que substance active un composé de formule 1 obtenu selon revendication 12.
